# EUROPEAN PATENT SPECIFICATION

(11) **EP 4 347 880 B1**
(45) Date of publication and mention of the grant of the patent: **04.02.2026**
(21) Application number: 22733822.5
(22) Date of filing: 01.06.2022
(51) Int. Cl.: C12Q 1/6853

(54) **SAMPLE ANALYSIS USING ASYMMETRIC CIRCULARIZABLE PROBES**
PROBENANALYSE MIT ASYMMETRISCHEN ZIRKULARISIERBAREN SONDEN
ANALYSE D'ÉCHANTILLON À L'AIDE DE SONDES CIRCULARISABLES ASYMÉTRIQUES

(30) Priority: 02.06.2021 US 202163196120 P
(43) Date of publication of application: 10.04.2024
(73) Proprietor: 10X Genomics, Inc., Pleasanton, CA 94588 (US); Lee, Hao Zhe, 171 72 Solna (SE); Surova, Olga, 171 65 Solna (SE)
(72) Inventor: HERNÁNDEZ NEUTA, Jorge, Iván, Pleasanton, CA 94588-3260 (US); NILSSON BERNITZ, Mats, Pleasanton, CA 94588-3260 (US); LEE, Hao Zhe, 171 72 Solna (SE); SUROVA, Olga, 171 65 Solna (SE)
(74) Representative: J A Kemp LLP
(86) International application number: PCT/US2022/031802
(87) International publication number: WO 2022/256422

(56) References cited:
- WO-A1-2017/019481
- WO-A1-2019/068880
- WO-A2-02/057491
- WO-A2-2005/010199
- TOMASZ KRZYWKOWSKI ET AL: "Fidelity of RNA templated end-joining by chlorella virus DNA ligase and a novel iLock assay with improved direct RNA detection accuracy", NUCLEIC ACIDS RESEARCH, vol. 45, no. 18, 13 October 2017 (2017-10-13), GB, pages e161 - e161, XP055521436, ISSN: 0305-1048, DOI: 10.1093/nar/gkx708
- LEE HOWER ET AL: "Direct RNA targeted transcriptomic profiling in tissue using Hybridization-based RNA In Situ Sequencing (HybRISS)", BIORXIV, 2 December 2020 (2020-12-02), XP055953500, Retrieved from the Internet <URL:https://www.biorxiv.org/content/10.1101/2020.12.02.408781v1.full.pdf> [retrieved on 20220822], DOI: 10.1101/2020.12.02.408781

## Description

### FIELD

The present disclosure relates in some aspects to methods and compositions for analysis of a target nucleic acid, such as *in situ* detection of a region of interest in a polynucleotide in a tissue sample.

### BACKGROUND

Methods are available for analyzing nucleic acids present in a biological sample, such as a cell or a tissue. For instance, advances in single molecule fluorescent *in situ* hybridization (smFISH) have enabled nanoscale-resolution imaging of RNA in cells and tissues. However, analysis of short sequences (e.g., single nucleotide polymorphisms (SNPs) or point mutations) on individual transcripts has remained challenging. Improved methods for analyzing nucleic acids present in a biological sample, such as *in situ* SNP genotyping, are needed. Provided herein are methods and compositions that address such and other needs. WO 2019/068880 discloses methods for detecting a target nucleic acid molecule in a sample comprising contacting said sample with a ligatable probe comprising one or more parts and allowing said probe to hybridise to the target nucleic acid molecule, ligating any probe which has hybridised to the target nucleic acid molecule, amplifying the ligated probe, and detecting the amplification product. Krzywkowski et al (2017) Nucleic Acids Research 45(18) discusses the fidelity of RNA templated end-joining by chlorella virus DNA ligase and a novel iLock assay with improved direct RNA detection accuracy.

### BRIEF SUMMARY

Ligation-based assays to identify and/or distinguish between sequence(s) of interest in a target nucleic acid can suffer from lack of specificity for multiple reasons, including properties of the ligase and/or the target nucleic acid. This lack of fidelity can result in formation and detection of a ligation product, even when the sequence of interest does not match the interrogatory region of a probe, producing a high level of background or false positive results. For instance, the SplintR^{®} Ligase (also known as the PBCV-1 DNA Ligase or Chorella virus DNA Ligase) efficiently catalyzes RNA-templated ligation of DNA ends. However, the ligase may not discriminate sequences while it ligates and can have high template independent ligation efficiency. A conventional padlock probe having an interrogatory base G may be designed to detect the complementary C in an RNA transcript. When the ligase fidelity is low, however, the padlock probe may be ligated when hybridized to a target having an A, T/U, or G instead of C, leading to false positive results. Using additional padlock probes having interrogatory bases other than G may not increase assay specificity; in fact, the additional "control" padlock probes may themselves suffer from template independent ligation and consequently false positive results. Thus, there is a need for ligation methods with higher specificity for detection of short sequences (e.g., one or more nucleotides) for genotyping *in situ.*

Provided herein is a method for analyzing a biological sample, comprising (a) contacting the biological sample with a circularizable probe, wherein: the circularizable probe comprises a 5' hybridization region and a 3' hybridization region that hybridize to adjacent first and second target regions, respectively, in a target nucleic acid in the biological sample, the 5' hybridization region and the 3' hybridization region are different in length, the 5' hybridization region or the 3' hybridization region comprises an interrogatory sequence complementary to a sequence of interest in the first or second target region, respectively, the interrogatory sequence does not comprise a ligatable end, and wherein the interrogatory sequence is in the shorter one of the 5' hybridization region and the 3' hybridization region; (b) circularizing the circularizable probe hybridized to the target nucleic acid to form a circularized probe; and (c) detecting the circularized probe or a product thereof in the biological sample. The circularizable probe comprises a linear oligonucleotide that, upon hybridization to a nucleic acid molecule (e.g., a target nucleic acid and/or a splint), forms a circularizable probe (e.g., a padlock probe that can be circularized). The circularizable probe can be circularized via ligation (or primer extension for gap filling followed by ligation) using the target nucleic acid or the splint as a template.

The circularizable probe comprises a 5' hybridization region and a 3' hybridization region that hybridize to adjacent first and second target regions, respectively, in a target nucleic acid in the biological sample. The 5' hybridization region and the 3' hybridization region are asymmetric. The 5' hybridization region can be longer than the 3' hybridization region, or vice versa. The adjacent first and second target regions in the target nucleic acid are asymmetric. The first target region can be longer than the second target region, or vice versa.

The 5' hybridization region or the 3' hybridization region comprises an interrogatory sequence complementary to a sequence of interest in the first or second target region, respectively. The interrogatory sequence can be internal in the 5' hybridization region or the 3' hybridization region. In any of the embodiments herein, the interrogatory sequence may not comprise a 5' or 3' terminal nucleotide. The interrogatory sequence does not comprise a ligatable end. The interrogatory sequence is an internal sequence in the 5' hybridization region or the 3' hybridization region of the circularizable probe which hybridize to the first and second target regions, respectively, in the target nucleic acid. In any of the embodiments herein, the interrogatory sequence may not comprise a nucleotide which, with or without processing of the circularizable probe or probe set, participates in a subsequent ligation reaction, for example, a ligation that is catalyzed by a ligase having RNA-splinted ligase activity.

The method further comprises circularizing the circularizable probe hybridized to the target nucleic acid to form a circularized probe. The method further comprises detecting the circularized probe or a product thereof in the biological sample.

In any of the embodiments herein, the target nucleic acid can comprise RNA. In any of the embodiments herein, the target nucleic acid can be an mRNA. In any of the embodiments herein, the circularizable probe or probe set can comprise DNA. In any of the embodiments herein, the circularizable probe or probe set can comprise one or more ribonucleotides. In any of the embodiments herein, the one or more ribonucleotides can be at and/or near a ligatable 3' end of the circularizable probe or probe set.

In any of the embodiments herein, the circularizable probe can be a padlock probe. In any of the embodiments herein, the 3' terminal nucleotide of the circularizable probe can be a ribonucleotide. In any of the embodiments herein, the circularizable probe can comprise one, two, three, four to more ribonucleotides. In any of the embodiments herein, the ribonucleotide(s) can be at or near the 3' terminus of the circularizable probe, for example, at or near the 3' terminal nucleotide, at or near the second nucleotide, at or near the third nucleotide, at or near the fourth nucleotide, at or near the fifth nucleotide, all from the 3' terminus of the circularizable probe.

The interrogatory sequence is in the shorter one of the 5' hybridization region and the 3' hybridization region. In any of the embodiments herein, the 5' hybridization region of a circularizable probe herein can be shorter or longer than the 3' hybridization region.

In any of the embodiments herein, the 5' hybridization region and the 3' hybridization region can be independently 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, or 35 nucleotides in length. In any of the embodiments herein, the lengths of the 5' hybridization region and the 3' hybridization region can differ by 1, 2, 3, 4, 5, 6, 7, 8, 9, 10, 11, 12, 13, 14, 15, 16, 17, 18, 19, 20, 21, 22, 23, 24, or 25 nucleotides. In any of the embodiments herein, the 5' hybridization region can comprise the interrogatory sequence and can be shorter than the 3' hybridization region by about 5, 6, 7, 8, 9, 10, 11, 12, 13, 14, or 15 nucleotides. In any of the embodiments herein, the 5' hybridization region can be about 10 nucleotides shorter than the 3' hybridization region.

In any of the embodiments herein, the sample can be contacted with a circularizable probe and the interrogatory sequence in the circularizable probe can be one, two, three, four, or five nucleotides in length. In any of the embodiments herein, the interrogatory sequence can be an interrogatory nucleotide, and the sequence of interest can be a single nucleotide of interest selected from the group consisting of a single-nucleotide polymorphism (SNP), a single-nucleotide variant (SNV), a single-nucleotide substitution, a point mutation, a single-nucleotide deletion, and a single-nucleotide insertion. For instance, the interrogatory nucleotide in a first circularizable probe disclosed herein can be complementary to a first variant of an SNP in the target nucleic acid, and the method may comprise contacting the biological sample with a second circularizable probe or probe set essentially identical to the first circularizable probe. The interrogatory nucleotide in the second circularizable probe or probe set can be complementary to a second variant of the SNP. The first and second circularizable probe or probe set may each comprise a barcode sequence that corresponds to the SNP variant that the circularizable probe or probe set targets.

In any of the embodiments herein, the 5' terminal nucleotide of the circularizable probe can be the first nucleotide of the 5' hybridization region, and an interrogatory nucleotide can be the second, third, fourth, fifth, sixth, seventh, eighth, ninth, or tenth nucleotide of the 5' hybridization region. In some examples, the interrogatory nucleotide is the fourth, fifth, or sixth nucleotide from the 5' phosphate group of the circularizable probe. In some examples, the interrogatory nucleotide is the fifth nucleotide of the 5' hybridization region. In any of the embodiments herein, the 3' terminal nucleotide of the circularizable probe can be the first nucleotide of the 3' hybridization region, and an interrogatory nucleotide can be the second, third, fourth, fifth, sixth, seventh, eighth, ninth, or tenth nucleotide of the 3' hybridization region.

In any of the embodiments herein, a 3' end and a 5' end of the circularizable probe or probe set can be ligated using the target nucleic acid as a template. In some embodiments, the 3' end and the 5' end are ligated without gap filling prior to ligation. In some embodiments, the ligation of the 3' end and the 5' end is preceded by gap filling. The gap may be 1, 2, 3, 4, 5, or more nucleotides.

In any of the embodiments herein, the circularizing step may comprise ligation selected from the group consisting of enzymatic ligation, chemical ligation, template dependent ligation, and/or template independent ligation. In any of the embodiments herein, the ligation can comprise using a ligase having an RNA-templated DNA ligase activity and/or an RNA-templated RNA ligase activity. In any of the embodiments herein, the ligation can comprise using a ligase selected from the group consisting of a Chlorella virus DNA ligase (PBCV DNA ligase), a T4 RNA ligase, a T4 DNA ligase, and a single-stranded DNA (ssDNA) ligase. In any of the embodiments herein, the ligation can comprise using a PBCV-1 DNA ligase or variant or derivative thereof and/or a T4 RNA ligase 2 (T4 Rnl2) or variant or derivative thereof.

In any of the embodiments herein, the method can further comprise prior to the circularizing step, a step of removing molecules of the circularizable probe that are not bound to the target nucleic acid from the biological sample. In any of the embodiments herein, the method can further comprise prior to the circularizing step, a step of removing molecules of the circularizable probe that are bound to the target nucleic acid but comprise one or more mismatches in the interrogatory sequence from the biological sample. In any of the embodiments herein, the method can further comprise prior to the circularizing step, a step of allowing circularizable probe molecules that are bound to the target nucleic acid but comprise one or more mismatches in the interrogatory sequence to dissociate from the target nucleic acid, while circularizable probe molecules comprising no mismatch in the interrogatory sequence remain bound to the target nucleic acid. In any of the embodiments herein, under the same conditions, the molecules comprising one or more mismatches can be less stably bound to the target nucleic acid than the molecules comprising no mismatch in the interrogatory sequence. In any of the embodiments herein, the method can comprise one or more stringency washes. For instance, one or more stringency washes can be used to remove circularizable probe molecules that are not bound to the target nucleic acid, and/or circularizable probe molecules that are bound to the target nucleic acid but comprise one or more mismatches in the interrogatory sequence.

In any of the embodiments herein, the method can further comprise generating the product of the circularized probe *in situ* in the biological sample. In any of the embodiments herein, the product can be generated using rolling circle amplification (RCA). In any of the embodiments herein, the RCA can comprise a linear RCA, a branched RCA, a dendritic RCA, or any combination thereof. In any of the embodiments herein, the product can be generated using a polymerase selected from the group consisting of Phi29 DNA polymerase, Phi29-like DNA polymerase, M2 DNA polymerase, B103 DNA polymerase, GA-1 DNA polymerase, phi-PRD1 polymerase, Vent DNA polymerase, Deep Vent DNA polymerase, Vent (exo-) DNA polymerase, KlenTaq DNA polymerase, DNA polymerase I, Klenow fragment of DNA polymerase I, DNA polymerase III, T3 DNA polymerase, T4 DNA polymerase, T5 DNA polymerase, T7 DNA polymerase, Bst polymerase, rBST DNA polymerase, N29 DNA polymerase, TopoTaq DNA polymerase, T7 RNA polymerase, SP6 RNA polymerase, T3 RNA polymerase, and a variant or derivative thereof.

In any of the embodiments herein, the product can be immobilized in the biological sample. In any of the embodiments herein, the product can be crosslinked to one or more other molecules in the biological sample.

In any of the embodiments herein, the method can further comprise imaging the biological sample to detect the circularized probe or product thereof. In any of the embodiments herein, the imaging can comprise detecting a signal associated with a fluorescently labeled probe that directly or indirectly binds to a rolling circle amplification product of the circularized probe. In any of the embodiments herein, a sequence of the rolling circle amplification product can be analyzed *in situ* in the biological sample. In any of the embodiments herein, the sequence of the rolling circle amplification product can be analyzed by sequential hybridization, sequencing by hybridization, sequencing by ligation, sequencing by synthesis, sequencing by binding, or a combination thereof.

In any of the embodiments herein, the sequence of the rolling circle amplification product can comprise one or more barcode sequences or complements thereof. In any of the embodiments herein, the one or more barcode sequences can comprise a barcode sequence corresponding to the target nucleic acid. In any of the embodiments herein, the one or more barcode sequences can comprise a barcode sequence corresponding to the sequence of interest, such as variant(s) of a single nucleotide of interest.

In any of the embodiments herein, the detecting step can comprise contacting the biological sample with one or more detectably-labeled probes that directly or indirectly hybridize to the rolling circle amplification product, and dehybridizing the one or more detectably-labeled probes from the rolling circle amplification product. In any of the embodiments herein, the contacting and dehybridizing steps can be repeated with the one or more detectably-labeled probes and/or one or more other detectably-labeled probes that directly or indirectly hybridize to the rolling circle amplification product.

In any of the embodiments herein, the detecting step can comprise contacting the biological sample with one or more intermediate probes that directly or indirectly hybridize to the rolling circle amplification product, wherein the one or more intermediate probes are detectable using one or more detectably-labeled probes. In any of the embodiments herein, the detecting step can further comprise dehybridizing the one or more intermediate probes and/or the one or more detectably-labeled probes from the rolling circle amplification product. In any of the embodiments herein, the contacting and dehybridizing steps can be repeated with the one or more intermediate probes, the one or more detectably-labeled probes, one or more other intermediate probes, and/or one or more other detectably-labeled probes.

In any of the embodiments herein, the biological sample can be a fixed and/or permeabilized biological sample. In any of the embodiments herein, the biological sample can be a tissue sample. In any of the embodiments herein, the biological sample can be a formalin-fixed, paraffin-embedded (FFPE) tissue sample, a frozen tissue sample, or a fresh tissue sample. In any of the embodiments herein, the tissue sample can be a tissue slice between about 1 µm and about 50 µm in thickness, optionally wherein the tissue slice is between about 5 µm and about 35 µm in thickness. In any of the embodiments herein, the biological sample can be crosslinked. In any of the embodiments herein, the biological sample can be embedded in a hydrogel matrix. In any of the embodiments herein, the biological sample can be cleared.

In any of the embodiments herein, the biological sample may not be embedded in a hydrogel matrix.

Also disclosed herein in some aspects is a kit for analyzing a biological sample, comprising a circularizable probe or probe set comprising a 5' hybridization region and a 3' hybridization region that hybridize to adjacent first and second target regions, respectively, in a target nucleic acid, wherein the 5' hybridization region and the 3' hybridization region are different in length; the 5' hybridization region or the 3' hybridization region comprises an interrogatory sequence complementary to a sequence of interest in the first or second target region, respectively; the interrogatory sequence does not comprise a ligatable end (e.g., the interrogatory sequence is an internal sequence in the 5' hybridization region and does not comprise the 5' terminal nucleotide) and the interrogatory sequence is in the shorter one of the 5' hybridization region and the 3' hybridization region.

### BRIEF DESCRIPTION OF THE FIGURES

The drawings illustrate certain embodiments of the features and advantages of this disclosure. These embodiments are not intended to limit the scope of the appended claims in any manner.

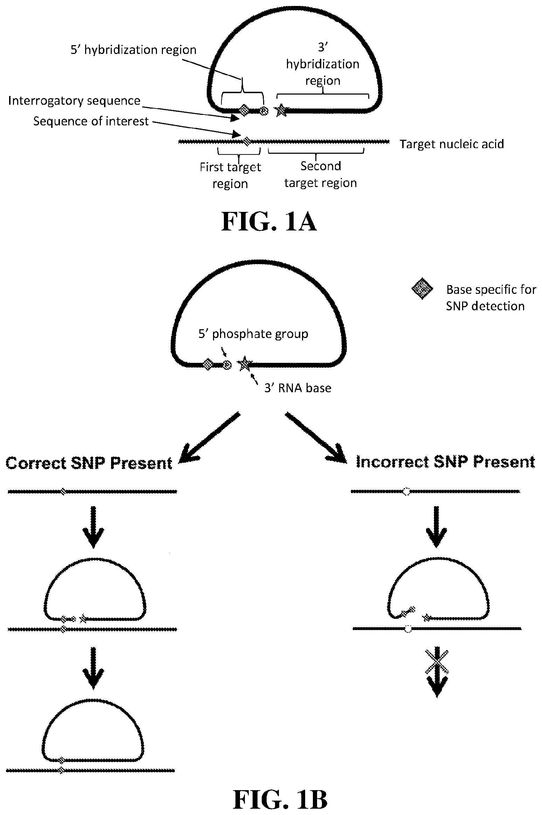

**FIGS. 1A-1B** show an exemplary method using an asymmetric circularizable probe (e.g., a padlock probe) for detecting a region of interest (e.g., an SNP) in a target nucleic acid. One arm of the asymmetric circularizable probe comprises an internal interrogatory nucleotide (e.g., base specific for SNP detection). The circularizable probe may comprise an optional 3' RNA base. The presence of a complementary base in the SNP location in the target nucleic acid favors stable hybridization of the circularizable probe and subsequent circularization. In contrast, the interrogatory nucleotide-containing arm is less stably hybridized when there is a mismatch between the interrogatory nucleotide and the SNP location in the target nucleic acid. The interrogatory nucleotide-containing arm (e.g., the shorter arm as shown in the figure) can dissociate from the target nucleic acid and prevent circularization of the circularizable probe by a ligase having poor fidelity (e.g., on RNA templates), thus reducing false positive signals due to indiscriminating ligase activity.

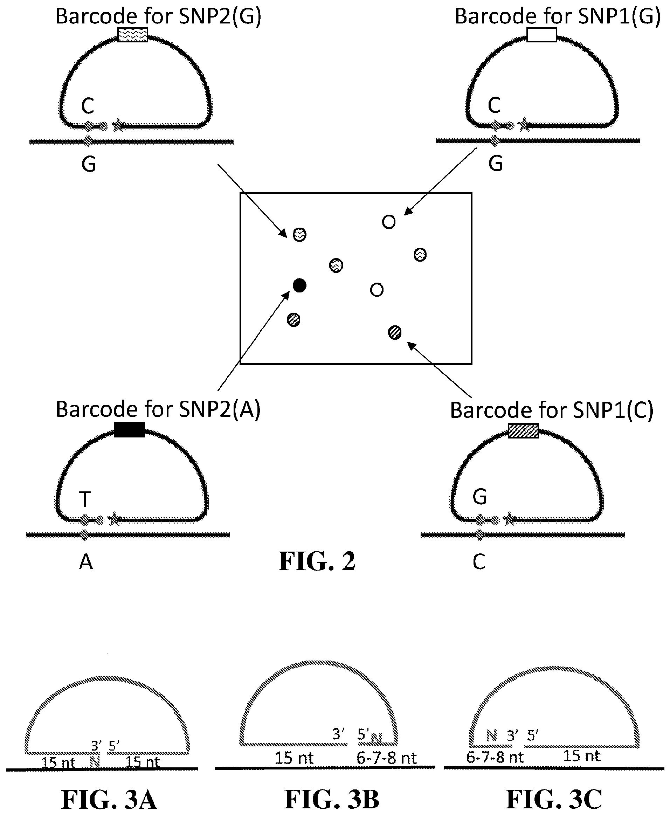

**FIG. 2** depicts an example of multiplexed *in situ* detection of two SNPs - SNP1 (sequence variation G/C) and SNP2 (sequence variation G/A) - in a sample. SNP1 and SNP2 are different SNPs and may be in different gene loci, e.g., on different chromosomes. DNA and/or RNA molecules in the sample may be detected. The barcoded circularizable probes or probe sets can be hybridized to the sample at the same time or sequentially. The sequence variations at the same SNP position can be differentially barcoded and detected, e.g., SNP1(G) and SNP1(C) each is associated with a different barcode. The single nucleotide of interest in different SNPs can also be differentially barcoded and detected, e.g., SNP1(G) and SNP2(G) each is associated with a different barcode.
**FIGS. 3A-3C** are schematics of probe configurations with symmetric and asymmetric hybridization regions. **FIG. 3A** is a schematic of a symmetric probe configuration, with an interrogatory base (N) at the 3' terminus. **FIGS. 3B-3C** are schematics of asymmetric probe configurations, having either a shorter 5' arm **(****FIG. 3B****)** or a shorter 3' arm **(****FIG. 3C****),** and an interrogatory base (N) at an inner position of the shorter arm.

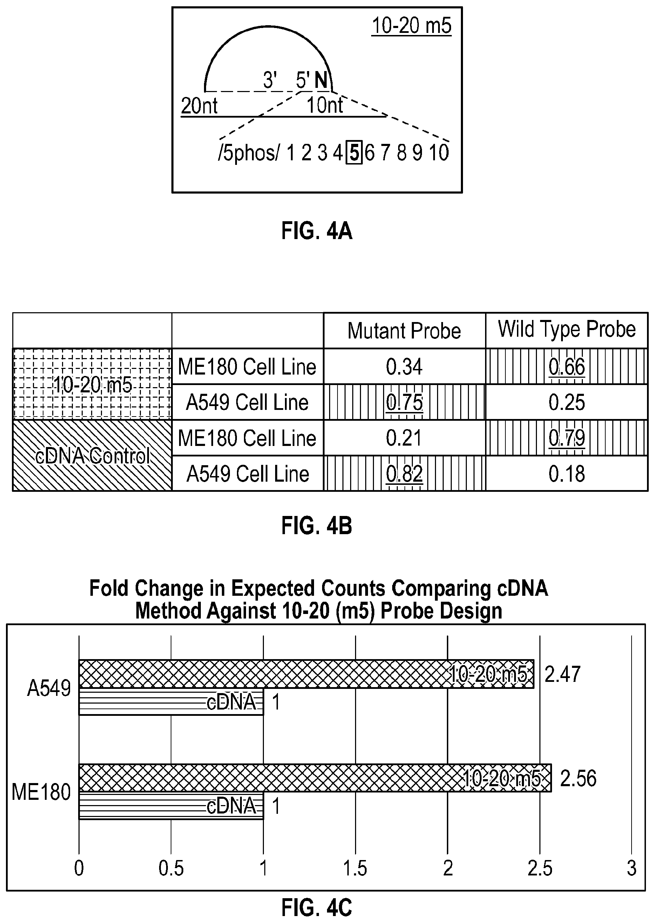

**FIGS. 4A-4B** show an exemplary asymmetric circularizable probe 10-20 m5 and results of using 10-20 m5 for *KRAS* mutant detection. **FIG. 4A** is a schematic of the 10-20 m5 asymmetric circularizable probe which comprises 10 nucleotides on the 5' arm and 20 nucleotides on the 3' arm, with an interrogatory base (N) on the 5^{th} base (from the 5' phosphate group) of the shorter arm. **FIG. 4B** shows the expected bias in count using the 10-20 m5 asymmetric circularizable probe on *KRAS* in a direct RNA (dRNA) approach without converting RNA into cDNA. A cDNA approach was used as control, in which RNA was first reverse transcribed into cDNA and a symmetric circularizable probe with the interrogatory base at the circularizable probe ligation junction was used to detect signals associated with *KRAS* wildtype and mutants in cultured cells. Signals from the dRNA approach (wildtype and mutant probes) and the cDNA approach (wildtype and mutant probes) were detected in ME180 (*KRAS* wildtype) and A549 (*KRAS* G12S mutant) cell lines. The results represent the average from three independent experiments. **FIG. 4C** shows the fold change in expected counts using the 10-20 m5 probe design, as compared to the cDNA control.

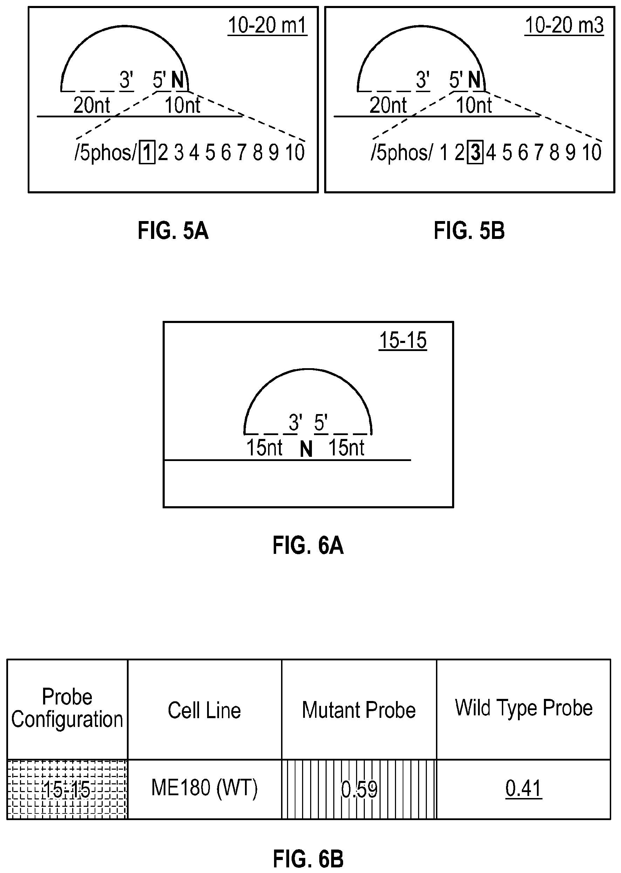

**FIGS. 5A-5B** depict an asymmetric probe design with an interrogatory base at the first position (10-20 m1) or third position (10-20 m3).
**FIGS. 6A-6B** show the evaluation of a 15-15 symmetric probe design. **FIG. 6A** is a schematic of the 15-15 symmetric probe designs. The 15-15 probe design has 15-nucleotide long 3' and 5' arms, with an interrogatory base (N) at the 3' end. **FIG. 6B** shows the expected bias of the 15-15 symmetric probe design in detection of *KRAS* RNA in ME180 cells. The expected bias value is bolded and underlined.

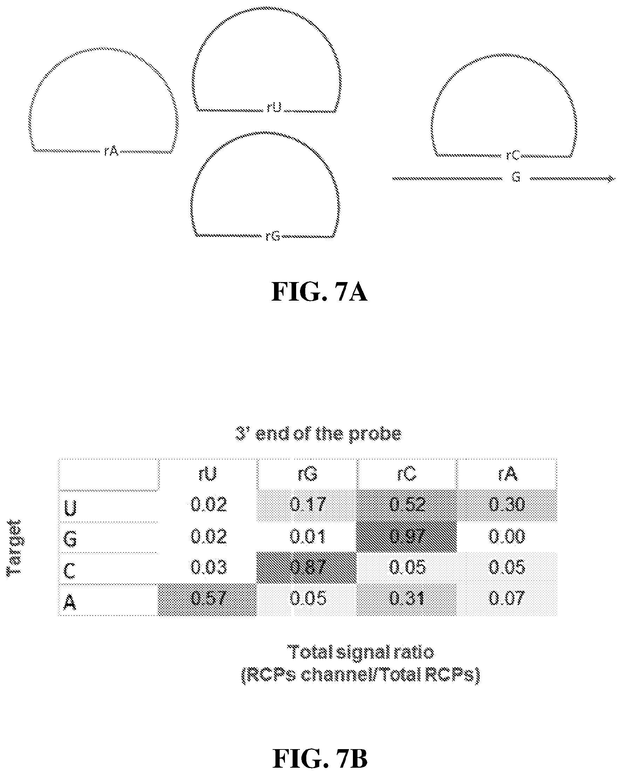

**FIGS. 7A-7B** show the validation of symmetric circularizable probes with an interrogatory ribonuclease base at the 3' end. Pools of circularizable probes was used, each having a different interrogatory ribonucleotide at the 3' end, and with only one of the probes matching a mutation in the target **(****FIG. 7A). FIG. 7B** shows a heat map of the specificity of the four different symmetric circularizable probe designs. The efficiency is indicated by the total signal ratio. The total signal ratio is depicted as the ratio of RCA products (RCPs) per detection channel divided by the total RCPs.

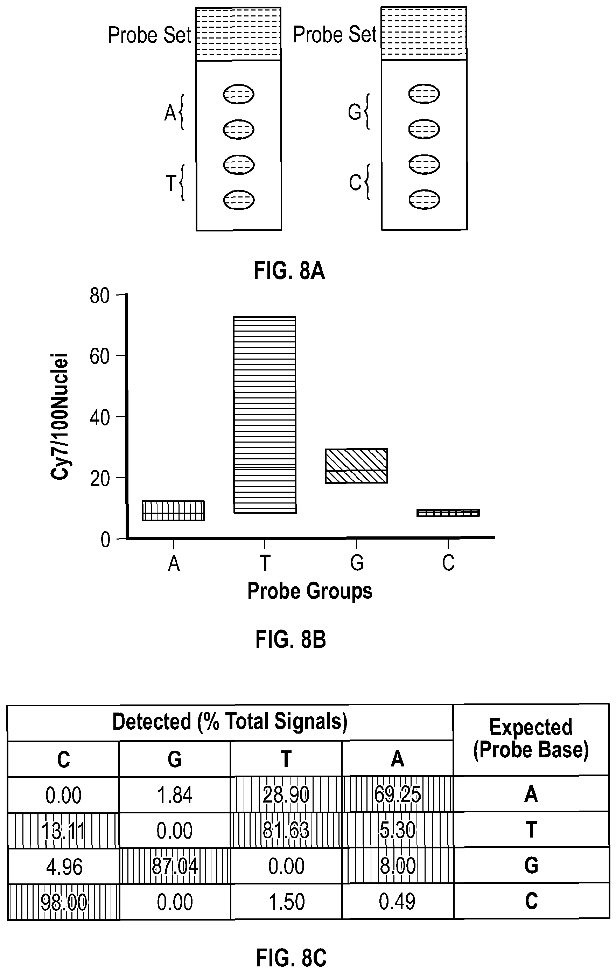

**FIGS. 8A-8C** show the detection of *pcp4* mRNA in mouse brain sections using 10-20 m5 asymmetric circularizable probes. Pools of circularizable probes was used, each having a different internal interrogatory base in the shorter arm, and with only one of the probes matching a mutation in the target. **FIG. 8A** is a schematic of the experimental setup. Each probe set contains the matching probe in addition to three mismatching probes. **FIG. 8B** shows the efficiency of the *pcp4* 10-20 probes as measured by anchor counts. **FIG. 8C** shows a heat map of the specificity of the *pcp4* 10-20 probes.

### DETAILED DESCRIPTION

The technical information set out below may in some respects go beyond the disclosure of the invention, which is defined by the appended claims. The additional technical information is provided to place the invention in a broader technical context and to illustrate possible related technical developments. Such additional technical information which does not fall within the scope of the appended claims is not part of the invention. Accordingly, any aspects, embodiments and examples of the present disclosure which do not fall under the scope of the appended claims do not form part of the invention and are merely provided for illustrative purposes. If a definition set forth herein is contrary to or otherwise inconsistent with a definition set forth in the patents, applications, published applications and other publications that are referenced, the definition set forth herein prevails over the definition that is referenced.

The section headings used herein are for organizational purposes only and are not to be construed as limiting the subject matter described.

### I. OVERVIEW

Certain ligases that are able to perform RNA templated ligation of DNA oligonucleotides, have limited discrimination properties towards mismatching nucleotides close to the ligation junction site. This limits the specificity of oligonucleotide ligation-based methods on RNA targets, since for these methods, the ligase plays an important role to be able to identify mutations. Padlock probes, being a derivative oligonucleotide ligation assay can be used for this purpose and has been described for detection of mutations on DNA templates. Larsson et al., Nature Methods 1(3):227-232, 2004. Most DNA ligases do not tolerate RNA templates, and the ones that do, for example, Chlorella virus DNA ligase (PBCV-1 DNA ligase), have poor end-joining fidelity and are able to ligate the junction site of the probe even if mismatches are present. Krzywkowski and Nilsson, Nucleic Acids Res 45:e161, 2017. Thus, the application of padlock probes for detecting mutations on RNA is limited. Krzywkowski et al., RNA 25:82-89, 2019.

In some aspects, provided herein are compositions and methods that overcome the limited ligase power to discriminate short nucleotide sequences such as single nucleotide mismatches in target nucleic acids *in situ.*

In some embodiments, disclosed herein are oligonucleotide probes that allow the specific detection of short nucleotide sequences of interest, such as single nucleotide polymorphism and point mutations, on target RNA sequences without converting (e.g., by reverse transcription) the RNA sequences to DNA sequences. In some embodiments, to address the limitations due to poor ligase specificity, circularizable probes or probes sets such as padlock probes comprising asymmetric target-complementary sequences (referred as "arms") are used to provide a level of discrimination to a padlock probe based assay for detecting short nucleotide sequences of interest on RNA templates. In some embodiments, the asymmetric geometry of the arms introduces a differential hybridization step, where the complete hybridization of the shorter arm is the limiting step of the reaction. In some embodiments, if the hybridization of the shorter arm is destabilized due to incomplete hybridization caused by, for instance, a sequence complementarity mismatch with the target RNA sequence, the probe arms are not joint by a ligase (e.g., due to removal or dissociation of an unstably hybridized circularizable probe or probe set from a target RNA), resulting in a negative signal.

In some embodiments, in addition to the asymmetric geometry of the circularizable probe arms (e.g., padlock probe arms), a circularizable probe disclosed herein also comprises an internal interrogatory sequence (e.g., an internal interrogatory nucleotide). In some embodiments, the shorter arm of the circularizable probe comprises an internal interrogatory sequence. For instance, the internal interrogatory sequence does not comprise a free 5' or 3' terminal nucleotide of the shorter arm. In some embodiments, the longer arm of the circularizable probe comprises an internal interrogatory sequence. For instance, the internal interrogatory sequence does not comprise a free 5' or 3' terminal nucleotide of the longer arm. In some embodiments, the circularizable probe ends are ligated using a target nucleic acid as template when the internal interrogatory sequence is complementary to a sequence of interest in the target nucleic acid. However, the internal interrogatory sequence does not comprise a nucleotide that provides a 5' phosphate or 3' hydroxyl group for the circularizable probe ligation. In other words, the internal interrogatory sequence in some embodiments does not comprise a nucleotide that is at a ligation junction. In some embodiments, the position of an interrogatory base in a circularizable probe can vary, e.g., 1, 2, 3, 4, 5, 6, or more nucleotides away from the ligation junction site on either the shorter or the longer arm.

It was generally believed that in order to distinguish single nucleotide of interest using circularizable probe ligation, the ligation junction of the circularizable probe should be placed exactly upon the single nucleotide of interest within a target (e.g., a transcript). Liu et al., Nucleic Acids Research, gkab120, 2021. Surprisingly, the present inventors have demonstrated that a circularizable probe comprising an internal interrogatory nucleotide that is not at the ligation junction can be used to successfully detect a single nucleotide of interest. In addition, detection specificity (e.g., the ability to distinguish a point mutation and wildtype nucleotide) using these circularizable probes is comparable to probes having interrogatory bases at the ligation junction (Example 1, **FIG. 4B****).** In some embodiments, the asymmetric circularizable probes disclosed here can directly target RNA molecules and exhibit increased detection efficiency compared to cDNA-based *in situ* detection using padlock probes (Example 1, **FIG. 4C****).**

### II. Samples, Analytes, and Target Sequences

### A. Samples

A sample disclosed herein can be or be derived from any biological sample. Methods and compositions disclosed herein may be used for analyzing a biological sample, which may be obtained from a subject using any of a variety of techniques including, but not limited to, biopsy, surgery, and laser capture microscopy (LCM), and generally comprises cells and/or other biological material from the subject. In addition to the subjects described above, a biological sample can be obtained from a prokaryote such as a bacterium, an archaea, a virus, or a viroid. A biological sample can also be obtained from non-mammalian organisms (e.g., a plant, an insect, an arachnid, a nematode, a fungus, or an amphibian). A biological sample can also be obtained from a eukaryote, such as a tissue sample, a patient derived organoid (PDO) or patient derived xenograft (PDX). A biological sample from an organism may comprise one or more other organisms or components therefrom. For example, a mammalian tissue section may comprise a prion, a viroid, a virus, a bacterium, a fungus, or components from other organisms, in addition to mammalian cells and non-cellular tissue components. Subjects from which biological samples can be obtained can be healthy or asymptomatic individuals, individuals that have or are suspected of having a disease (e.g., a patient with a disease such as cancer) or a predisposition to a disease, and/or individuals in need of therapy or suspected of needing therapy.

The biological sample can comprise any number of macromolecules, for example, cellular macromolecules and organelles (e.g., mitochondria and nuclei). The biological sample can be obtained as a tissue sample, such as a tissue section, biopsy, a core biopsy, needle aspirate, or fine needle aspirate. The sample can be a fluid sample, such as a blood sample, urine sample, or saliva sample. The sample can be a skin sample, a colon sample, a cheek swab, a histology sample, a histopathology sample, a plasma or serum sample, a tumor sample, living cells, cultured cells, a clinical sample such as, for example, whole blood or blood-derived products, blood cells, or cultured tissues or cells, including cell suspensions. In some embodiments, the biological sample may comprise cells which are deposited on a surface.

Biological samples can be derived from a homogeneous culture or population of the subjects or organisms mentioned herein or alternatively from a collection of several different organisms, for example, in a community or ecosystem.

Biological samples can include one or more diseased cells. A diseased cell can have altered metabolic properties, gene expression, protein expression, and/or morphologic features. Examples of diseases include inflammatory disorders, metabolic disorders, nervous system disorders, and cancer. Cancer cells can be derived from solid tumors, hematological malignancies, cell lines, or obtained as circulating tumor cells. Biological samples can also include fetal cells and immune cells.

Biological samples can include analytes (e.g., protein, RNA, and/or DNA) embedded in a 3D matrix. In some embodiments, amplicons (e.g., rolling circle amplification products) derived from or associated with analytes (e.g., protein, RNA, and/or DNA) can be embedded in a 3D matrix. In some embodiments, a 3D matrix may comprise a network of natural molecules and/or synthetic molecules that are chemically and/or enzymatically linked, e.g., by crosslinking. In some embodiments, a 3D matrix may comprise a synthetic polymer. In some embodiments, a 3D matrix comprises a hydrogel.

In some embodiments, a substrate herein can be any support that is insoluble in aqueous liquid and which allows for positioning of biological samples, analytes, features, and/or reagents (e.g., probes) on the support. In some embodiments, a biological sample can be attached to a substrate. Attachment of the biological sample can be irreversible or reversible, depending upon the nature of the sample and subsequent steps in the analytical method. In certain embodiments, the sample can be attached to the substrate reversibly by applying a suitable polymer coating to the substrate, and contacting the sample to the polymer coating. The sample can then be detached from the substrate, e.g., using an organic solvent that at least partially dissolves the polymer coating. Hydrogels are examples of polymers that are suitable for this purpose.

In some embodiments, the substrate can be coated or functionalized with one or more substances to facilitate attachment of the sample to the substrate. Suitable substances that can be used to coat or functionalize the substrate include, but are not limited to, lectins, poly-lysine, antibodies, and polysaccharides.

A variety of steps can be performed to prepare or process a biological sample for and/or during an assay. Except where indicated otherwise, the preparative or processing steps described below can generally be combined in any manner and in any order to appropriately prepare or process a particular sample for and/or analysis.

### (i) Tissue Sectioning

A biological sample can be harvested from a subject (e.g., via surgical biopsy, whole subject sectioning) or grown in vitro on a growth substrate or culture dish as a population of cells, and prepared for analysis as a tissue slice or tissue section. Grown samples may be sufficiently thin for analysis without further processing steps. Alternatively, grown samples, and samples obtained via biopsy or sectioning, can be prepared as thin tissue sections using a mechanical cutting apparatus such as a vibrating blade microtome. As another alternative, in some embodiments, a thin tissue section can be prepared by applying a touch imprint of a biological sample to a suitable substrate material.

The thickness of the tissue section can be a fraction of (e.g., less than 0.9, 0.8, 0.7, 0.6, 0.5, 0.4, 0.3, 0.2, or 0.1) the maximum cross-sectional dimension of a cell. However, tissue sections having a thickness that is larger than the maximum cross-section cell dimension can also be used. For example, cryostat sections can be used, which can be, e.g., 10-20 µm thick.

More generally, the thickness of a tissue section typically depends on the method used to prepare the section and the physical characteristics of the tissue, and therefore sections having a wide variety of different thicknesses can be prepared and used. For example, the thickness of the tissue section can be at least 0.1, 0.2, 0.3, 0.4, 0.5, 0.7, 1.0, 1.5, 2, 3, 4, 5, 6, 7, 8, 9, 10, 12, 13, 14, 15, 20, 30, 40, or 50 µm. Thicker sections can also be used if desired or convenient, e.g., at least 70, 80, 90, or 100 µm or more. Typically, the thickness of a tissue section is between 1-100 µm, 1-50 µm, 1-30 µm, 1-25 µm, 1-20 µm, 1-15 µm, 1-10 µm, 2-8 µm, 3-7 µm, or 4-6 µm, but as mentioned above, sections with thicknesses larger or smaller than these ranges can also be analysed.

Multiple sections can also be obtained from a single biological sample. For example, multiple tissue sections can be obtained from a surgical biopsy sample by performing serial sectioning of the biopsy sample using a sectioning blade. Spatial information among the serial sections can be preserved in this manner, and the sections can be analysed successively to obtain three-dimensional information about the biological sample.

### (ii) Freezing

In some embodiments, the biological sample (e.g., a tissue section as described above) can be prepared by deep freezing at a temperature suitable to maintain or preserve the integrity (e.g., the physical characteristics) of the tissue structure. The frozen tissue sample can be sectioned, e.g., thinly sliced, onto a substrate surface using any number of suitable methods. For example, a tissue sample can be prepared using a chilled microtome (e.g., a cryostat) set at a temperature suitable to maintain both the structural integrity of the tissue sample and the chemical properties of the nucleic acids in the sample. Such a temperature can be, e.g., less than -15°C, less than -20°C, or less than -25°C.

### (iii) Fixation and Post-fixation

In some embodiments, the biological sample can be prepared using formalin-fixation and paraffin-embedding (FFPE), which are established methods. In some embodiments, cell suspensions and other non-tissue samples can be prepared using formalin-fixation and paraffin-embedding. Following fixation of the sample and embedding in a paraffin or resin block, the sample can be sectioned as described above. Prior to analysis, the paraffin-embedding material can be removed from the tissue section (e.g., deparaffinization) by incubating the tissue section in an appropriate solvent (e.g., xylene) followed by a rinse (e.g., 99.5% ethanol for 2 minutes, 96% ethanol for 2 minutes, and 70% ethanol for 2 minutes).

As an alternative to formalin fixation described above, a biological sample can be fixed in any of a variety of other fixatives to preserve the biological structure of the sample prior to analysis. For example, a sample can be fixed via immersion in ethanol, methanol, acetone, paraformaldehyde (PFA)-Triton, and combinations thereof.

In some embodiments, acetone fixation is used with fresh frozen samples, which can include, but are not limited to, cortex tissue, mouse olfactory bulb, human brain tumor, human post-mortem brain, and breast cancer samples. When acetone fixation is performed, pre- permeabilization steps (described below) may not be performed. Alternatively, acetone fixation can be performed in conjunction with permeabilization steps.

In some embodiments, the methods provided herein comprises one or more post-fixing (also referred to as postfixation) steps. In some embodiments, one or more post-fixing step is performed after contacting a sample with a polynucleotide disclosed herein, e.g., one or more probes such as a circularizable probe or probe set or a circular probe. In some embodiments, one or more post-fixing step is performed after a hybridization complex comprising a probe and a target is formed in a sample. In some embodiments, one or more post-fixing step is performed prior to a ligation reaction disclosed herein, such as the ligation to circularize a circularizable probe or probe set (e.g., a padlock probe).

In some embodiments, one or more post-fixing step is performed after contacting a sample with a binding or labelling agent (e.g., an antibody or antigen binding fragment thereof) for a non-nucleic acid analyte such as a protein analyte. The labelling agent can comprise a nucleic acid molecule (e.g., reporter oligonucleotide) comprising a sequence corresponding to the labelling agent and therefore corresponds to (e.g., uniquely identifies) the analyte. In some embodiments, the labelling agent can comprise a reporter oligonucleotide comprising one or more barcode sequences.

A post-fixing step may be performed using any suitable fixation reagent disclosed herein, for example, 3% (w/v) paraformaldehyde in DEPC-PBS.

### (iv) Embedding

As an alternative to paraffin embedding described above, a biological sample can be embedded in any of a variety of other embedding materials to provide structural substrate to the sample prior to sectioning and other handling steps. In some cases, the embedding material can be removed e.g., prior to analysis of tissue sections obtained from the sample. Suitable embedding materials include, but are not limited to, waxes, resins (e.g., methacrylate resins), epoxies, and agar.

In some embodiments, the biological sample can be embedded in a matrix (e.g., a hydrogel matrix). Embedding the sample in this manner typically involves contacting the biological sample with a hydrogel such that the biological sample becomes surrounded by the hydrogel. For example, the sample can be embedded by contacting the sample with a suitable polymer material, and activating the polymer material to form a hydrogel. In some embodiments, the hydrogel is formed such that the hydrogel is internalized within the biological sample.

The composition and application of the hydrogel-matrix to a biological sample typically depends on the nature and preparation of the biological sample (e.g., sectioned, non-sectioned, type of fixation). As one example, where the biological sample is a tissue section, the hydrogel-matrix can include a monomer solution and an ammonium persulfate (APS) initiator/tetramethylethylenediamine (TEMED) accelerator solution. As another example, where the biological sample consists of cells (e.g., cultured cells or cells disassociated from a tissue sample), the cells can be incubated with the monomer solution and APS/TEMED solutions. For cells, hydrogel-matrix gels are formed in compartments, including but not limited to devices used to culture, maintain, or transport the cells. For example, hydrogel-matrices can be formed with monomer solution plus APS/TEMED added to the compartment to a depth ranging from about 0.1 µm to about 2 mm.

Additional methods and aspects of hydrogel embedding of biological samples are described for example in Chen et al., Science 347(6221):543-548, 2015.

### (v) Staining

To facilitate visualization, biological samples can be stained using a wide variety of stains and staining techniques. In some embodiments, for example, a sample can be stained using any number of stains, including but not limited to, acridine orange, Bismarck brown, carmine, coomassie blue, cresyl violet, DAPI, eosin, ethidium bromide, acid fuchsine, haematoxylin, Hoechst stains, iodine, methyl green, methylene blue, neutral red, Nile blue, Nile red, osmium tetroxide, propidium iodide, rhodamine, or safranine.

The sample can be stained using hematoxylin and eosin (H&E) staining techniques, using Papanicolaou staining techniques, Masson's trichrome staining techniques, silver staining techniques, Sudan staining techniques, and/or using Periodic Acid Schiff (PAS) staining techniques. PAS staining is typically performed after formalin or acetone fixation. In some embodiments, the sample can be stained using Romanowsky stain, including Wright's stain, Jenner's stain, Can-Grunwald stain, Leishman stain, and Giemsa stain.

In some embodiments, biological samples can be destained. Methods of destaining or discoloring a biological sample generally depend on the nature of the stain(s) applied to the sample. For example, in some embodiments, one or more immunofluorescent stains are applied to the sample via antibody coupling. Such stains can be removed using techniques such as cleavage of disulfide linkages via treatment with a reducing agent and detergent washing, chaotropic salt treatment, treatment with antigen retrieval solution, and treatment with an acidic glycine buffer. Methods for multiplexed staining and destaining are described, for example, in Bolognesi et al., J. Histochem. Cytochem. 2017; 65(8): 431-444, Lin et al., Nat Commun. 2015; 6:8390, Pirici et al., J. Histochem. Cytochem. 2009; 57:567-75, and Glass et al., J. Histochem. Cytochem. 2009; 57:899-905.

### (vi) Isometric Expansion

In some embodiments, a biological sample embedded in a matrix (e.g., a hydrogel) can be isometrically expanded. Isometric expansion methods that can be used include hydration, a preparative step in expansion microscopy, as described in Chen et al., Science 347(6221):543-548, 2015.

Isometric expansion can be performed by anchoring one or more components of a biological sample to a gel, followed by gel formation, proteolysis, and swelling. In some embodiments, analytes in the sample, products of the analytes, and/or probes associated with analytes in the sample can be anchored to the matrix (e.g., hydrogel). Isometric expansion of the biological sample can occur prior to immobilization of the biological sample on a substrate, or after the biological sample is immobilized to a substrate. In some embodiments, the isometrically expanded biological sample can be removed from the substrate prior to contacting the substrate with probes disclosed herein.

In general, the steps used to perform isometric expansion of the biological sample can depend on the characteristics of the sample (e.g., thickness of tissue section, fixation, cross-linking), and/or the analyte of interest (e.g., different conditions to anchor RNA, DNA, and protein to a gel).

In some embodiments, proteins in the biological sample are anchored to a swellable gel such as a polyelectrolyte gel. An antibody can be directed to the protein before, after, or in conjunction with being anchored to the swellable gel. DNA and/or RNA in a biological sample can also be anchored to the swellable gel via a suitable linker. Examples of such linkers include, but are not limited to, 6-((Acryloyl)amino) hexanoic acid (Acryloyl-X SE) (available from ThermoFisher, Waltham, MA), Label-IT Amine (available from MirusBio, Madison, WI) and Label X (described for example in Chen et al., Nat. Methods 13:679-684, 2016).

Isometric expansion of the sample can increase the spatial resolution of the subsequent analysis of the sample. The increased resolution in spatial profiling can be determined by comparison of an isometrically expanded sample with a sample that has not been isometrically expanded.

In some embodiments, a biological sample is isometrically expanded to a size at least 2x, 2.1x, 2.2x, 2.3x, 2.4x, 2.5x, 2.6x, 2.7x, 2.8x, 2.9x, 3x, 3.1x, 3.2x, 3.3x, 3.4x, 3.5x, 3.6x, 3.7x, 3.8x, 3.9x, 4x, 4.1x, 4.2x, 4.3x, 4.4x, 4.5x, 4.6x, 4.7x, 4.8x, or 4.9x its non-expanded size. In some embodiments, the sample is isometrically expanded to at least 2x and less than 20x of its non-expanded size.

### (vii) Crosslinking and De-crosslinking

In some embodiments, the biological sample is reversibly cross-linked prior to or during an *in situ* assay round. In some aspects, the analytes, polynucleotides and/or amplification product (e.g., amplicon) of an analyte or a probe bound thereto can be anchored to a polymer matrix. For example, the polymer matrix can be a hydrogel. In some embodiments, one or more of the polynucleotide probe(s) and/or amplification product (e.g., amplicon) thereof can be modified to contain functional groups that can be used as an anchoring site to attach the polynucleotide probes and/or amplification product to a polymer matrix. In some embodiments, a modified probe comprising oligo dT may be used to bind to mRNA molecules of interest, followed by reversible crosslinking of the mRNA molecules.

In some embodiments, the biological sample is immobilized in a hydrogel via cross-linking of the polymer material that forms the hydrogel. Cross-linking can be performed chemically and/or photochemically, or alternatively by any other hydrogel-formation method. A hydrogel may include a macromolecular polymer gel including a network. Within the network, some polymer chains can optionally be cross-linked, although cross-linking does not always occur.

In some embodiments, a hydrogel can include hydrogel subunits, such as, but not limited to, acrylamide, bis-acrylamide, polyacrylamide and derivatives thereof, poly(ethylene glycol) and derivatives thereof (e.g. PEG-acrylate (PEG-DA), PEG-RGD), gelatin-methacryloyl (GelMA), methacrylated hyaluronic acid (MeHA), polyaliphatic polyurethanes, polyether polyurethanes, polyester polyurethanes, polyethylene copolymers, polyamides, polyvinyl alcohols, polypropylene glycol, polytetramethylene oxide, polyvinyl pyrrolidone, polyacrylamide, poly(hydroxyethyl acrylate), and poly(hydroxyethyl methacrylate), collagen, hyaluronic acid, chitosan, dextran, agarose, gelatin, alginate, protein polymers, methylcellulose, and the like, and combinations thereof.

In some embodiments, a hydrogel includes a hybrid material, e.g., the hydrogel material includes elements of both synthetic and natural polymers. Examples of suitable hydrogels are described, for example, in U.S. Patent Nos. 6,391,937, 9,512,422, and 9,889,422, and in U.S. Patent Application Publication Nos. 2017/0253918, 2018/0052081 and 2010/0055733.

In some embodiments, the hydrogel can form the substrate. In some embodiments, the substrate includes a hydrogel and one or more second materials. In some embodiments, the hydrogel is placed on top of one or more second materials. For example, the hydrogel can be pre-formed and then placed on top of, underneath, or in any other configuration with one or more second materials. In some embodiments, hydrogel formation occurs after contacting one or more second materials during formation of the substrate. Hydrogel formation can also occur within a structure (e.g., wells, ridges, projections, and/or markings) located on a substrate.

In some embodiments, hydrogel formation on a substrate occurs before, contemporaneously with, or after probes are provided to the sample. For example, hydrogel formation can be performed on the substrate already containing the probes.

In some embodiments, hydrogel formation occurs within a biological sample. In some embodiments, a biological sample (e.g., tissue section) is embedded in a hydrogel. In some embodiments, hydrogel subunits are infused into the biological sample, and polymerization of the hydrogel is initiated by an external or internal stimulus.

In embodiments in which a hydrogel is formed within a biological sample, functionalization chemistry can be used. In some embodiments, functionalization chemistry includes hydrogel-tissue chemistry (HTC). Any hydrogel-tissue backbone (e.g., synthetic or native) suitable for HTC can be used for anchoring biological macromolecules and modulating functionalization. Non-limiting examples of methods using HTC backbone variants include CLARITY, PACT, ExM, SWITCH and ePACT. In some embodiments, hydrogel formation within a biological sample is permanent. For example, biological macromolecules can permanently adhere to the hydrogel allowing multiple rounds of interrogation. In some embodiments, hydrogel formation within a biological sample is reversible.

In some embodiments, additional reagents are added to the hydrogel subunits before, contemporaneously with, and/or after polymerization. For example, additional reagents can include but are not limited to oligonucleotides (e.g., probes), endonucleases to fragment DNA, fragmentation buffer for DNA, DNA polymerase enzymes, dNTPs used to amplify the nucleic acid and to attach the barcode to the amplified fragments. Other enzymes can be used, including without limitation, RNA polymerase, transposase, ligase, proteinase K, and DNAse. Additional reagents can also include reverse transcriptase enzymes, including enzymes with terminal transferase activity, primers, and switch oligonucleotides. In some embodiments, optical labels are added to the hydrogel subunits before, contemporaneously with, and/or after polymerization.

In some embodiments, HTC reagents are added to the hydrogel before, contemporaneously with, and/or after polymerization. In some embodiments, a cell labelling agent is added to the hydrogel before, contemporaneously with, and/or after polymerization. In some embodiments, a cell-penetrating agent is added to the hydrogel before, contemporaneously with, and/or after polymerization.

Hydrogels embedded within biological samples can be cleared using any suitable method. For example, electrophoretic tissue clearing methods can be used to remove biological macromolecules from the hydrogel-embedded sample. In some embodiments, a hydrogel-embedded sample is stored before or after clearing of hydrogel, in a medium (e.g., a mounting medium, methylcellulose, or other semi-solid mediums).

In some embodiments, a method disclosed herein comprises de-crosslinking the reversibly cross-linked biological sample. The de-crosslinking does not need to be complete. In some embodiments, only a portion of crosslinked molecules in the reversibly cross-linked biological sample are de-crosslinked and allowed to migrate.

### (viii) Tissue Permeabilization and Treatment

In some embodiments, a biological sample can be permeabilized to facilitate transfer of species (such as probes) into the sample. If a sample is not permeabilized sufficiently, the amount of species (such as probes) in the sample may be too low to enable adequate analysis. Conversely, if the tissue sample is too permeable, the relative spatial relationship of the analytes within the tissue sample can be lost. Hence, a balance between permeabilizing the tissue sample enough to obtain good signal intensity while still maintaining the spatial resolution of the analyte distribution in the sample is desirable.

In general, a biological sample can be permeabilized by exposing the sample to one or more permeabilizing agents. Suitable agents for this purpose include, but are not limited to, organic solvents (e.g., acetone, ethanol, and methanol), cross-linking agents (e.g., paraformaldehyde), detergents (e.g., saponin, Triton X-100^{™} or Tween-20^{™}), and enzymes (e.g., trypsin, proteases). In some embodiments, the biological sample can be incubated with a cellular permeabilizing agent to facilitate permeabilization of the sample. Additional methods for sample permeabilization are described, for example, in Jamur et al., Method Mol. Biol. 588:63-66, 2010. Any suitable method for sample permeabilization can generally be used in connection with the samples described herein.

In some embodiments, the biological sample can be permeabilized by adding one or more lysis reagents to the sample. Examples of suitable lysis agents include, but are not limited to, bioactive reagents such as lysis enzymes that are used for lysis of different cell types, e.g., gram positive or negative bacteria, plants, yeast, mammalian, such as lysozymes, achromopeptidase, lysostaphin, labiase, kitalase, lyticase, and a variety of other commercially available lysis enzymes.

Other lysis agents can additionally or alternatively be added to the biological sample to facilitate permeabilization. For example, surfactant-based lysis solutions can be used to lyse sample cells. Lysis solutions can include ionic surfactants such as, for example, sarcosyl and sodium dodecyl sulfate (SDS). More generally, chemical lysis agents can include, without limitation, organic solvents, chelating agents, detergents, surfactants, and chaotropic agents.

**In** some embodiments, the biological sample can be permeabilized by non-chemical permeabilization methods. Non-chemical permeabilization methods that can be used include, but are not limited to, physical lysis techniques such as electroporation, mechanical permeabilization methods (e.g., bead beating using a homogenizer and grinding balls to mechanically disrupt sample tissue structures), acoustic permeabilization (e.g., sonication), and thermal lysis techniques such as heating to induce thermal permeabilization of the sample.

Additional reagents can be added to a biological sample to perform various functions prior to analysis of the sample. In some embodiments, DNase and RNase inactivating agents or inhibitors such as proteinase K, and/or chelating agents such as EDTA, can be added to the sample. For example, a method disclosed herein may comprise a step for increasing accessibility of a nucleic acid for binding, e.g., a denaturation step to opening up DNA in a cell for hybridization by a probe. For example, proteinase K treatment may be used to free up DNA with proteins bound thereto.

### (ix) Selective Enrichment of RNA Species

In some embodiments, where RNA is the analyte, one or more RNA analyte species of interest can be selectively enriched. For example, one or more species of RNA of interest can be selected by addition of one or more oligonucleotides to the sample. In some embodiments, the additional oligonucleotide is a sequence used for priming a reaction by an enzyme (e.g., a polymerase). For example, one or more primer sequences with sequence complementarity to one or more RNAs of interest can be used to amplify the one or more RNAs of interest, thereby selectively enriching these RNAs.

Alternatively, one or more species of RNA can be down-selected (e.g., removed) using any of a variety of methods. For example, probes can be administered to a sample that selectively hybridize to ribosomal RNA (rRNA), thereby reducing the pool and concentration of rRNA in the sample. Additionally and alternatively, duplex-specific nuclease (DSN) treatment can remove rRNA (see, e.g., Archer, et al, Selective and flexible depletion of problematic sequences from RNA-seq libraries at the cDNA stage, BMC Genomics, 15 401, (2014)). Furthermore, hydroxyapatite chromatography can remove abundant species (e.g., rRNA) (see, e.g., Vandernoot, V.A., cDNA normalization by hydroxyapatite chromatography to enrich transcriptome diversity in RNA-seq applications, Biotechniques, 53(6) 373-80, (2012)).

A biological sample may comprise one or a plurality of analytes of interest. Methods for performing multiplexed assays to analyze two or more different analytes in a single biological sample are provided.

### B. Analytes

The methods and compositions disclosed herein can be used to detect and analyze a wide variety of different analytes, such as one or more target nucleic acids in a biological sample. In some embodiments, the methods and compositions disclosed herein can be in combination with other methods to detect and analyze a variety of additional analytes, such as additional target nucleic acids and/or non-nucleic acid target analytes. In some aspects, an analyte can include any biological substance, structure, moiety, or component to be analyzed. In some aspects, a target disclosed herein may similarly include any analyte of interest. In some examples, a target or analyte can be directly or indirectly detected.

Analytes can be derived from a specific type of cell and/or a specific subcellular region. For example, analytes can be derived from cytosol, from cell nuclei, from mitochondria, from microsomes, and more generally, from any other compartment, organelle, or portion of a cell. Permeabilizing agents that specifically target certain cell compartments and organelles can be used to selectively release analytes from cells for analysis, and/or allow access of one or more reagents (e.g., probes for analyte detection) to the analytes in the cell or cell compartment or organelle.

The analyte may include any biomolecule or chemical compound, including a macromolecule such as a protein or peptide, a lipid or a nucleic acid molecule, or a small molecule, including organic or inorganic molecules. The analyte may be a cell or a microorganism, including a virus, or a fragment or product thereof. An analyte can be any substance or entity for which a specific binding partner (e.g. an affinity binding partner) can be developed. Such a specific binding partner may be a nucleic acid probe (for a nucleic acid analyte) and may lead directly to the generation of a RCA template (e.g. a padlock or other circularizable probe). Alternatively, the specific binding partner may be coupled to a nucleic acid, which may be detected using an RCA strategy, e.g. in an assay which uses or generates a circular nucleic acid molecule which can be the RCA template.

Analytes of particular interest may include nucleic acid molecules, such as DNA (e.g. genomic DNA, mitochondrial DNA, plastid DNA, viral DNA, etc.) and RNA (e.g. mRNA, microRNA, rRNA, snRNA, viral RNA, etc.), and synthetic and/or modified nucleic acid molecules, (e.g. including nucleic acid domains comprising or consisting of synthetic or modified nucleotides such as LNA, PNA, morpholino, etc.), proteinaceous molecules such as peptides, polypeptides, proteins or prions or any molecule which includes a protein or polypeptide component, etc., or fragments thereof, or a lipid or carbohydrate molecule, or any molecule which comprise a lipid or carbohydrate component. The analyte may be a single molecule or a complex that contains two or more molecular subunits, e.g. including but not limited to protein-DNA complexes, which may or may not be covalently bound to one another, and which may be the same or different. Thus in addition to cells or microorganisms, such a complex analyte may also be a protein complex or protein interaction. Such a complex or interaction may thus be a homo- or hetero-multimer. Aggregates of molecules, e.g. proteins may also be target analytes, for example aggregates of the same protein or different proteins. The analyte may also be a complex between proteins or peptides and nucleic acid molecules such as DNA or RNA, e.g. interactions between proteins and nucleic acids, e.g. regulatory factors, such as transcription factors, and DNA or RNA.

### (i) Endogenous Analytes

In some embodiments, an analyte herein is endogenous to a biological sample and can include nucleic acid analytes and non-nucleic acid analytes. Methods and compositions disclosed herein can be used to analyze nucleic acid analytes (e.g., using a nucleic acid probe or probe set that directly or indirectly hybridizes to a nucleic acid analyte) and/or non-nucleic acid analytes (e.g., using a labelling agent that comprises a reporter oligonucleotide and binds directly or indirectly to a non-nucleic acid analyte) in any suitable combination.

Examples of non-nucleic acid analytes include, but are not limited to, lipids, carbohydrates, peptides, proteins, glycoproteins (N-linked or O-linked), lipoproteins, phosphoproteins, specific phosphorylated or acetylated variants of proteins, amidation variants of proteins, hydroxylation variants of proteins, methylation variants of proteins, ubiquitylation variants of proteins, sulfation variants of proteins, viral coat proteins, extracellular and intracellular proteins, antibodies, and antigen binding fragments. In some embodiments, the analyte is inside a cell or on a cell surface, such as a transmembrane analyte or one that is attached to the cell membrane. In some embodiments, the analyte can be an organelle (e.g., nuclei or mitochondria). In some embodiments, the analyte is an extracellular analyte, such as a secreted analyte. Exemplary analytes include, but are not limited to, a receptor, an antigen, a surface protein, a transmembrane protein, a cluster of differentiation protein, a protein channel, a protein pump, a carrier protein, a phospholipid, a glycoprotein, a glycolipid, a cell-cell interaction protein complex, an antigen-presenting complex, a major histocompatibility complex, an engineered T-cell receptor, a T-cell receptor, a B-cell receptor, a chimeric antigen receptor, an extracellular matrix protein, a posttranslational modification (e.g., phosphorylation, glycosylation, ubiquitination, nitrosylation, methylation, acetylation or lipidation) state of a cell surface protein, a gap junction, and an adherens junction.

Examples of nucleic acid analytes include DNA analytes such as single-stranded DNA (ssDNA), double-stranded DNA (dsDNA), genomic DNA, methylated DNA, specific methylated DNA sequences, fragmented DNA, mitochondrial DNA, *in situ* synthesized PCR products, and RNA/DNA hybrids. The DNA analyte can be a transcript of another nucleic acid molecule (e.g., DNA or RNA such as mRNA) present in a tissue sample.

Examples of nucleic acid analytes also include RNA analytes such as various types of coding and non-coding RNA. Examples of the different types of RNA analytes include messenger RNA (mRNA), including a nascent RNA, a pre-mRNA, a primary-transcript RNA, and a processed RNA, such as a capped mRNA (e.g., with a 5' 7-methyl guanosine cap), a polyadenylated mRNA (poly-A tail at the 3' end), and a spliced mRNA in which one or more introns have been removed. Also included in the analytes disclosed herein are non-capped mRNA, a non-polyadenylated mRNA, and a non-spliced mRNA. The RNA analyte can be a transcript of another nucleic acid molecule (e.g., DNA or RNA such as viral RNA) present in a tissue sample. Examples of a non-coding RNAs (ncRNA) that is not translated into a protein include transfer RNAs (tRNAs) and ribosomal RNAs (rRNAs), as well as small non-coding RNAs such as microRNA (miRNA), small interfering RNA (siRNA), Piwi-interacting RNA (piRNA), small nucleolar RNA (snoRNA), small nuclear RNA (snRNA), extracellular RNA (exRNA), small Cajal body-specific RNAs (scaRNAs), and the long ncRNAs such as Xist and HOTAIR. The RNA can be small (e.g., less than 200 nucleic acid bases in length) or large (e.g., RNA greater than 200 nucleic acid bases in length). Examples of small RNAs include 5.8S ribosomal RNA (rRNA), 5S rRNA, tRNA, miRNA, siRNA, snoRNAs, piRNA, tRNA-derived small RNA (tsRNA), and small rDNA-derived RNA (srRNA). The RNA can be double-stranded RNA or single-stranded RNA. The RNA can be circular RNA. The RNA can be a bacterial rRNA (e.g., 16s rRNA or 23s rRNA).

In some embodiments described herein, an analyte may be a denatured nucleic acid, wherein the resulting denatured nucleic acid is single-stranded. The nucleic acid may be denatured, for example, optionally using formamide, heat, or both formamide and heat. In some embodiments, the nucleic acid is not denatured for use in a method disclosed herein.

In certain embodiments, an analyte can be extracted from a live cell. Processing conditions can be adjusted to ensure that a biological sample remains live during analysis, and analytes are extracted from (or released from) live cells of the sample. Live cell-derived analytes can be obtained only once from the sample, or can be obtained at intervals from a sample that continues to remain in viable condition.

Methods and compositions disclosed herein can be used to analyze any number of analytes. For example, the number of analytes that are analyzed can be at least about 2, at least about 3, at least about 4, at least about 5, at least about 6, at least about 7, at least about 8, at least about 9, at least about 10, at least about 11, at least about 12, at least about 13, at least about 14, at least about 15, at least about 20, at least about 25, at least about 30, at least about 40, at least about 50, at least about 100, at least about 1,000, at least about 10,000, at least about 100,000 or more different analytes present in a region of the sample or within an individual feature of the substrate.

In any embodiment described herein, the analyte (e.g., target analyte such as target nucleic acid) can comprise a target sequence. In some embodiments, the target sequence may be endogenous to the sample, generated in the sample, added to the sample, or associated with an analyte in the sample. In some embodiments, the target sequence is a single-stranded target sequence (e.g., a sequence in a rolling circle amplification product). In some embodiments, the analytes comprise one or more single-stranded target sequences. In one aspect, a first single-stranded target sequence is not identical to a second single-stranded target sequence. In another aspect, a first single-stranded target sequence is identical to one or more second single-stranded target sequence. In some embodiments, the one or more second single-stranded target sequence is comprised in the same analyte (e.g., nucleic acid) as the first single-stranded target sequence. Alternatively, the one or more second single-stranded target sequence is comprised in a different analyte (e.g., nucleic acid) from the first single-stranded target sequence.

### (ii) Labelling Agents

In some embodiments, provided herein are methods and compositions for analyzing endogenous analytes (e.g., RNA, ssDNA, and cell surface or intracellular proteins and/or metabolites) in a sample using one or more labelling agents. In some embodiments, the methods and compositions comprising probes disclosed herein can be combined with analysis using one or more labelling agents. In some embodiments, an analyte labelling agent may include an agent that interacts with an analyte (e.g., an endogenous analyte in a sample). In some embodiments, the labelling agents can comprise a reporter oligonucleotide that is indicative of the analyte or portion thereof interacting with the labelling agent. For example, the reporter oligonucleotide may comprise a barcode sequence that permits identification of the labelling agent. In some cases, the sample contacted by the labelling agent can be further contacted with a probe (e.g., a single-stranded probe sequence), that hybridizes to a reporter oligonucleotide of the labelling agent, in order to identify the analyte associated with the labelling agent. In some embodiments, the analyte labelling agent comprises an analyte binding moiety and a labelling agent barcode domain comprising one or more barcode sequences, e.g., a barcode sequence that corresponds to the analyte binding moiety and/or the analyte. An analyte binding moiety barcode includes to a barcode that is associated with or otherwise identifies the analyte binding moiety. In some embodiments, by identifying an analyte binding moiety by identifying its associated analyte binding moiety barcode, the analyte to which the analyte binding moiety binds can also be identified. An analyte binding moiety barcode can be a nucleic acid sequence of a given length and/or sequence that is associated with the analyte binding moiety. An analyte binding moiety barcode can generally include any of the variety of aspects of barcodes described herein.

In some embodiments, the method comprises one or more post-fixing (also referred to as post-fixation) steps after contacting the sample with one or more labelling agents.

In the methods and systems described herein, one or more labelling agents capable of binding to or otherwise coupling to one or more features may be used to characterize analytes, cells and/or cell features. In some instances, cell features include cell surface features. Analytes may include, but are not limited to, a protein, a receptor, an antigen, a surface protein, a transmembrane protein, a cluster of differentiation protein, a protein channel, a protein pump, a carrier protein, a phospholipid, a glycoprotein, a glycolipid, a cell-cell interaction protein complex, an antigen-presenting complex, a major histocompatibility complex, an engineered T-cell receptor, a T-cell receptor, a B-cell receptor, a chimeric antigen receptor, a gap junction, an adherens junction, or any combination thereof. In some instances, cell features may include intracellular analytes, such as proteins, protein modifications (e.g., phosphorylation status or other post-translational modifications), nuclear proteins, nuclear membrane proteins, or any combination thereof.

In some embodiments, an analyte binding moiety may include any molecule or moiety capable of binding to an analyte (e.g., a biological analyte, e.g., a macromolecular constituent). A labelling agent may include, but is not limited to, a protein, a peptide, an antibody (or an epitope binding fragment thereof), a lipophilic moiety (such as cholesterol), a cell surface receptor binding molecule, a receptor ligand, a small molecule, a bi-specific antibody, a bi-specific T-cell engager, a T-cell receptor engager, a B-cell receptor engager, a pro-body, an aptamer, a monobody, an affimer, a darpin, and a protein scaffold, or any combination thereof. The labelling agents can include (e.g., are attached to) a reporter oligonucleotide that is indicative of the cell surface feature to which the binding group binds. For example, the reporter oligonucleotide may comprise a barcode sequence that permits identification of the labelling agent. For example, a labelling agent that is specific to one type of cell feature (e.g., a first cell surface feature) may have coupled thereto a first reporter oligonucleotide, while a labelling agent that is specific to a different cell feature (e.g., a second cell surface feature) may have a different reporter oligonucleotide coupled thereto. For a description of exemplary labelling agents, reporter oligonucleotides, and methods of use, see, e.g., U.S. Pat. 10,550,429; U.S. Pat. Pub. 2019/0177800; and U.S. Pat. Pub. 2019/0367969.

In some embodiments, an analyte binding moiety includes one or more antibodies or antigen binding fragments thereof. The antibodies or antigen binding fragments including the analyte binding moiety can specifically bind to a target analyte. In some embodiments, the analyte is a protein (e.g., a protein on a surface of the biological sample (e.g., a cell) or an intracellular protein). In some embodiments, a plurality of analyte labelling agents comprising a plurality of analyte binding moieties bind a plurality of analytes present in a biological sample. In some embodiments, the plurality of analytes includes a single species of analyte (e.g., a single species of polypeptide). In some embodiments in which the plurality of analytes includes a single species of analyte, the analyte binding moieties of the plurality of analyte labelling agents are the same. In some embodiments in which the plurality of analytes includes a single species of analyte, the analyte binding moieties of the plurality of analyte labelling agents are the different (e.g., members of the plurality of analyte labelling agents can have two or more species of analyte binding moieties, wherein each of the two or more species of analyte binding moieties binds a single species of analyte, e.g., at different binding sites). In some embodiments, the plurality of analytes includes multiple different species of analyte (e.g., multiple different species of polypeptides).

In other instances, e.g., to facilitate sample multiplexing, a labelling agent that is specific to a particular cell feature may have a first plurality of the labelling agent (e.g., an antibody or lipophilic moiety) coupled to a first reporter oligonucleotide and a second plurality of the labelling agent coupled to a second reporter oligonucleotide.

In some aspects, these reporter oligonucleotides may comprise nucleic acid barcode sequences that permit identification of the labelling agent which the reporter oligonucleotide is coupled to. The selection of oligonucleotides as the reporter may provide advantages of being able to generate significant diversity in terms of sequence, while also being readily attachable to most biomolecules, e.g., antibodies, etc., as well as being detectable.

Attachment (coupling) of the reporter oligonucleotides to the labelling agents may be achieved through any of a variety of direct or indirect, covalent or non-covalent associations or attachments. For example, oligonucleotides may be covalently attached to a portion of a labelling agent (such a protein, e.g., an antibody or antibody fragment) using chemical conjugation techniques (e.g., Lightning-Link^{®} antibody labelling kits available from Innova Biosciences), as well as other non-covalent attachment mechanisms, e.g., using biotinylated antibodies and oligonucleotides (or beads that include one or more biotinylated linker, coupled to oligonucleotides) with an avidin or streptavidin linker. Antibody and oligonucleotide biotinylation techniques are available. See, e.g., Fang, et al., "Fluoride-Cleavable Biotinylation Phosphoramidite for 5'-end-Labelling and Affinity Purification of Synthetic Oligonucleotides," Nucleic Acids Res. Jan. 15, 2003; 31(2):708-715. Likewise, protein and peptide biotinylation techniques have been developed and are readily available. See, e.g., U.S. Pat. No. 6,265,552. Furthermore, click reaction chemistry may be used to couple reporter oligonucleotides to labelling agents. Commercially available kits, such as those from Thunderlink and Abcam, and techniques common in the art may be used to couple reporter oligonucleotides to labelling agents as appropriate. In another example, a labelling agent is indirectly (e.g., via hybridization) coupled to a reporter oligonucleotide comprising a barcode sequence that identifies the label agent. For instance, the labelling agent may be directly coupled (e.g., covalently bound) to a hybridization oligonucleotide that comprises a sequence that hybridizes with a sequence of the reporter oligonucleotide. Hybridization of the hybridization oligonucleotide to the reporter oligonucleotide couples the labelling agent to the reporter oligonucleotide. In some embodiments, the reporter oligonucleotides are releasable from the labelling agent, such as upon application of a stimulus. For example, the reporter oligonucleotide may be attached to the labeling agent through a labile bond (e.g., chemically labile, photolabile, thermally labile, etc.) as generally described for releasing molecules from supports elsewhere herein.

**In** some cases, the labelling agent can comprise a reporter oligonucleotide and a label. A label can be fluorophore, a radioisotope, a molecule capable of a colorimetric reaction, a magnetic particle, or any other suitable molecule or compound capable of detection. The label can be conjugated to a labelling agent (or reporter oligonucleotide) either directly or indirectly (e.g., the label can be conjugated to a molecule that can bind to the labelling agent or reporter oligonucleotide). In some cases, a label is conjugated to a first oligonucleotide that is complementary (e.g., hybridizes) to a sequence of the reporter oligonucleotide.

**In** some embodiments, multiple different species of analytes (e.g., polypeptides) from the biological sample can be subsequently associated with the one or more physical properties of the biological sample. For example, the multiple different species of analytes can be associated with locations of the analytes in the biological sample. Such information (e.g., proteomic information when the analyte binding moiety(ies) recognizes a polypeptide(s)) can be used in association with other spatial information (e.g., genetic information from the biological sample, such as DNA sequence information, transcriptome information (e.g., sequences of transcripts), or both). For example, a cell surface protein of a cell can be associated with one or more physical properties of the cell (e.g., a shape, size, activity, or a type of the cell). The one or more physical properties can be characterized by imaging the cell. The cell can be bound by an analyte labelling agent comprising an analyte binding moiety that binds to the cell surface protein and an analyte binding moiety barcode that identifies that analyte binding moiety. Results of protein analysis in a sample (e.g., a tissue sample or a cell) can be associated with DNA and/or RNA analysis in the sample. In some embodiments, a labelling agent disclosed herein can be used for multiplex assays detecting a plurality of nucleic acid and/or non-nucleic acid analytes in combination with detection using asymmetric probes, where the asymmetric probes can be used for detecting one or more endogenous analytes (e.g., SNPs in genomic DNA and/or RNA such as mRNA transcripts).

### (iii) Products of Endogenous Analyte and/or Labelling Agent

**In** some embodiments, provided herein are methods and compositions for analyzing one or more products of an endogenous analyte and/or a labelling agent in a biological sample. In some embodiments, an endogenous analyte (e.g., a viral or cellular DNA or RNA) or a product (e.g., a hybridization product, a ligation product, an extension product (e.g., by a DNA or RNA polymerase), a replication product, a transcription/reverse transcription product, and/or an amplification product such as a rolling circle amplification (RCA) product) thereof is analyzed. In some examples, the product can be generated using polynucleotides as described in Section III and by performing hybridizations, ligations, amplification or other steps as described in Section IV. In some embodiments, a labelling agent that directly or indirectly binds to an analyte in the biological sample is analyzed. In some embodiments, a product (e.g., a hybridization product, a ligation product, an extension product (e.g., by a DNA or RNA polymerase), a replication product, a transcription/reverse transcription product, and/or an amplification product such as a rolling circle amplification (RCA) product) of a labelling agent that directly or indirectly binds to an analyte in the biological sample is analyzed. In some embodiments, one or more types of analytes can be analyzed using a combination of methods described herein.

### (a) Hybridization

In some embodiments, a product of an endogenous analyte and/or a labelling agent is a hybridization product comprising the pairing of substantially complementary or complementary nucleic acid sequences within two different molecules, one of which is the endogenous analyte or the labelling agent (e.g., reporter oligonucleotide attached thereto). The other molecule can be another endogenous molecule or another labelling agent such as a probe. Pairing can be achieved by any process in which a nucleic acid sequence joins with a substantially or fully complementary sequence through base pairing to form a hybridization complex. For purposes of hybridization, two nucleic acid sequences are "substantially complementary" if at least 60% (e.g., at least 70%, at least 80%, or at least 90%) of their individual bases are complementary to one another.

Various probes and probe sets can be hybridized to an endogenous analyte and/or a labelling agent and each probe may comprise one or more barcode sequences. For example, the probes and probe sets may comprise polynucleotides as described in Section III. Exemplary barcoded probes or probe sets may be based on a padlock probe, a gapped padlock probe, a SNAIL (Splint Nucleotide Assisted Intramolecular Ligation) probe set, a PLAYR (Proximity Ligation Assay for RNA) probe set, a PLISH (Proximity Ligation *in situ* Hybridization) probe set, and RNA-templated ligation probes. The specific probe or probe set design can vary.

### (b) Ligation

In some embodiments, a product of an endogenous analyte and/or a labelling agent is a ligation product. In some embodiments, the ligation product is formed between two or more endogenous analytes. In some embodiments, the ligation product is formed between an endogenous analyte and a labelling agent. In some embodiments, the ligation product is formed between two or more labelling agent. In some embodiments, the ligation product is an intramolecular ligation of an endogenous analyte. In some embodiments, the ligation product is an intramolecular ligation of a labelling agent, for example, the circularization of a circularizable probe or probe set upon hybridization to a target sequence. The target sequence can be comprised in an endogenous analyte (e.g., nucleic acid such as a genomic DNA or mRNA) or a product thereof (e.g., cDNA from a cellular mRNA transcript), or in a labelling agent (e.g., the reporter oligonucleotide) or a product thereof.

In some embodiments, provided herein is a probe or probe set capable of DNA-templated ligation, such as from a cDNA molecule. See, e.g., U.S. Pat. 8,551,710. In some embodiments, provided herein is a probe or probe set capable of RNA-templated ligation. See, e.g., U.S. Pat. Pub. 2020/0224244. In some embodiments, the probe set is a SNAIL probe set. See, e.g., U.S. Pat. Pub. 2019/0055594. In some embodiments, provided herein is a multiplexed proximity ligation assay. See, e.g., U.S. Pat. Pub. 2014/0194311. In some embodiments, provided herein is a probe or probe set capable of proximity ligation, for instance a proximity ligation assay for RNA (e.g., PLAYR) probe set. See, e.g., U.S. Pat. Pub. 2016/0108458. In some embodiments, a circular probe can be indirectly hybridized to the target nucleic acid. In some embodiments, the circular construct is formed from a probe set capable of proximity ligation, for instance a proximity ligation *in situ* hybridization (PLISH) probe set. See, e.g., U.S. Pat. Pub. 2020/0224243.

In some embodiments, the ligation involves chemical ligation. In some embodiments, the ligation involves template dependent ligation. In some embodiments, the ligation involves template independent ligation. In some embodiments, the ligation involves enzymatic ligation.

In some embodiments, the enzymatic ligation involves use of a ligase. In some aspects, the ligase used herein comprises an enzyme that is commonly used to join polynucleotides together or to join the ends of a single polynucleotide. An RNA ligase, a DNA ligase, or another variety of ligase can be used to ligate two nucleotide sequences together. Ligases comprise ATP-dependent double-strand polynucleotide ligases, NAD-i-dependent double-strand DNA or RNA ligases and single-strand polynucleotide ligases, for example any of the ligases described in EC 6.5.1.1 (ATP-dependent ligases), EC 6.5.1.2 (NAD+-dependent ligases), EC 6.5.1.3 (RNA ligases). Specific examples of ligases comprise bacterial ligases such as E. coli DNA ligase, Tth DNA ligase, Thermococcus sp. (strain 9° N) DNA ligase (9°N^{™} DNA ligase, New England Biolabs), Taq DNA ligase, Ampligase^{™} (Epicentre Biotechnologies) and phage ligases such as T3 DNA ligase, T4 DNA ligase and T7 DNA ligase and mutants thereof. In some embodiments, the ligase is a T4 RNA ligase. In some embodiments, the ligase is a splintR ligase. In some embodiments, the ligase is a single stranded DNA ligase. In some embodiments, the ligase is a T4 DNA ligase. In some embodiments, the ligase has an DNA-splinted DNA ligase activity. In some embodiments, the ligase has an RNA-splinted DNA ligase activity. In some embodiments, the ligase has an RNA-splinted RNA ligase activity.

In some embodiments, the ligation herein is a direct ligation. In some embodiments, the ligation herein is an indirect ligation. "Direct ligation" means that the ends of the polynucleotides hybridize immediately adjacently to one another to form a substrate for a ligase enzyme resulting in their ligation to each other (intramolecular ligation). Alternatively, "indirect" means that the ends of the polynucleotides hybridize non-adjacently to one another, e.g., separated by one or more intervening nucleotides or "gaps". In some embodiments, said ends are not ligated directly to each other, but instead occurs either via the intermediacy of one or more intervening (so-called "gap" or "gap-filling" (oligo)nucleotides) or by the extension of the 3' end of a probe to "fill" the "gap" corresponding to said intervening nucleotides (intermolecular ligation). In some cases, the gap of one or more nucleotides between the hybridized ends of the polynucleotides may be "filled" by one or more "gap" (oligo)nucleotide(s) which are complementary to a splint, circularizable probe, or target nucleic acid. The gap may be a gap of 1 to 60 nucleotides or a gap of 1 to 40 nucleotides or a gap of 3 to 40 nucleotides. In specific embodiments, the gap may be a gap of about 1, 2, 3, 4, 5, 6, 7, 8, 9 or 10 or more nucleotides, of any integer (or range of integers) of nucleotides in between the indicated values. In some embodiments, the gap between said terminal regions may be filled by a gap oligonucleotide or by extending the 3' end of a polynucleotide. In some cases, ligation involves ligating the ends of the probe to at least one gap (oligo)nucleotide, such that the gap (oligo)nucleotide becomes incorporated into the resulting polynucleotide. In some embodiments, the ligation herein is preceded by gap filling. In other embodiments, the ligation herein does not require gap filling.

In some embodiments, ligation of the polynucleotides produces polynucleotides with melting temperature higher than that of unligated polynucleotides. Thus, in some aspects, ligation stabilizes the hybridization complex containing the ligated polynucleotides prior to subsequent steps, comprising amplification and detection.

In some aspects, a high fidelity ligase, such as a thermostable DNA ligase (e.g., a *Taq* DNA ligase), is used. Thermostable DNA ligases are active at elevated temperatures, allowing further discrimination by incubating the ligation at a temperature near the melting temperature (Tₘ) of the DNA strands. This selectively reduces the concentration of annealed mismatched substrates (expected to have a slightly lower Tₘ around the mismatch) over annealed fully base-paired substrates. Thus, high-fidelity ligation can be achieved through a combination of the intrinsic selectivity of the ligase active site and balanced conditions to reduce the incidence of annealed mismatched dsDNA.

In some embodiments, the ligation herein is a proximity ligation of ligating two (or more) nucleic acid sequences that are in proximity with each other, e.g., through enzymatic means (e.g., a ligase). In some embodiments, proximity ligation can include a "gap-filling" step that involves incorporation of one or more nucleic acids by a polymerase, based on the nucleic acid sequence of a template nucleic acid molecule, spanning a distance between the two nucleic acid molecules of interest (see, e.g., U.S. Patent No. 7,264,929). A wide variety of different methods can be used for proximity ligating nucleic acid molecules, including (but not limited to) "sticky-end" and "blunt-end" ligations. Additionally, single-stranded ligation can be used to perform proximity ligation on a single-stranded nucleic acid molecule. Sticky-end proximity ligations involve the hybridization of complementary single-stranded sequences between the two nucleic acid molecules to be joined, prior to the ligation event itself. Blunt-end proximity ligations generally do not include hybridization of complementary regions from each nucleic acid molecule because both nucleic acid molecules lack a single-stranded overhang at the site of ligation.

### (c) Primer Extension and Amplification

In some embodiments, a product is a primer extension product of an analyte, a labelling agent, a probe or probe set bound to the analyte (e.g., a padlock probe bound to genomic DNA, mRNA, or cDNA), or a probe or probe set bound to the labelling agent (e.g., a padlock probe bound to one or more reporter oligonucleotides from the same or different labelling agents).

A primer is generally a single-stranded nucleic acid sequence having a 3' end that can be used as a substrate for a nucleic acid polymerase in a nucleic acid extension reaction. RNA primers are formed of RNA nucleotides, and are used in RNA synthesis, while DNA primers are formed of DNA nucleotides and used in DNA synthesis. Primers can also include both RNA nucleotides and DNA nucleotides (e.g., in a random or designed pattern). Primers can also include other natural or synthetic nucleotides described herein that can have additional functionality. In some examples, DNA primers can be used to prime RNA synthesis and vice versa (e.g., RNA primers can be used to prime DNA synthesis). Primers can vary in length. For example, primers can be about 6 bases to about 120 bases. For example, primers can include up to about 25 bases. A primer, may in some cases, refer to a primer binding sequence. In some embodiments, a primer extension reaction comprises any method where two nucleic acid sequences become linked (e.g., hybridized) by an overlap of their respective terminal complementary nucleic acid sequences (for example, 3' termini). Such linking can be followed by nucleic acid extension (e.g., an enzymatic extension) of one, or both termini using the other nucleic acid sequence as a template for extension. Enzymatic extension can be performed by an enzyme including, but not limited to, a polymerase and/or a reverse transcriptase.

In some embodiments, a product of an endogenous analyte and/or a labelling agent is an amplification product of one or more polynucleotides, for instance, a circular probe or circularizable probe or probe set. In some embodiments, the amplifying is achieved by performing rolling circle amplification (RCA). In other embodiments, a primer that hybridizes to the circular probe or circularized probe is added and used as such for amplification. In some embodiments, the RCA comprises a linear RCA, a branched RCA, a dendritic RCA, or any combination thereof.

In some embodiments, the amplification is performed at a temperature between or between about 20°C and about 60°C. In some embodiments, the amplification is performed at a temperature between or between about 30°C and about 40°C. In some aspects, the amplification step, such as the rolling circle amplification (RCA) is performed at a temperature between at or about 25°C and at or about 50°C, such as at or about 25°C, 27°C, 29°C, 31°C, 33°C, 35°C, 37°C, 39°C, 41°C, 43°C, 45°C, 47°C, or 49°C.

In some embodiments, upon addition of a DNA polymerase in the presence of appropriate dNTP precursors and other cofactors, a primer is elongated to produce multiple copies of the circular template. This amplification step can utilize isothermal amplification or non-isothermal amplification. In some embodiments, after the formation of the hybridization complex and association of the amplification probe, the hybridization complex is rolling-circle amplified to generate a cDNA nanoball (e.g., amplicon) containing multiple copies of the cDNA. Techniques for rolling circle amplification (RCA) that can be used herein include linear RCA, a branched RCA, a dendritic RCA, or any combination thereof. See, e.g., Baner et al, Nucleic Acids Research, 26:5073-5078, 1998; Lizardi et al, Nature Genetics 19:226, 1998; Mohsen et al., Acc Chem Res. 2016 November 15; 49(11): 2540-2550; Schweitzer et al. Proc. Natl Acad. Sci. USA 97:101 13- 1 19, 2000; Faruqi et al, BMC Genomics 2:4, 2000; Nallur et al, Nucl. Acids Res. 29:e118, 2001; Dean et al. Genome Res. 11: 1095- 1099, 2001; Schweitzer et al, Nature Biotech. 20:359-365, 2002; U.S. Patent Nos. 6,054,274, 6,291,187, 6,323,009, 6,344,329 and 6,368,801. Exemplary polymerases for use in RCA comprise DNA polymerase such phi29 (φ29) polymerase, Klenow fragment, *Bacillus stearothermophilus* DNA polymerase (BST), T4 DNA polymerase, T7 DNA polymerase, or DNA polymerase I. In some aspects, DNA polymerases that have been engineered or mutated to have desirable characteristics can be employed. In some embodiments, the polymerase is phi29 DNA polymerase.

In some aspects, during the amplification step, modified nucleotides can be added to the reaction to incorporate the modified nucleotides in the amplification product (e.g., nanoball). Exemplary of the modified nucleotides comprise amine-modified nucleotides. In some aspects of the methods, for example, for anchoring or cross-linking of the generated amplification product (e.g., nanoball) to a scaffold, to cellular structures and/or to other amplification products (e.g., other nanoballs). In some aspects, the amplification products comprises a modified nucleotide, such as an amine-modified nucleotide. In some embodiments, the amine-modified nucleotide comprises an acrylic acid N-hydroxysuccinimide moiety modification. Examples of other amine-modified nucleotides comprise, but are not limited to, a 5-Aminoallyl-dUTP moiety modification, a 5-Propargylamino-dCTP moiety modification, a N6-6-Aminohexyl-dATP moiety modification, or a 7-Deaza-7-Propargylamino-dATP moiety modification.

In some aspects, the polynucleotides and/or amplification product (e.g., amplicon) can be anchored to a polymer matrix. For example, the polymer matrix can be a hydrogel. In some embodiments, one or more of the polynucleotide probe(s) can be modified to contain functional groups that can be used as an anchoring site to attach the polynucleotide probes and/or amplification product to a polymer matrix. Exemplary modification and polymer matrix that can be employed in accordance with the provided embodiments comprise those described in, for example, US 2016/0024555, US 2018/0251833, US 2017/0219465, US 10,138,509, US 10,494,662, US 11,078,520, US 11,299,767, US 10,266,888, US 11,118,220, US 2021/0363579, US 2021/0324450, and US 2021/0215581. In some examples, the scaffold also contains modifications or functional groups that can react with or incorporate the modifications or functional groups of the probe set or amplification product. In some examples, the scaffold can comprise oligonucleotides, polymers or chemical groups, to provide a matrix and/or support structures.

The amplification products may be immobilized within the matrix generally at the location of the nucleic acid being amplified, thereby creating a localized colony of amplicons. The amplification products may be immobilized within the matrix by steric factors. The amplification products may also be immobilized within the matrix by covalent or noncovalent bonding. In this manner, the amplification products may be considered to be attached to the matrix. By being immobilized to the matrix, such as by covalent bonding or cross-linking, the size and spatial relationship of the original amplicons is maintained. By being immobilized to the matrix, such as by covalent bonding or cross-linking, the amplification products are resistant to movement or unraveling under mechanical stress.

In some aspects, the amplification products are copolymerized and/or covalently attached to the surrounding matrix thereby preserving their spatial relationship and any information inherent thereto. For example, if the amplification products are those generated from DNA or RNA within a cell embedded in the matrix, the amplification products can also be functionalized to form covalent attachment to the matrix preserving their spatial information within the cell thereby providing a subcellular localization distribution pattern. In some embodiments, the provided methods involve embedding the one or more polynucleotide probe sets and/or the amplification products in the presence of hydrogel subunits to form one or more hydrogel-embedded amplification products. In some embodiments, the hydrogel-tissue chemistry described comprises covalently attaching nucleic acids to in situ synthesized hydrogel for tissue clearing, enzyme diffusion, and multiple-cycle sequencing while an existing hydrogel-tissue chemistry method cannot. In some embodiments, to enable amplification product embedding in the tissue-hydrogel setting, amine-modified nucleotides are comprised in the amplification step (e.g., RCA), functionalized with an acrylamide moiety using acrylic acid N-hydroxysuccinimide esters, and copolymerized with acrylamide monomers to form a hydrogel.

In some embodiments, the RCA template may comprise the target analyte, or a part thereof, where the target analyte is a nucleic acid, or it may be provided or generated as a proxy, or a marker, for the analyte. A RCA-based detection system can be used for the detection of different analytes, e.g., where the signal is provided by generating a RCP from a circular RCA template which is provided or generated in the assay, and the RCP is detected to detect the analyte. The RCP may thus be regarded as a reporter which is detected to detect the target analyte. However, the RCA template may also be regarded as a reporter for the target analyte; the RCP is generated based on the RCA template, and comprises complementary copies of the RCA template. The RCA template determines the signal which is detected, and is thus indicative of the target analyte. As will be described in more detail below, the RCA template may be a probe, or a part or component of a probe, or may be generated from a probe, or it may be a component of a detection assay (e.g., a reagent in a detection assay), which is used as a reporter for the assay, or a part of a reporter, or signal-generation system. The RCA template used to generate the RCP may thus be a circular (e.g. circularized) reporter nucleic acid molecule, namely from any RCA-based detection assay which uses or generates a circular nucleic acid molecule as a reporter for the assay. Since the RCA template generates the RCP reporter, it may be viewed as part of the reporter system for the assay.

In some embodiments, a product herein includes a molecule or a complex generated in a series of reactions, e.g., hybridization, ligation, extension, replication, transcription/reverse transcription, and/or amplification (e.g., rolling circle amplification), in any suitable combination. For example, a product comprising a target sequence for a probe disclosed herein may be a hybridization complex formed of a cellular nucleic acid in a sample and an exogenously added nucleic acid probe. The exogenously added nucleic acid probe may comprise an overhang that does not hybridize to the cellular nucleic acid but hybridizes to another probe. The exogenously added nucleic acid probe may be optionally ligated to a cellular nucleic acid molecule or another exogenous nucleic acid molecule. In other examples, a product comprising a target sequence for a probe disclosed herein may be an RCP of a circularizable probe or probe set which hybridizes to a cellular nucleic acid molecule (e.g., genomic DNA or mRNA) or product thereof (e.g., a transcript such as cDNA, a DNA-templated ligation product of two probes, or an RNA-templated ligation product of two probes). In other examples, a product comprising a target sequence for a probe disclosed herein may a probe hybridizing to an RCP. The probe may comprise an overhang that does not hybridize to the RCP but hybridizes to another probe. The probe may be optionally ligated to a cellular nucleic acid molecule or another probe, e.g., an anchor probe that hybridize to the RCP.

### III. POLYNUCLEOTIDES AND HYBRIDIZATION COMPLEXES

Disclosed herein in some aspects are nucleic acid probes and/or probe sets (e.g., circularizable probes or probes sets such as padlock probes comprising asymmetric target-complementary sequences) that are introduced into a cell or used to otherwise contact a biological sample such as a tissue sample. The probes may comprise any of a variety of entities that can hybridize to a nucleic acid, typically by Watson-Crick base pairing, such as DNA, RNA, LNA, PNA, etc., depending on the application. The nucleic acid probe typically contains a targeting sequence that is able to bind to at least a portion of a target nucleic acid, In some embodiments specifically. The nucleic acid probe may be able to bind to a specific target nucleic acid (e.g., an mRNA, or other nucleic acids as discussed herein). In some embodiments, the nucleic acid probes may be determined using a detectable label, and/or by using secondary nucleic acid probes able to bind to the nucleic acid probes. In some embodiments, the nucleic acid probes are compatible with one or more biological and/or chemical reactions. For instance, a nucleic acid probe disclosed herein can serve as a template or primer for a polymerase, a template or substrate for a ligase, a substrate for a click chemistry reaction, and/or a substrate for a nuclease (e.g., endonuclease for cleavage).

Provided herein are methods involving the use of one or more probes (e.g., a padlock probe) for analyzing one or more target nucleic acid(s), such as a target nucleic acid (for example, a messenger RNA) present in a cell or a biological sample, such as a tissue sample. Also provided are probes, sets of probes, compositions, kits, systems and devices for use in accordance with the provided methods. In some aspects, the provided methods and systems can be applied to detect, image, quantitate, or determine the presence or absence of one or more target nucleic acid(s) or portions thereof (e.g., presence or absence of sequence variants such as point mutations and SNPs). In some aspects, the provided methods can be applied to detect, image, quantitate, or determine the sequence of one or more target nucleic acid(s), comprising sequence variants such as point mutations and SNPs.

**In** some aspects, the provided embodiments can be employed for *in situ* detection and/or sequencing of a target nucleic acid in a cell, e.g., in cells of a biological sample or a sample derived from a biological sample, such as a tissue section on a solid support, such as on a transparent slide.

**In** some aspects, provided herein are *in situ* assays using microscopy as a readout, e.g., nucleic acid sequencing, hybridization, or other detection or determination methods involving an optical readout. In some aspects, detection or determination of a sequence of one, two, three, four, five, or more nucleotides of a target nucleic acid is performed *in situ* in a cell in an intact tissue. In some aspects, detection or determination of a sequence is performed such that the localization of the target nucleic acid (or product or a derivative thereof associated with the target nucleic acid) in the originating sample is detected. In some embodiments, the assay comprises detecting the presence or absence of an amplification product or a portion thereof (e.g., RCA product). In some embodiments, a method for spatially profiling analytes such as the transcriptome or a subset thereof in a biological sample is provided. Methods, compositions, kits, devices, and systems for these *in situ* assays, comprising spatial genomics and transcriptomics assays, are provided. In some embodiments, a provided method is quantitative and preserves the spatial information within a tissue sample without physically isolating cells or using homogenates. In some embodiments, the present disclosure provides methods for high-throughput profiling one or more single nucleotides of interest in a large number of targets *in situ,* such as transcripts and/or DNA loci, for detecting and/or quantifying nucleic acids in cells, tissues, organs or organisms.

In some aspects, the methods disclosed herein involve the use of one or more probes or probe sets that hybridize to a target nucleic acid, such as an RNA molecule, wherein at least one probe is a circularizable probe comprising asymmetric arms that hybridize to a nucleic acid molecule. Exemplary probes may be based on a padlock probe, a gapped padlock probe, a SNAIL (Splint Nucleotide Assisted Intramolecular Ligation) probe set, a PLAYR (Proximity Ligation Assay for RNA) probe set, a PLISH (Proximity Ligation *in situ* Hybridization) probe set, and RNA-templated ligation probes. The specific probe or probe set design can vary. In some embodiments, a primary probe (e.g., a DNA probe that directly binds to an RNA target) is amplified through rolling circle amplification. In some embodiments, the primary probes, such as a circularizable probe or a probe set that comprises a circularizable probe, contain one or more barcodes. In some embodiments, one or more barcodes are indicative of a sequence in the target nucleic acid, such as a single nucleotide of interest (e.g., SNPs or point mutations), a dinucleotide sequence, or a short sequence of about 5 nucleotides in length.

A suitable circularizable probe or probe set may be a circularizable RCA template in the form of a linear molecule having ligatable ends which may circularized by ligating the ends together directly or indirectly, e.g., to each other, or to the respective ends of an intervening ("gap") oligonucleotide or to an extended 3' end of the circularizable RCA template. A circularizable template may also be provided in two or more parts, namely two or more molecules (e.g., oligonucleotides) which may be ligated together to form a circle. In some embodiments, the RCA template is circularized by ligation prior to RCA. Ligation may be templated using a ligation template, and in the case of padlock and molecular inversion probes and such like the target analyte may provide the ligation template, or it may be separately provided. In some embodiments, the circularizable RCA template (or template part or portion) comprises at its respective 3' and 5' ends regions of complementarity to corresponding cognate complementary regions (or binding sites) in the ligation template, which may be adjacent where the ends are directly ligated to each other, or non-adjacent, with an intervening "gap" sequence, where indirect ligation is to take place.

In the case of padlock probes, in one embodiment the ends of the padlock probe may be brought into proximity to each other by hybridization to adjacent sequences on a target nucleic acid molecule (such as a target analyte), which acts as a ligation template, thus allowing the ends to be ligated together to form a circular nucleic acid molecule, allowing the circularized padlock probe to act as a template for an RCA reaction. In such an example the terminal sequences of the padlock probe which hybridize to the target nucleic acid molecule can be specific to the target analyte in question, and can be replicated repeatedly in the RCA product. They may therefore act as a marker sequence indicative of that target analyte or a sequence thereof such as an SNP site in a target nucleic acid. Accordingly, it can be seen that the marker sequence in the RCA product may be equivalent to a sequence present in the target analyte itself. Alternatively, a marker sequence (e.g., tag or barcode sequence) may be provided in the non-target complementary parts of the padlock probe. In still a further embodiment, the marker sequence may be present in the gap oligonucleotide which is hybridized between the respective hybridized ends of the padlock probe, where they are hybridized to non-adjacent sequences in the target molecule.

In some embodiments, the detection method of the present disclosure may be used in the detection of a single nucleotide polymorphism, or indeed any variant base, in the target nucleic acid sequence. Circularizable probes for use in such a method may comprise a 5' additional sequence at the 5' end of the probe. Cleavage of the probe to remove the 5' additional sequence provides a 5' ligatable end, which may be ligated to the 3' ligatable end of the probe. As disclosed in detail elsewhere herein, the "arm" of the cleaved probe that comprises the 5' ligatable end and the "arm" comprising the 3' ligatable end can be asymmetric, e.g., the 5' "arm" can be shorter than the 3' "arm" such as shown in **FIG. 4A****.**

In some embodiments, a nucleic acid probe (e.g., a circularizable probe) disclosed herein can be pre-assembled from multiple components, e.g., prior to contacting the nucleic acid probe with a target nucleic acid or a sample. In some embodiments, a nucleic acid probe disclosed herein can be assembled during and/or after contacting a target nucleic acid or a sample with multiple components. In some embodiments, a nucleic acid probe disclosed herein is assembled *in situ* in a sample. In some embodiments, the multiple components can be contacted with a target nucleic acid or a sample in any suitable order and any suitable combination. For instance, a first component and a second component can be contacted with a target nucleic acid, to allow binding between the components and/or binding between the first and/or second components with the target nucleic acid. Optionally a reaction involving either or both components and/or the target nucleic acid, between the components, and/or between either one or both components and the target nucleic acid can be performed, such as hybridization, ligation, primer extension and/or amplification, chemical or enzymatic cleavage, click chemistry, or any combination thereof. In some embodiments, a third component can be added prior to, during, or after the reaction. In some embodiments, a third component can be added prior to, during, or after contacting the sample with the first and/or second components. In some embodiments, the first, second, and third components can be contacted with the sample in any suitable combination, sequentially or simultaneously. In some embodiments, the nucleic acid probe can be assembled *in situ* in a stepwise manner, each step with the addition of one or more components, or in a dynamic process where all components are assembled together. One or more removing steps, e.g., by washing the sample such as under stringent conditions, may be performed at any point during the assembling process to remove or destabilize undesired intermediates and/or components at that point and increase the chance of accurate probe assembly and specific target binding of the assembled probe.

In some aspects, the provided methods involve analyzing, e.g., detecting or determining, one or more sequences present in the polynucleotides and/or in an amplification product, such as in an amplification product of a circularized circularizable probe, which comprises one or more barcode sequences. In some embodiments, the analysis comprises determining the sequence of all or a portion of the amplification product. In some embodiments, the analysis comprises detecting a sequence present in the amplification product. In some embodiments, the sequence of all or a portion of the amplification product is indicative of the identity of a region (e.g., a single nucleotide) of interest in a target nucleic acid. In some embodiments, the analysis can be used to correlate a sequence detected in an amplification product to a circularizable probe (e.g., via a barcode). In some embodiments, the detection of a sequence in an amplification product can provide information regarding the location and/or identity of an associated target nucleic acid (e.g., hybridized by the circularizable probe) in a sample. In some embodiments, due to amplification of one or more polynucleotides (e.g., a padlock probe), particular sequences present in the amplification product or complementary sequences thereof can be detected even when a polynucleotide is present at low levels before the amplification. For example, the number of copies of the barcode sequence(s) and/or a complementary sequence thereof is increased by virtue of the amplification of a probe comprising the barcode sequence(s) and/or complementary sequence thereof, thereby enabling specific and sensitive detection of a signal indicative of the identity of a short region (e.g., a single nucleotide) of interest in a target nucleic acid. In particular embodiments, the amplification product is an *in situ* rolling circle amplification (RCA) product of a circularized circularizable probe.

In some embodiments, provided herein are methods for assessing one or more target nucleic acids, such as a plurality of different mRNAs, in a biological sample, such as a cell or a tissue sample (such as a tissue section). In some embodiments, the target nucleic acid comprises DNA. In some embodiments, the target nucleic acid comprises RNA. In some embodiments, the circularizable probe or probe set comprise DNA. In some embodiments, the target nucleic acid is RNA and the circularizable probe or probe set comprises DNA.

In some aspects, the provided methods are employed for *in situ* analysis of target nucleic acids, for example for *in situ* sequencing or multiplexed analysis in intact tissues or a sample with preserved cellular or tissue structure. In some aspects, the provided methods can be used to detect or determine the identity or amount *in situ* of single nucleotides of interest in target nucleic acids, for instance of single nucleotide polymorphisms of genes of interest.

In some aspects, the provided methods involve a step of contacting, or hybridizing, one or more polynucleotides, such as any of the circularizable probes described herein, to a cell or a sample containing a target nucleic acid with a region (e.g., single nucleotide) of interest in order to form a hybridization complex. In some aspects, the provided methods comprise one or more steps of ligating the polynucleotides, for instance of ligating the ends of the circularizable probe to form a circularized probe. In some aspects, the provided methods involve a step of amplifying one of the polynucleotides (e.g., a padlock probe or a circularized probe produced therefrom), to generate an amplification product. In some aspects, the provided methods involve a step of detecting and/or determining the sequence of all or a portion of the amplification product (for example, of one or more barcodes contained in the amplification product) and/or one or more of the polynucleotides with or without amplification, for instance any barcodes contained therein. In some aspects, the provided methods involve performing one or more of the steps described herein, simultaneously and/or sequentially.

In some embodiments, a circularizable probe disclosed herein comprise 1, 2, 3, 4, 5, 6, 7, 8, 9, 10, 11, 12, 13, 14, 15 or more, 20 or more, 32 or more, 40 or more, or 50 or more barcode sequences. The barcode sequences may be positioned anywhere within the nucleic acid probe. If more than one barcode sequences are present, the barcode sequences may be positioned next to each other, and/or interspersed with other sequences. In some embodiments, two or more of the barcode sequences may also at least partially overlap. In some embodiments, two or more of the barcode sequences in the same probe do not overlap. In some embodiments, all of the barcode sequences in the same probe are separated from one another by at least a phosphodiester bond (e.g., they may be immediately adjacent to each other but do not overlap), such as 1, 2, 3, 4, 5, 6, 7, 8, 9, 10 or more nucleotides apart.

The barcode sequences, if present, may be of any length. If more than one barcode sequence is used, the barcode sequences may independently have the same or different lengths, such as at least 5, at least 10, at least 15, at least 20, at least 25, at least 30, at least 35, at least 40, at least 50 nucleotides in length. In some embodiments, the barcode sequence may be no more than 120, no more than 112, no more than 104, no more than 96, no more than 88, no more than 80, no more than 72, no more than 64, no more than 56, no more than 48, no more than 40, no more than 32, no more than 24, no more than 16, or no more than 8 nucleotides in length. Combinations of any of these are also possible, e.g., the barcode sequence may be between 5 and 10 nucleotides, between 8 and 15 nucleotides, etc.

The barcode sequence may be arbitrary or random. In certain cases, the barcode sequences are chosen so as to reduce or minimize homology with other components in a sample, e.g., such that the barcode sequences do not themselves bind to or hybridize with other nucleic acids suspected of being within the cell or other sample. In some embodiments, between a particular barcode sequence and another sequence (e.g., a cellular nucleic acid sequence in a sample or other barcode sequences in probes added to the sample), the homology may be less than 10%, less than 8%, less than 7%, less than 6%, less than 5%, less than 4%, less than 3%, less than 2%, or less than 1%. In some embodiments, the homology may be less than 20, 19, 18, 17, 16, 15, 14, 13, 12, 11, 10, 9, 8, 7, 6, 5, 4, 3, or 2 bases, and in some embodiments, the bases are consecutive bases.

In some embodiments, the number of distinct barcode sequences in a population of nucleic acid probes is less than the number of distinct targets (e.g., nucleic acid analytes and/or protein analytes) of the nucleic acid probes, and yet the distinct targets may still be uniquely identified from one another, e.g., by encoding a probe with a different combination of barcode sequences. However, not all possible combinations of a given set of barcode sequences need be used. For instance, each probe may contain 1, 2, 3, 4, 5, 6, 7, 8, 9, 10, 11, 12, 13, 14, 15, etc. or more barcode sequences. In some embodiments, a population of nucleic acid probes may each contain the same number of barcode sequences, although in other cases, there may be different numbers of barcode sequences present on the various probes. In some embodiments, the barcode sequences or any subset thereof in the population of nucleic acid probes can be independently and/or combinatorially detected and/or decoded.

As an illustrative example, a first circularizable probe may contain a first target-binding sequence, a first barcode sequence, and a second barcode sequence, while a second, different circularizable probe may contain a second target-binding sequence (that is different from the first target-binding sequence in the first probe), the same first barcode sequence as in the first probe, but a third barcode sequence instead of the second barcode sequence. Such circularizable probes may thereby be distinguished by determining the various barcode sequence combinations present or associated with a given probe at a given location in a sample.

In some embodiments, disclosed herein is a method for analyzing a biological sample, comprising contacting the biological sample with a circularizable probe or probe set, wherein the circularizable probe comprises a 5' hybridization region and a 3' hybridization region for hybridizing to adjacent first and second regions, respectively, in a target nucleic acid, and the 5' hybridization region and the 3' hybridization region are different in length. In some embodiments, the 5' hybridization region and/or the 3' hybridization region comprises an interrogatory sequence for interrogating a sequence of interest in the first or second region, respectively. In some embodiments, the shorter one of the 5' hybridization region and the 3' hybridization region comprises the interrogatory sequence, such as an interrogatory nucleotide for a single nucleotide of interest in the target nucleic acid. In some embodiments, the sequence of interest in a first molecule of the target nucleic acid is complementary to the interrogatory sequence, whereas the sequence of interest in a second molecule of the target nucleic acid comprises a mismatch with the interrogatory sequence. In some embodiments, the mismatch is not at the 5' or 3' terminal nucleotide of the circularizable probe. In some embodiments, the method further comprises ligating the 5' and the 3' hybridization regions of the circularizable probe hybridized to the first molecule of the target nucleic acid to form a circularized circularizable probe, whereas under the same or similar conditions the 5' and the 3' hybridization regions of the circularizable probe hybridized to the second molecule of the target nucleic acid are not ligated. In some embodiments, the method further comprises detecting the circularized circularizable probe or probe set in the biological sample. In some embodiments, a signal associated with the circularized circularizable probe or probe set is detected *in situ* at the location of the first molecule of the target nucleic acid.

In some embodiments, provided herein is a method for analyzing a biological sample, comprising contacting the biological sample with a circularizable probe or probe set. In some embodiments, the circularizable probe or probe set comprises a linear oligonucleotide sequence that, upon hybridization to an RNA molecule of interest, forms a probe that can be circularized. In some embodiments, one or more oligonucleotides can be included in a probe set comprising a circularizable probe and used as primer(s) to prime RCA of a circular template formed by circularizing the circularizable probe. The circularizable probe or probe set can be circularized via ligation (or primer extension followed by ligation) using the target RNA and/or the DNA splint as a template, e.g., as described in Section II.B.(iii). The circularizable probe or probe set can comprise asymmetric 5' and 3' hybridization regions that hybridize to adjacent first and second target regions, respectively, in the target RNA. In some embodiments, a circularizable probe or probe set comprises a polynucleotide (e.g., as shown in **FIG. 1A****)** with two regions for hybridizing to a first and second target region in the target RNA, one region is at the 5' end (e.g., 5' arm) of the circularizable probe (e.g., 5' hybridization region) and the other region is at the 3' end (e.g., 3' arm) of the circularizable probe (e.g., 3' hybridization region). In some embodiments, the circularizable probe comprises 5' and 3' hybridization regions that are asymmetric in length that hybridize to the target regions in the target RNA. The 5' hybridization region can be longer than the 3' hybridization region, or vice versa. The first target region can be longer than the second target region, or vice versa. The internal interrogatory sequence can be on the 5' or 3' arm and does not participate in a subsequent padlock ligation step.

The internal interrogatory sequence can be in the shorter one of the 5' hybridization region and the 3' hybridization region. In some embodiments, the internal interrogatory sequence can be in the longer one of the 5' hybridization region and the 3' hybridization region. In any of the embodiments herein, the 5' hybridization region of a circularizable probe herein can be shorter or longer than the 3' hybridization region. In any of the embodiments herein, the 5' hybridization region and the 3' hybridization region can be independently 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, or more nucleotides in length. In some embodiments, the 5' hybridization region is between about 5 and about 20 nucleotides in length, while the 3' hybridization region is between about 10 and about 40 nucleotides in length. In some embodiments, the 5' hybridization region is between about 10 and about 15 nucleotides in length, while the 3' hybridization region is between about 15 and about 30 nucleotides in length. In some embodiments, the 5' hybridization region is between about 8 and about 12 nucleotides in length, while the 3' hybridization region is between about 16 and about 24 nucleotides in length. In some embodiments, the 5' hybridization region is about 10 nucleotides in length, while the 3' hybridization region is about 20 nucleotides in length.

In any of the embodiments herein, the lengths of the 5' hybridization region and the 3' hybridization region can differ by 1, 2, 3, 4, 5, 6, 7, 8, 9, 10, 11, 12, 13, 14, 15, 16, 17, 18, 19, 20, 21, 22, 23, 24, or 25 nucleotides. In any of the embodiments herein, the 5' hybridization region can comprise the interrogatory sequence and can be shorter than the 3' hybridization region by about 5, 6, 7, 8, 9, 10, 11, 12, 13, 14, or 15 nucleotides. In any of the embodiments herein, the 5' hybridization region can be about 1, 2, 3, 4, 5, 6, 7, 8, 9, 10, 11, 12, 13, 14, or 15 nucleotides shorter than the 3' hybridization region.

In any of the embodiments herein, the 5' terminal nucleotide of the circularizable probe can be the first nucleotide of the 5' hybridization region, and an interrogatory nucleotide can be the second, third, fourth, fifth, sixth, seventh, eighth, ninth, or tenth nucleotide of the 5' hybridization region. In some examples, the interrogatory nucleotide is the fourth, fifth, or sixth nucleotide from the 5' phosphate group of the circularizable probe. In some examples, the interrogatory nucleotide is the fifth nucleotide of the 5' hybridization region. In any of the embodiments herein, the 3' terminal nucleotide of the circularizable probe can be the first nucleotide of the 3' hybridization region, and an interrogatory nucleotide can be the second, third, fourth, fifth, sixth, seventh, eighth, ninth, or tenth nucleotide of the 3' hybridization region.

In any of the embodiments herein, the 5' hybridization region can be between about 5 and about 10 nucleotides shorter than the 3' hybridization region, and the interrogatory nucleotide can be the second, third, fourth, fifth, sixth, seventh, eighth, ninth, or tenth nucleotide of the 5' hybridization region.

In any of the embodiments herein, the circularizable probe or probe set can comprise one, two, three, four, or more ribonucleotides. In some embodiments, a circularizable probe or probe set disclosed herein can comprise one, two, three, four, or more ribonucleotides in a DNA backbone. In any of the embodiments herein, the one or more ribonucleotides can be at and/or near a ligatable 3' end of the circularizable probe or probe set. FIG. 1B shows an exemplary method using an asymmetric circularizable probe for detecting a region of interest (e.g., an SNP) in a target nucleic acid. One arm of the asymmetric circularizable probe comprises an internal interrogatory nucleotide (e.g., base specific for SNP detection). The circularizable probe may comprise a 3' RNA base, which is optional. The presence of a complementary base in the SNP location in the target nucleic acid favors stable hybridization of the circularizable probe and subsequent padlock circularization. In contrast, the interrogatory nucleotide-containing arm is less stably hybridized when there is a mismatch between the interrogatory nucleotide and the SNP location in the target nucleic acid. The interrogatory nucleotide-containing arm (e.g., the shorter arm as shown in the figure) can dissociate from the target nucleic acid and prevent circularization of the circularizable probe by a ligase having poor fidelity (e.g., on RNA templates), thus reducing false positive signals due to indiscriminating ligase activity.

### IV. LIGATION, AMPLIFICATION, AND DETECTION

In some embodiments, the methods of the present disclosure include the step of performing rolling circle amplification in the presence of a target nucleic acid of interest.

In some embodiments, the method comprises using a circular or circularizable construct (e.g., described in Section III) hybridized to the polynucleotides of interest to generate the target nucleic acid (e.g., comprising a sequence of the polynucleotide(s) of interest or one or more barcode sequences associated with the polynucleotide(s) of interest). In some embodiments, the RCA comprises a linear RCA. In some embodiments, the RCA comprises a branched RCA. In some embodiments, the RCA comprises a dendritic RCA. In some embodiments, the RCA comprises any combination of the foregoing. Any suitable methods and conditions for hybridization of probes, ligation, amplification, and detection may be used, e.g., as described in Sections II-IV.

In some embodiments, the circular construct is formed using ligation. In some embodiments, the circular construct is formed using template primer extension followed by ligation. In some embodiments, the circular construct is formed by providing an insert between ends to be ligated. In some embodiments, the circular construct is formed using a combination of any of the foregoing. In some embodiments, the ligation is a DNA-DNA templated ligation. In some embodiments, the ligation is an RNA-RNA templated ligation. In some embodiments, the ligation is an RNA-DNA templated ligation. In some embodiments, a splint is provided as a template for ligation.

In some embodiments, the circular construct is directly hybridized to the target nucleic acid. In some embodiments, the circular construct is formed from a circularizable probe (e.g., a padlock probe). In some embodiments, the circular construct is formed from a probe or probe set capable of DNA-templated ligation. See, e.g., U.S. Pat. 8,551,710. In some embodiments, the circular construct is formed from a probe or probe set capable of RNA-templated ligation. Exemplary RNA-templated ligation probes and methods are described in US 2020/0224244. In some embodiments, the circular construct is formed from a specific amplification of nucleic acids via intramolecular ligation (e.g., SNAIL) probe set. See, e.g., US 2019/0055594 or US 2021/0164039. In some embodiments, the circular construct is formed from a probe capable of proximity ligation, for instance a proximity ligation assay for RNA (e.g., PLAYR) probe set. See, e.g., US 2016/0108458. In some embodiments, the circular construct is indirectly hybridized to the target nucleic acid. In some embodiments, the circular construct is formed from a probe set capable of proximity ligation, for instance a proximity ligation *in situ* hybridization (PLISH) probe set. See, e.g., US 2020/0224243. Any suitable combination of the probe designs described herein can be used.

The nature of the ligation reaction depends on the structural components of the polynucleotides used to form the padlock or circular probe. In one embodiment, the polynucleotides comprise complementary docking regions that self-assemble the two or more polynucleotides into a padlock probe that is either ready for ligation because no gaps exist between the docking regions, or is ready for a fill-in process, which will then permit the ligation of the polynucleotides to form the circularizable probe. In another embodiment, the docking regions are complementary to a splint primer. In one embodiment, the splint primer is complementary to one pair of docking regions of two polynucleotides. In another embodiment, the splint primer is complementary to two pairs of docking regions. In one aspect of this embodiment, the splint primer has two regions of complementarity to the docking regions of the polynucleotides that form the circularizable probe. Typically, a splint probe of this embodiment will comprise a first docking region complementary sequence, a spacer, and a second docking region complementary sequence.

In some embodiments, a 3' end and a 5' end of the circularizable probe or probe set can be ligated using the target nucleic acid (e.g., RNA) as a template. In some embodiments, the 3' end and the 5' end are ligated without gap filling prior to ligation. In some embodiments, the ligation of the 3' end and the 5' end is preceded by gap filling. The gap may be 1, 2, 3, 4, 5, or more nucleotides.

In some embodiments, the circularizing step may comprise ligation selected from the group consisting of enzymatic ligation, chemical ligation, template dependent ligation, and/or template independent ligation. In any of the embodiments herein, the ligation can comprise using a ligase having an RNA-templated DNA ligase activity and/or an RNA-templated RNA ligase activity. In any of the embodiments herein, the ligation can comprise using a ligase selected from the group consisting of a Chlorella virus DNA ligase (PBCV DNA ligase), a T4 RNA ligase, a T4 DNA ligase, and a single-stranded DNA (ssDNA) ligase. In any of the embodiments herein, the ligation can comprise using a PBCV-1 DNA ligase or variant or derivative thereof and/or a T4 RNA ligase 2 (T4 Rnl2) or variant or derivative thereof.

In some embodiments, the method can further comprise prior to the circularizing step, a step of removing molecules of the circularizable probe or probe set that are not bound to the target nucleic acid from the biological sample. In any of the embodiments herein, the method can further comprise prior to the circularizing step, a step of removing molecules of the circularizable probe or probe set that are bound to the target nucleic acid but comprise one or more mismatches in the interrogatory sequence from the biological sample. In any of the embodiments herein, the method can further comprise prior to the circularizing step, a step of allowing circularizable probe molecules that are bound to the target nucleic acid but comprise one or more mismatches in the interrogatory sequence to dissociate from the target nucleic acid, while circularizable probe molecules comprising no mismatch in the interrogatory sequence remain bound to the target nucleic acid. In some embodiments, the method can further comprise prior to the circularizing step, a step of allowing circularizable probe molecules that are bound to the target nucleic acid but comprise one or more mismatches in the hybridization region comprising the interrogatory region to dissociate from the target nucleic acid, while circularizable probe molecules comprising no mismatch in the interrogatory sequence remain bound to the target nucleic acid. In any of the embodiments herein, under the same conditions, the molecules comprising one or more mismatches can be less stably bound to the target nucleic acid than the molecules comprising no mismatch in the interrogatory sequence. In any of the embodiments herein, the method can comprise one or more stringency washes. For instance, one or more stringency washes can be used to remove circularizable probe molecules that are not bound to the target nucleic acid, and/or circularizable probe molecules that are bound to the target nucleic acid but comprise one or more mismatches in the interrogatory sequence.

Following formation of, e.g., the circularized padlock probe or otherwise providing a circular probe, in some instances, an amplification primer is added. In other instances, the amplification primer is added with the circularizable probes. In some instances, the amplification primer may also be complementary to the target nucleic acid and the circularizable probe (e.g., a SNAIL probe). In some embodiments, a washing step is performed to remove any unbound probes, primers, etc. In some embodiments, the wash is a stringency wash. Washing steps can be performed at any point during the process to remove non-specifically bound probes, probes that have ligated, etc.

Upon addition of a DNA polymerase in the presence of appropriate dNTP precursors and other cofactors, the amplification primer is elongated by replication of multiple copies of the template (e.g., a concatemer of the template is generated). This amplification product can be detected using, e.g., the secondary and higher order probes and detection oligonucleotides described herein. In any of the embodiments herein, a sequence in the amplification product can be determined or otherwise analyzed, for example by using detectably labeled probes and imaging. The sequencing or analysis of the amplification products can comprise sequencing by hybridization, sequencing by ligation, and/or fluorescent *in situ* sequencing, and/or wherein the *in situ* hybridization comprises sequential fluorescent *in situ* hybridization. In some instances, sequencing using, e.g., the secondary and higher order probes and detection oligonucleotides described herein.

In any of the embodiments herein, the method can further comprise generating the product of the circularized probe *in situ* in the biological sample. In any of the embodiments herein, the product can be generated using rolling circle amplification (RCA). In any of the embodiments herein, the RCA can comprise a linear RCA, a branched RCA, a dendritic RCA, or any combination thereof. In any of the embodiments herein, the product can be generated using a polymerase selected from the group consisting of Phi29 DNA polymerase, Phi29-like DNA polymerase, M2 DNA polymerase, B103 DNA polymerase, GA-1 DNA polymerase, phi-PRD1 polymerase, Vent DNA polymerase, Deep Vent DNA polymerase, Vent (exo-) DNA polymerase, KlenTaq DNA polymerase, DNA polymerase I, Klenow fragment of DNA polymerase I, DNA polymerase III, T3 DNA polymerase, T4 DNA polymerase, T5 DNA polymerase, T7 DNA polymerase, Bst polymerase, rBST DNA polymerase, N29 DNA polymerase, TopoTaq DNA polymerase, T7 RNA polymerase, SP6 RNA polymerase, T3 RNA polymerase, and a variant or derivative thereof.

In any of the embodiments herein, the product can be immobilized in the biological sample. In any of the embodiments herein, the product can be crosslinked to one or more other molecules in the biological sample. Any suitable methods for tethering and immobilization may be used e.g., any described in Section II.

In any of the embodiments herein, the method can further comprise imaging the biological sample to detect the circularized probe or product thereof. In any of the embodiments herein, the imaging can comprise detecting a signal associated with a fluorescently labeled probe that directly or indirectly binds to a rolling circle amplification product of the circularized probe. In any of the embodiments herein, a sequence of the rolling circle amplification product can be analyzed *in situ* in the biological sample. In any of the embodiments herein, the sequence of the rolling circle amplification product can be analyzed by sequential hybridization, sequencing by hybridization, sequencing by ligation, sequencing by synthesis, sequencing by binding, or a combination thereof.

In any of the embodiments herein, the sequence of the rolling circle amplification product can comprise one or more barcode sequences or complements thereof. In any of the embodiments herein, the one or more barcode sequences can comprise a barcode sequence corresponding to the target nucleic acid. In any of the embodiments herein, the one or more barcode sequences can comprise a barcode sequence corresponding to the sequence of interest, such as variant(s) of a single nucleotide of interest.

In any of the embodiments herein, the detecting step can comprise contacting the biological sample with one or more detectably-labeled probes that directly or indirectly hybridize to the rolling circle amplification product, and dehybridizing the one or more detectably-labeled probes from the rolling circle amplification product. In any of the embodiments herein, the contacting and dehybridizing steps can be repeated with the one or more detectably-labeled probes and/or one or more other detectably-labeled probes that directly or indirectly hybridize to the rolling circle amplification product.

In any of the embodiments herein, the detecting step can comprise contacting the biological sample with one or more intermediate probes that directly or indirectly hybridize to the rolling circle amplification product, wherein the one or more intermediate probes are detectable using one or more detectably-labeled probes. In any of the embodiments herein, the detecting step can further comprise dehybridizing the one or more intermediate probes and/or the one or more detectably-labeled probes from the rolling circle amplification product. In any of the embodiments herein, the contacting and dehybridizing steps can be repeated with the one or more intermediate probes, the one or more detectably-labeled probes, one or more other intermediate probes, and/or one or more other detectably-labeled probes.

In some embodiments, the detection may be spatial, e.g., in two or three dimensions. In some embodiments, the detection may be quantitative, e.g., the amount or concentration of a primary nucleic acid probe (and of a target nucleic acid) may be determined. In some embodiments, the primary probes, secondary probes, higher order probes, and/or detectably labeled probes may comprise any of a variety of entities able to hybridize a nucleic acid, e.g., DNA, RNA, LNA, and/or PNA, etc., depending on the application.

In some embodiments, disclosed herein is a multiplexed assay where multiple targets (e.g., nucleic acids such as genes or RNA transcripts, or protein targets) are probed with multiple primary probes (e.g., padlock primary probes), and optionally multiple secondary probes hybridizing to the primary barcodes (or complementary sequences thereof) are all hybridized at once, followed by sequential secondary barcode detection and decoding of the signals. In some embodiments, detection of barcodes or subsequences of the barcode can occur in a cyclic manner.

In some embodiments, a method for analyzing a region of interest in a target nucleic acid is a multiplexed assay where multiple probes (e.g., padlock probes) are used to detect multiple regions of interest simultaneously (e.g., variations at the same location of a target nucleic acid and/or SNPs in various locations). In some embodiments, one or more detections of one or more regions of interest may occur simultaneously. In some embodiments, one or more detections of one or more regions of interest may occur sequentially. In some embodiments, multiple circularizable probes of the same circularizable probe design are used to detect one or more regions of interest, using different barcodes associated with each region of interest. In some embodiments, multiple circularizable probes of different circularizable probe design are used to detect one or more regions of interest, using different barcodes (e.g., each barcode associated with a target nucleic acid or sequence thereof). In some embodiments, the one or more regions of interest are localized on the same molecule (e.g., RNA or DNA). In alternative embodiments, the one or more single nucleotides of interest are localized on different molecules.

In some embodiments, disclosed herein is a method for analyzing a biological sample, comprising contacting the biological sample with a circularizable probe or probe set, wherein the circularizable probe comprises a 5' hybridization region and a 3' hybridization region that hybridize to adjacent first and second target regions, respectively, in a target RNA in the biological sample, where the 5' hybridization region and the 3' hybridization region are different in length. In some embodiments, the shorter one of the 5' hybridization region and the 3' hybridization region comprises an interrogatory nucleotide complementary to a single nucleotide of interest in the first or second target region, respectively. For instance, the 5' hybridization region can be the shorter one and can comprise the interrogatory nucleotide. In other examples, the 3' hybridization region can be the shorter one and can comprise the interrogatory nucleotide. In any of the embodiments herein, the interrogatory nucleotide may not be a 5' or 3' terminal nucleotide.

In any of the embodiments herein, the interrogatory nucleotide can be an internal nucleotide in the 5' hybridization region or the 3' hybridization region. In any of the embodiments herein, the interrogatory nucleotide may not comprise a ligatable end. In any of the embodiments herein, the interrogatory nucleotide may not be a nucleotide which, with or without processing of the circularizable probe or probe set, participates in a subsequent ligation reaction, for example, a ligation that is catalyzed by a ligase having RNA-splinted ligase activity. In some embodiments, upon processing of the circularizable probe or probe set hybridized to the target nucleic acid, the interrogatory nucleotide may be exposed and participate in a subsequent ligation reaction catalyzed by a ligase having RNA-splinted ligase activity. In some embodiments, a ligatable 5' phosphate group or 3' hydroxyl group of the interrogatory nucleotide may be exposed upon processing of the circularizable probe or probe set. In any of the embodiments herein, the interrogatory nucleotide can be a ribonucleotide, a deoxyribonucleotide, or a variant or derivative thereof.

In some embodiments, a probe disclosed herein (e.g., a circularizable probe) can comprise a flap. In some embodiments, a circularizable probe disclosed herein (e.g., a padlock probe) can comprise a 5' flap which may be recognized by a structure-specific cleavage enzyme, e.g., an enzyme capable of recognizing the junction between single-stranded 5' overhang and a DNA duplex, and cleaving the single-stranded overhang. It will be understood that the branched three-strand structure which is the substrate for the structure-specific cleavage enzyme may be formed by 5' end of one probe part and the 3' end of another probe part when both have hybridized to the target nucleic acid molecule, as well as by the 5' and 3' ends of a one-part probe. Enzymes suitable for such cleavage include Flap endonucleases (FENS), which are a class of enzymes having endonucleolytic activity and being capable of catalyzing the hydrolytic cleavage of the phosphodiester bond at the junction of single- and double-stranded DNA. Thus, in some embodiment, cleavage of the additional sequence 5' to the first target-specific binding site is performed by a structure-specific cleavage enzyme, e.g. a Flap endonuclease. Suitable Flap endonucleases are described in Ma et al. 2000. JBC 275, 24693- 24700 and in US 2020/0224244 and may include *P. furiosus* (Pfu), *A. fulgidus* (Afu), *M. jannaschii* (Mja) or *M. thermoautotrophicum* (Mth). In other embodiments an enzyme capable of recognizing and degrading a single-stranded oligonucleotide having a free 5' end may be used to cleave an additional sequence (5' flap) from a structure as described above. Thus, an enzyme having 5' nuclease activity may be used to cleave a 5' additional sequence. Such 5' nuclease activity may be 5' exonuclease and/or 5' endonuclease activity. A 5' nuclease enzyme is capable of recognizing a free 5' end of a single-stranded oligonucleotide and degrading said single-stranded oligonucleotide. A 5' exonuclease degrades a single-stranded oligonucleotide having a free 5' end by degrading the oligonucleotide into constituent mononucleotides from its 5' end. A 5' endonuclease activity may cleave the 5' flap sequence internally at one or more nucleotides. Further, a 5' nuclease activity may take place by the enzyme traversing the single-stranded oligonucleotide to a region of duplex once it has recognized the free 5' end, and cleaving the single-stranded region into larger constituent nucleotides (e.g. dinucleotides or trinucleotides), or cleaving the entire 5' single- stranded region, e.g. as described in Lyamichev et al. 1999. PNAS 96, 6143-6148 for Taq DNA polymerase and the 5' nuclease thereof. Preferred enzymes having 5' nuclease activity include Exonuclease VIII, or a native or recombinant DNA polymerase enzyme from *Thermus aquaticus* (Taq), *Thermus thermophilus* or *Thermus flavus,* or the nuclease domain therefrom.

In any of the embodiments herein, the method can further comprise ligating the ends of the circularizable probe hybridized to the target RNA to form a circularized circularizable probe. In any of the embodiments herein, the method can further comprise generating a rolling circle amplification product of the circularized circularizable probe. In any of the embodiments herein, the method can further comprise detecting a signal associated with the rolling circle amplification product in the biological sample.

In some embodiments, disclosed herein is a method for analyzing a biological sample, comprising contacting the biological sample with a circularizable probe or probe set, wherein: the circularizable probe or probe set comprises a 5' hybridization region and a 3' hybridization region that hybridize to adjacent first and second target regions, respectively, in a target RNA in the biological sample; the 5' hybridization region is shorter than the 3' hybridization region and comprises an interrogatory nucleotide complementary to a single nucleotide of interest in the first target region of a first molecule of the target RNA, wherein a second molecule of the target RNA comprises a mismatch with the interrogatory nucleotide; and the interrogatory nucleotide is any one of the second to the tenth nucleotides from the 5' end of the circularizable probe. In some embodiments, the method further comprises allowing the circularizable probe or a portion thereof (e.g., the 5' hybridization region) to dissociate from the second molecule of the target RNA, wherein under the same conditions, the circularizable probe (e.g., including the 5' hybridization region) remains hybridized to the first molecule of the target RNA. In some embodiments, the method further comprises ligating the ends of the circularizable probe hybridized to the first molecule of the target RNA to form a circularized circularizable probe; generating a rolling circle amplification product of the circularized circularizable probe; and detecting a signal associated with the rolling circle amplification product in the biological sample. In any of the preceding embodiments, the circularizable probe hybridized to the second molecule of the target RNA can be destabilized and/or removed from the biological sample while the circularizable probe remains hybridized to the first molecule of the target RNA, such that a circularized circularizable probe hybridized to the second molecule of the target RNA is not formed. In any of the preceding embodiments, a signal associated with the second molecule of the target RNA may not be detected, such that a signal associated with the rolling circle amplification product is indicative of the first molecule and not the second molecule of the target RNA in the biological sample.

In some embodiments, provided herein is a method for analyzing a biological sample, comprising contacting the biological sample with a first circularizable probe or probe set and a second circularizable probe or probe set. In some embodiments, the first circularizable probe or probe set comprises a 5' hybridization region and a 3' hybridization region that hybridize to adjacent first and second target regions, respectively, in a target nucleic acid such as a target RNA, and the 5' hybridization region and the 3' hybridization region are different in length. In any of the embodiments herein, the shorter one of the 5' hybridization region and the 3' hybridization region can comprise a first interrogatory nucleotide complementary to a first variant of a single nucleotide of interest in the first or second target region, respectively. In any of the embodiments herein, the first interrogatory nucleotide can be any one of the second to the tenth nucleotides from the 5' end of the first circularizable probe or probe set. In any of the embodiments herein, the second circularizable probe or probe set can comprise the 5' hybridization region and the 3' hybridization region, except that the second circularizable probe or probe set can comprise a second interrogatory nucleotide complementary to a second variant of the single nucleotide of interest. In any of the embodiments herein, the method can further comprise circularizing the ends of the first circularizable probe and/or the ends of the second circularizable probe hybridized to the target RNA; generating a first rolling circle amplification product of the circularized first circularizable probe or probe set and/or a second rolling circle amplification product of the circularized second circularizable probe or probe set; and detecting signals associated with the first and/or second rolling circle amplification products, thereby detecting the first and/or second variants of the single nucleotide of interest in the biological sample. In any of the embodiments herein, the first circularizable probe or probe set can comprise a first barcode sequence corresponding to the first variant and the second circularizable probe or probe set can comprise a second barcode sequence corresponding to the second variant. In any of the embodiments herein, a signal detected in the detecting step at a given location in the biological sample can be associated with the first barcode sequence (or complement thereof) or the second barcode sequence (or complement thereof). As such, the signal at the given location can be associated with the first or second variant of the single nucleotide of interest. In any of the embodiments herein, the method can further comprise imaging the biological sample to detect the first or second variant of the single nucleotide of interest at multiple locations *in situ,* for instance, by detecting the first barcode sequence (or complement thereof) or the second barcode sequence (or complement thereof).

**FIG.** 2 depicts an example of multiplexed *in situ* detection of two SNPs - SNP1 (sequence variation G/C) and SNP2 (sequence variation G/A) - in a sample. SNP1 and SNP2 are different SNPs and may be in different gene loci, e.g., on different chromosomes. DNA and/or RNA molecules in the sample may be detected. The barcoded circularizable probes or probe sets (e.g., padlock probes) can be hybridized to the sample at the same time or sequentially. The sequence variations at the same SNP position can be differentially barcoded and detected, e.g., SNP1(G) and SNP1(C) each is associated with a different barcode. The single nucleotide of interest in different SNPs can also be differentially barcoded and detected, e.g., SNP1(G) and SNP2(G) each is associated with a different barcode.

In some aspects, the provided methods involve analyzing, e.g., detecting or determining, one or more sequences present in the polynucleotides and/or in a product or derivative thereof, such as in an amplified circularizable probe or probe set. In some embodiments, the circularizable probe or probe set or product thereof can be detected by providing detection probes, such as probes for performing a chain reaction that forms an amplification product, e.g., HCR. In some embodiments, the amplification is non-enzymatic. In some embodiments, the analysis comprises determining the sequence of all or a portion of the amplification product. In some embodiments, the analysis comprises detecting a sequence present in the amplification product. In some embodiments, the sequence of all or a portion of the amplification product is indicative of the identity of a region of interest in a target nucleic acid. In other embodiments, the provided methods involve analyzing, e.g., detecting or determining, one or more sequences present in the polynucleotide probes (e.g., a barcode sequence present in a circularizable probe or probe set or product thereof).

In some embodiments, the methods comprise sequencing all or a portion of the amplification product, such as one or more barcode sequences present in the amplification product. In some embodiments, the analysis and/or sequence determination comprises sequencing all or a portion of the amplification product or the probe(s) and/or in situ hybridization to the amplification product or the probe(s). In some embodiments, the sequencing step involves sequencing by hybridization, sequencing by ligation, and/or fluorescent *in situ* sequencing, hybridization-based *in situ* sequencing and/or wherein the *in situ* hybridization comprises sequential fluorescent *in situ* hybridization. In some embodiments, the analysis and/or sequence determination comprises detecting a polymer generated by a hybridization chain reaction (HCR) reaction, see e.g., US 2017/0009278 for exemplary probes and HCR reaction components. In some embodiments, the detection or determination comprises hybridizing to the amplification product a detection oligonucleotide labeled with a fluorophore, an isotope, a mass tag, or a combination thereof. In some embodiments, the detection or determination comprises imaging the amplification product. In some embodiments, the target nucleic acid is an mRNA in a tissue sample, and the detection or determination is performed when the target nucleic acid and/or the amplification product is *in situ* in the tissue sample.

In some aspects, the provided methods comprise imaging the amplification product (e.g., amplicon) and/or one or more portions of the polynucleotides, for example, via binding of the detection probe and detecting the detectable label. In some embodiments, the detection probe comprises a detectable label that can be measured and quantitated. The terms "label" and "detectable label" comprise a directly or indirectly detectable moiety that is associated with (e.g., conjugated to) a molecule to be detected, e.g., a detectable probe, comprising, but not limited to, fluorophores, radioactive isotopes, fluorescers, chemiluminescers, enzymes, enzyme substrates, enzyme cofactors, enzyme inhibitors, chromophores, dyes, metal ions, metal sols, ligands (e.g., biotin or haptens) and the like.

The term "fluorophore" comprises a substance or a portion thereof that is capable of exhibiting fluorescence in the detectable range. Particular examples of labels that may be used in accordance with the provided embodiments comprise, but are not limited to phycoerythrin, Alexa dyes, fluorescein, YPet, CyPet, Cascade blue, allophycocyanin, Cy3, Cy5, Cy7, rhodamine, dansyl, umbelliferone, Texas red, luminol, acradimum esters, biotin, green fluorescent protein (GFP), enhanced green fluorescent protein (EGFP), yellow fluorescent protein (YFP), enhanced yellow fluorescent protein (EYFP), blue fluorescent protein (BFP), red fluorescent protein (RFP), firefly luciferase, Renilla luciferase, NADPH, beta-galactosidase, horseradish peroxidase, glucose oxidase, alkaline phosphatase, chloramphenical acetyl transferase, and urease.

Fluorescence detection in tissue samples can often be hindered by the presence of strong background fluorescence. "Autofluorescence" is the general term used to distinguish background fluorescence (that can arise from a variety of sources, including aldehyde fixation, extracellular matrix components, red blood cells, lipofuscin, and the like) from the desired immunofluorescence from the fluorescently labeled antibodies or probes. Tissue autofluorescence can lead to difficulties in distinguishing the signals due to fluorescent antibodies or probes from the general background. In some embodiments, a method disclosed herein utilizes one or more agents to reduce tissue autofluorescence, for example, Autofluorescence Eliminator (Sigma/EMD Millipore), TrueBlack Lipofuscin Autofluorescence Quencher (Biotium), MaxBlock Autofluorescence Reducing Reagent Kit (MaxVision Biosciences), and/or a very intense black dye (e.g., Sudan Black, or comparable dark chromophore).

In some embodiments, a detectable probe containing a detectable label can be used to detect one or more probe(s) and/or amplification products (e.g., amplicon) described herein. In some embodiments, the methods involve incubating the detectable probe containing the detectable label with the sample, washing unbound detectable probe, and detecting the label, e.g., by imaging. In some embodiments, a detectable probe may bind directly or indirectly to the probe and/or amplification products.

Examples of detectable labels comprise but are not limited to various radioactive moieties, enzymes, prosthetic groups, fluorescent markers, luminescent markers, bioluminescent markers, metal particles, protein-protein binding pairs and protein-antibody binding pairs. Examples of fluorescent proteins comprise, but are not limited to, yellow fluorescent protein (YFP), green fluorescence protein (GFP), cyan fluorescence protein (CFP), umbelliferone, fluorescein, fluorescein isothiocyanate, rhodamine, dichlorotriazinylamine fluorescein, dansyl chloride and phycoerythrin.

Examples of bioluminescent markers comprise, but are not limited to, luciferase (e.g., bacterial, firefly and click beetle), luciferin, aequorin and the like. Examples of enzyme systems having visually detectable signals comprise, but are not limited to, galactosidases, glucorimidases, phosphatases, peroxidases and cholinesterases. Identifiable markers also comprise radioactive compounds such as ¹²⁵I, ³⁵S, ¹⁴C, or ³H. Identifiable markers are commercially available from a variety of sources.

Examples of fluorescent labels and nucleotides and/or polynucleotides conjugated to such fluorescent labels comprise those described in, for example, Hoagland, Handbook of Fluorescent Probes and Research Chemicals, Ninth Edition (Molecular Probes, Inc., Eugene, 2002); Keller and Manak, DNA Probes, 2nd Edition (Stockton Press, New York, 1993); Eckstein, editor, Oligonucleotides and Analogues: A Practical Approach (IRL Press, Oxford, 1991); and Wetmur, Critical Reviews in Biochemistry and Molecular Biology, 26:227- 259 (1991). In some embodiments, exemplary techniques and methods methodologies applicable to the provided embodiments comprise those described in, for example, US 4,757,141, US 5,151,507 and US 5,091,519. In some embodiments, one or more fluorescent dyes are used as labels for labeled target sequences, for example, as described in US 5,188,934 (4,7-dichlorofluorescein dyes); US 5,366,860 (spectrally resolvable rhodamine dyes); US 5,847,162 (4,7- dichlororhodamine dyes); US 4,318,846 (ether-substituted fluorescein dyes); US 5,800,996 (energy transfer dyes); US 5,066,580 (xanthine dyes); and US 5,688,648 (energy transfer dyes). Labelling can also be carried out with quantum dots, as described in US 6,322,901, US 6,576,291, US 6,423,551, US 6,251,303, US 6,319,426, US 6,426,513, US 6,444,143, US 5,990,479, US 6,207,392, US 2002/0045045 and US 2003/0017264. In some embodiments, a fluorescent label comprises a signaling moiety that conveys information through the fluorescent absorption and/or emission properties of one or more molecules. Exemplary fluorescent properties comprise fluorescence intensity, fluorescence lifetime, emission spectrum characteristics and energy transfer.

Examples of commercially available fluorescent nucleotide analogues readily incorporated into nucleotide and/or polynucleotide sequences comprise, but are not limited to, Cy3-dCTP, Cy3-dUTP, Cy5-dCTP, Cy5-dUTP (Amersham Biosciences, Piscataway, N.J.), fluorescein- !2-dUTP, tetramethylrhodamine-6-dUTP, TEXAS RED^{™}-5-dUTP, CASCADE BLUE^{™}-7-dUTP, BODIPY TMFL-14-dUTP, BODIPY TMR-14-dUTP, BODIPY TMTR-14-dUTP, RHOD AMINE GREEN^{™}-5-dUTP, OREGON GREENR^{™} 488-5-dUTP, TEXAS RED^{™}-l2-dUTP, BODIPY^{™} 630/650-14-dUTP, BODIPY^{™} 650/665-14-dUTP, ALEXA FLUOR^{™} 488-5-dUTP, ALEXA FLUOR^{™} 532-5-dUTP, ALEXA FLUOR^{™} 568-5-dUTP, ALEXA FLUOR^{™} 594-5-dUTP, ALEXA FLUOR^{™} 546-14-dUTP, fluorescein- 12-UTP, tetramethylrhodamine-6-UTP, TEXAS RED^{™}-5- UTP, mCherry, CASCADE BLUE^{™}-7-UTP, BODIPY^{™} FL-14-UTP, BODIPY TMR- 14-UTP, BODIPY^{™} TR-14-UTP, RHOD AMINE GREEN^{™}-5-UTP, ALEXA FLUOR^{™} 488-5-UTP, and ALEXA FLUOR^{™} 546-14-UTP (Molecular Probes, Inc. Eugene, Oreg.). Methods for custom synthesis of nucleotides having other fluorophores can include those described in Henegariu et al. (2000) Nature Biotechnol. 18:345.

Other fluorophores available for post-synthetic attachment comprise, but are not limited to, ALEXA FLUOR^{™} 350, ALEXA FLUOR^{™} 532, ALEXA FLUOR^{™} 546, ALEXA FLUOR^{™} 568, ALEXA FLUOR^{™} 594, ALEXA FLUOR^{™} 647, BODIPY 493/503, BODIPY FL, BODIPY R6G, BODIPY 530/550, BODIPY TMR, BODIPY 558/568, BODIPY 558/568, BODIPY 564/570, BODIPY 576/589, BODIPY 581/591, BODIPY 630/650, BODIPY 650/665, Cascade Blue, Cascade Yellow, Dansyl, lissamine rhodamine B, Marina Blue, Oregon Green 488, Oregon Green 514, Pacific Blue, rhodamine 6G, rhodamine green, rhodamine red, tetramethyl rhodamine, Texas Red (available from Molecular Probes, Inc., Eugene, Oreg.), Cy2, Cy3.5, Cy5.5, and Cy7 (Amersham Biosciences, Piscataway, N.J.). FRET tandem fluorophores may also be used, comprising, but not limited to, PerCP-Cy5.5, PE-Cy5, PE-Cy5.5, PE-Cy7, PE-Texas Red, APC-Cy7, PE-Alexa dyes (610, 647, 680), and APC-Alexa dyes.

In some cases, metallic silver or gold particles may be used to enhance signal from fluorescently labeled nucleotide and/or polynucleotide sequences (Lakowicz et al. (2003) Bio Techniques 34:62).

Biotin, or a derivative thereof, may also be used as a label on a nucleotide and/or a polynucleotide sequence, and subsequently bound by a detectably labeled avidin/streptavidin derivative (e.g., phycoerythrin-conjugated streptavidin), or a detectably labeled anti-biotin antibody. Digoxigenin may be incorporated as a label and subsequently bound by a detectably labeled anti-digoxigenin antibody (e.g., fluoresceinated anti-digoxigenin). An aminoallyl-dUTP residue may be incorporated into a polynucleotide sequence and subsequently coupled to an N-hydroxy succinimide (NHS) derivatized fluorescent dye. In general, any member of a conjugate pair may be incorporated into a detection polynucleotide provided that a detectably labeled conjugate partner can be bound to permit detection. In some embodiments, an antibody comprises an antibody molecule of any class, or any sub-fragment thereof, such as a Fab.

Other suitable labels for a polynucleotide sequence may comprise fluorescein (FAM), digoxigenin, dinitrophenol (DNP), dansyl, biotin, bromodeoxyuridine (BrdU), hexahistidine (6xHis), and phosphor-amino acids (e.g., P-tyr, P-ser, P-thr). In some embodiments the following hapten/antibody pairs are used for detection, in which each of the antibodies is derivatized with a detectable label: biotin/a-biotin, digoxigenin/a- digoxigenin, dinitrophenol (DNP)/a-DNP, 5-Carboxyfluorescein (FAM)/a-FAM.

In some embodiments, a nucleotide and/or an polynucleotide sequence can be indirectly labeled, especially with a hapten that is then bound by a capture agent, e.g., as disclosed in US 5,344,757, US 5,702,888, US 5,354,657, US 5,198,537 and US 4,849,336, and US 5,073,562. Many different hapten-capture agent pairs are available for use. Exemplary haptens comprise, but are not limited to, biotin, des-biotin and other derivatives, dinitrophenol, dansyl, fluorescein, Cy5, and digoxigenin. For biotin, a capture agent may be avidin, streptavidin, or antibodies. Antibodies may be used as capture agents for the other haptens (many dye-antibody pairs being commercially available, e.g., Molecular Probes, Eugene, Oreg.).

In some aspects, the detecting involves using detection methods such as flow cytometry; sequencing; probe binding and electrochemical detection; pH alteration; catalysis induced by enzymes bound to DNA tags; quantum entanglement; Raman spectroscopy; terahertz wave technology; and/or scanning electron microscopy. In some aspects, the flow cytometry is mass cytometry or fluorescence-activated flow cytometry. In some aspects, the detecting comprises performing microscopy, scanning mass spectrometry or other imaging techniques described herein. In such aspects, the detecting comprises determining a signal, e.g., a fluorescent signal.

In some aspects, the detection (comprising imaging) is carried out using any of a number of different types of microscopy, e.g., confocal microscopy, two-photon microscopy, light-field microscopy, intact tissue expansion microscopy, and/or CLARITY^{™}-optimized light sheet microscopy (COLM).

In some embodiments, fluorescence microscopy is used for detection and imaging of the detection probe. In some aspects, a fluorescence microscope is an optical microscope that uses fluorescence and phosphorescence instead of, or in addition to, reflection and absorption to study properties of organic or inorganic substances. In fluorescence microscopy, a sample is illuminated with light of a wavelength which excites fluorescence in the sample. The fluoresced light, which is usually at a longer wavelength than the illumination, is then imaged through a microscope objective. Two filters may be used in this technique; an illumination (or excitation) filter which ensures the illumination is near monochromatic and at the correct wavelength, and a second emission (or barrier) filter which ensures none of the excitation light source reaches the detector. Alternatively, these functions may both be accomplished by a single dichroic filter. The "fluorescence microscope" comprises any microscope that uses fluorescence to generate an image, whether it is a more simple set up like an epifluorescence microscope, or a more complicated design such as a confocal microscope, which uses optical sectioning to get better resolution of the fluorescent image.

In some embodiments, confocal microscopy is used for detection and imaging of the detection probe. Confocal microscopy uses point illumination and a pinhole in an optically conjugate plane in front of the detector to eliminate out-of-focus signal. As only light produced by fluorescence very close to the focal plane can be detected, the image's optical resolution, particularly in the sample depth direction, is much better than that of wide-field microscopes. However, as much of the light from sample fluorescence is blocked at the pinhole, this increased resolution is at the cost of decreased signal intensity - so long exposures are often required. As only one point in the sample is illuminated at a time, 2D or 3D imaging requires scanning over a regular raster (e.g., a rectangular pattern of parallel scanning lines) in the specimen. The achievable thickness of the focal plane is defined mostly by the wavelength of the used light divided by the numerical aperture of the objective lens, but also by the optical properties of the specimen. The thin optical sectioning possible makes these types of microscopes particularly good at 3D imaging and surface profiling of samples. CLARITY^{™}-optimized light sheet microscopy (COLM) provides an alternative microscopy for fast 3D imaging of large clarified samples. COLM interrogates large immunostained tissues, permits increased speed of acquisition and results in a higher quality of generated data.

Other types of microscopy that can be employed comprise bright field microscopy, oblique illumination microscopy, dark field microscopy, phase contrast, differential interference contrast (DIC) microscopy, interference reflection microscopy (also known as reflected interference contrast, or RIC), single plane illumination microscopy (SPIM), super-resolution microscopy, laser microscopy, electron microscopy (EM), Transmission electron microscopy (TEM), Scanning electron microscopy (SEM), reflection electron microscopy (REM), Scanning transmission electron microscopy (STEM) and low-voltage electron microscopy (LVEM), scanning probe microscopy (SPM), atomic force microscopy (ATM), ballistic electron emission microscopy (BEEM), chemical force microscopy (CFM), conductive atomic force microscopy (C- AFM), electrochemical scanning tunneling microscope (ECSTM), electrostatic force microscopy (EFM), fluidic force microscope (FluidFM), force modulation microscopy (FMM), feature-oriented scanning probe microscopy (FOSPM), kelvin probe force microscopy (KPFM), magnetic force microscopy (MFM), magnetic resonance force microscopy (MRFM), near-field scanning optical microscopy (NSOM) (or SNOM, scanning near-field optical microscopy, SNOM, Piezoresponse Force Microscopy (PFM), PSTM, photon scanning tunneling microscopy (PSTM), PTMS, photothermal microspectroscopy/ microscopy (PTMS), SCM, scanning capacitance microscopy (SCM), SECM, scanning electrochemical microscopy (SECM), SGM, scanning gate microscopy (SGM), SHPM, scanning Hall probe microscopy (SHPM), SICM, scanning ion-conductance microscopy (SICM), SPSM spin polarized scanning tunneling microscopy (SPSM), SSRM, scanning spreading resistance microscopy (SSRM), SThM, scanning thermal microscopy (SThM), STM, scanning tunneling microscopy (STM), STP, scanning tunneling potentiometry (STP), SVM, scanning voltage microscopy (SVM), and synchrotron x-ray scanning tunneling microscopy (SXSTM), and intact tissue expansion microscopy (exM).

In some embodiments, sequencing can be performed *in situ. In situ* sequencing typically involves incorporation of a labeled nucleotide (e.g., fluorescently labeled mononucleotides or dinucleotides) in a sequential, template-dependent manner or hybridization of a labeled primer (e.g., a labeled random hexamer) to a nucleic acid template such that the identities (e.g., nucleotide sequence) of the incorporated nucleotides or labeled primer extension products can be determined, and consequently, the nucleotide sequence of the corresponding template nucleic acid. Aspects of in situ sequencing are described, for example, in Mitra et al., (2003) Anal. Biochem. 320, 55-65, and Lee et al., (2014) Science, 343(6177), 1360-1363. In addition, examples of methods and systems for performing in situ sequencing are described in US 2016/0024555, US 2019/0194709, and in US 10,138,509, US 10,494,662 and US. 10,179,932. Exemplary techniques for in situ sequencing comprise, but are not limited to, STARmap (described for example in Wang et al., (2018) Science, 361(6499) 5691), MERFISH (described for example in Moffitt, (2016) Methods in Enzymology, 572, 1-49), hybridization-based *in situ* sequencing (HybISS) (described for example in Gyllborg et al., Nucleic Acids Res (2020) 48(19):e112, and FISSEQ (described for example in US 2019/0032121).

In some embodiments, sequencing can be performed by sequencing-by-synthesis (SBS). In some embodiments, a sequencing primer is complementary to sequences at or near the one or more barcode(s). In such embodiments, sequencing-by-synthesis can comprise reverse transcription and/or amplification in order to generate a template sequence from which a primer sequence can bind. Exemplary SBS methods comprise those described for example, but not limited to, US 2007/0166705, US 2006/0188901, US 7,057,026, US 2006/0240439, US 2006/0281109, US 2011/005986, US 2005/0100900, US 9,217,178, US 2009/0118128, US 2012/0270305, US 2013/0260372, and US 2013/0079232.

In some embodiments, sequencing can be performed using single molecule sequencing by ligation. Such techniques utilize DNA ligase to incorporate oligonucleotides and identify the incorporation of such oligonucleotides. The oligonucleotides typically have different labels that are correlated with the identity of a particular nucleotide in a sequence to which the oligonucleotides hybridize. Aspects and features involved in sequencing by ligation are described, for example, in Shendure et al. Science (2005), 309: 1728-1732, and in US 5,599,675; US 5,750,341; US 6,969,488; US 6,172,218; US and 6,306,597.

In some embodiments, nucleic acid hybridization can be used for sequencing. These methods utilize labeled nucleic acid decoder probes that are complementary to at least a portion of a barcode sequence. Multiplex decoding can be performed with pools of many different probes with distinguishable labels. Non-limiting examples of nucleic acid hybridization sequencing are described for example in US 8,460,865, and in Gunderson et al., Genome Research 14:870-877 (2004).

In some embodiments, real-time monitoring of DNA polymerase activity can be used during sequencing. For example, nucleotide incorporations can be detected through fluorescence resonance energy transfer (FRET), as described for example in Levene et al., Science (2003), 299, 682-686, Lundquist et al., Opt. Lett. (2008), 33, 1026-1028, and Korlach et al., Proc. Natl. Acad. Sci. USA (2008), 105, 1176-1181.

In some aspects, the analysis and/or sequence determination can be carried out at room temperature for best preservation of tissue morphology with low background noise and error reduction. In some embodiments, the analysis and/or sequence determination comprises eliminating error accumulation as sequencing proceeds.

In some embodiments, the analysis and/or sequence determination involves washing to remove unbound polynucleotides, thereafter revealing a fluorescent product for imaging.

In some embodiments, the barcodes of the probes (e.g., the circularizable probes or probe sets disclosed herein) or complements or products thereof are targeted by detectably labeled detection oligonucleotides, such as fluorescently labeled oligonucleotides. In some embodiments, one or more decoding schemes are used to decode the signals, such as fluorescence, for sequence determination. In any of the embodiments herein, barcodes (e.g., primary and/or secondary barcode sequences) can be analyzed (e.g., detected or sequenced) using any suitable methods or techniques described in connection with RNA sequential probing of targets (RNA SPOTs), sequential fluorescent *in situ* hybridization (seqFISH), single-molecule fluorescent *in situ* hybridization (smFISH), multiplexed error-robust fluorescence *in situ* hybridization (MERFISH), hybridization-based *in situ* sequencing (HybISS), *in situ* sequencing, targeted *in situ* sequencing, fluorescent *in situ* sequencing (FISSEQ) (described for example in US 2019/0032121), or spatially-resolved transcript amplicon readout mapping (STARmap). In some embodiments, the methods provided herein comprise analyzing the barcodes by sequential hybridization and detection with a plurality of labelled probes (e.g., detection oligonucleotides). Barcodes can be analyzed (e.g., detected or sequenced) using exemplary decoding schemes as described in Eng et al., "Transcriptome-scale Super-Resolved Imaging in Tissues by RNA SeqFISH+," Nature 568(7751):235-239 (2019); Chen et al., "Spatially resolved, highly multiplexed RNA profiling in single cells," Science; 348(6233):aaa6090 (2015); Gyllborg et al., Nucleic Acids Res (2020) 48(19):e112; US 10,457,980 B2; US 2016/0369329 A1; WO 2018/026873 A1; and US 2017/0220733 A1. In some embodiments, these assays enable signal amplification, combinatorial decoding, and error correction schemes at the same time.

In some embodiments, sequence analysis of nucleic acids (e.g., nucleic acids such as RCA products comprising barcode sequences) can be performed by sequential hybridization (e.g., sequencing by hybridization and/or sequential *in situ* fluorescence hybridization). Sequential fluorescence hybridization can involve sequential hybridization of detection probes comprising an oligonucleotide and a detectable label. In some embodiments, a method disclosed herein comprises sequential hybridization of the detectable probes disclosed herein, including detectably labeled probes (e.g., fluorophore conjugated oligonucleotides) and/or probes that are not detectably labeled *per se* but are capable of binding (e.g., via nucleic acid hybridization) and being detected by detectably labeled probes. Exemplary methods comprising sequential fluorescence hybridization of detectable probes are described in US 2019/0161796, US 2020/0224244, US 2022/0010358, US 2021/0340618, and WO 2021/138676.

In some embodiments, described herein is a method of localized detection of multiple target nucleic acids and/or multiple sequences of interest (e.g., SNP) in the same or different target nucleic acids in a sample, wherein each target nucleic acid and/or sequence of interest is targeted by a circular or circularizable primary probe specific for said target nucleic acid and/or sequence of interest, and the circularizable primary probe comprises asymmetric hybridization regions (e.g., a longer 3' hybridization region or longer 5' hybridization region) and can be circularized upon hybridization to the target nucleic acid. Each of a plurality of circular or circularized probes can comprise a barcode sequence corresponding to a particular target nucleic acid and/or a barcode sequence corresponding to a particular sequence of interest (e.g., SNP). The plurality of circular or circularized probes can bind to molecules of the target nucleic acid at multiple locations in the sample, and can be amplified *in situ* by rolling circle amplification (RCA) to produce a rolling circle product (RCP). Each RCP can comprise multiple complementary copies of the barcode sequence corresponding to a particular target nucleic acid and/or a barcode sequence corresponding to a particular sequence of interest (e.g., SNP), wherein each barcode sequence can be decoded in multiple sequential decoding cycles each using hybridization probes (e.g., intermediate probes such as L-shaped probes) which hybridize to the complementary copies of the barcode sequences in an RCP and allow detectable signals to be generated. Signals associated with the barcode sequence in the sequential decoding cycles together yield a signal code sequence which can be used to identify the barcode sequence and its corresponding target nucleic acid sequence and/or sequence of interest (e.g., SNP).

In some embodiments, provided herein is a method of analyzing a sample, comprising: a) producing an amplification product such as RCA product in the sample, the amplification product comprising multiple copies of a barcode sequence (e.g., one of a plurality of different barcode sequences), wherein the barcode sequence is associated with a target nucleic acid sequence and/or a sequence of interest (e.g., SNP) and is assigned a signal code sequence, and wherein the sample is a cell or tissue sample; b) contacting the sample with a first intermediate probe and a first detectable probe to generate a first complex comprising the first intermediate probe hybridized to the amplification product and the first detectable probe hybridized to the first intermediate probe, wherein the first intermediate probe comprises (i) a hybridization region complementary to the barcode sequence and (ii) a first overhang sequence, and wherein the first detectable probe comprises (i) a sequence complementary to the first overhang sequence and (ii) a first optically detectable moiety; c) imaging the sample to detect a first signal from the first optically detectable moiety, wherein the first signal corresponds to a first signal code in the signal code sequence; d) contacting the sample with a second intermediate probe and a second detectable probe to generate a second complex comprising the second intermediate probe hybridized to the amplification product and the second detectable probe hybridized to the second intermediate probe, wherein the second intermediate probe comprises (i) a hybridization region complementary to the barcode sequence and (ii) a second overhang sequence, and wherein the second detectable probe comprises (i) a sequence complementary to the second overhang sequence and (ii) a second optically detectable moiety; and e) imaging the sample to detect a second signal from the second optically detectable moiety, wherein the second signal corresponds to a second signal code in the signal code sequence, wherein the signal code sequence comprising at least the first signal code and the second signal code is determined at a location in the sample, thereby decoding the barcode sequence and identifying the target nucleic acid sequence and/or sequence of interest (e.g., SNP) at the location in the sample. In some embodiments, the barcode sequence barcode sequence associated with the target nucleic acid sequence and/or sequence of interest (e.g., SNP) is selected from a plurality of barcode sequences, wherein the method comprises contacting the sample with a first pool of intermediate probes and a universal pool of detectable probes, wherein the first pool of intermediate probes comprises the first intermediate probe and the universal pool of detectable probes comprises the first detectable probe and the second detectable probe, wherein each intermediate probe in the first pool of intermediate probes comprises (i) a hybridization region complementary to one of the plurality of the barcode sequences and (ii) an overhang sequence complementary to a detectable probe of the universal pool of detectable probes; and the method comprises contacting the sample with a second pool of intermediate probes and the universal pool of detectable probes, wherein the second pool of intermediate probes comprises the second intermediate probe, and wherein each intermediate probe in the second pool of intermediate probes comprises (i) a hybridization region complementary to one of the plurality of the barcode sequences and (ii) an overhang sequence complementary to a detectable probe of the universal pool of detectable probes. In some embodiments, the method comprises identifying multiple different target nucleic acid sequence and/or sequence of interest (e.g., SNP)s present at locations in the sample, wherein each different target nucleic acid sequence and/or sequence of interest (e.g., SNP) is assigned a different signal code sequence and is targeted by a circularizable probe or probe set comprising a complement of a different barcode sequence of the plurality of barcode sequences. In some embodiments, the number of different intermediate probes in each pool of intermediate probes is greater than the number of different detectable probes in the universal pool of detectable probes. In some embodiments, the number of different detectable probes in the universal pool of detectable probes is four. In some embodiments, the number of different intermediate probes in each pool of intermediate probes is about 10, about 20, about 50, about 100, about 200, about 500, about 1,000, about 2,000, about 5,000, or more.

In some embodiments, each barcode sequence disclosed herein can be detected using sequential hybridization of intermediate probes (e.g., L-shaped probes) and detectable probes (e.g., fluorescently labeled probes) as described herein. The intermediate probes can comprise overhangs that hybridize to fluorescently labeled probes. The overhangs of the intermediate probes can mediate and/or initiate signal enhancement or amplification, such as hybridization chain reaction (HCR), linear oligonucleotide hybridization chain reaction (LO-HCR), or primer exchange reaction (PER), or any other signal enhancement or amplification methods described herein. For instance, each of the barcode sequences in FIG. 2 can be detected using sequential hybridization of probes described herein.

In some embodiments, a barcode sequence disclosed herein can be detected using a method comprising generating a rolling circle amplification (RCA) product molecule, a complex comprising an initiator and an amplifier for hybridization chain reaction (HCR), a complex comprising an initiator and an amplifier for linear oligonucleotide hybridization chain reaction (LO-HCR), a primer exchange reaction (PER) product molecule, a complex comprising a pre-amplifier and an amplifier for branched DNA (bDNA), or a complex comprising any two or more of the aforementioned molecules and complexes. For example, a bDNA complex or an HCR complex can be assembled on an RCA product. See, e.g., US 2021/0198727.

A signal associated with a circularizable probe or probe set disclosed herein (e.g., signals associated with barcode sequences) can be detected using a method comprising targeted deposition of detectable reactive molecules around the site of probe hybridization, targeted assembly of branched structures (e.g., bDNA or branched assay using locked nucleic acid (LNA)), programmed *in situ* growth of concatemers by enzymatic rolling circle amplification (RCA) (e.g., as described in US 2019/0055594), hybridization chain reaction, assembly of topologically catenated DNA structures using serial rounds of chemical ligation (clampFISH), signal amplification via hairpin-mediated concatemerization (e.g., as described in US 2020/0362398), e.g., primer exchange reactions such as signal amplification by exchange reaction (SABER) or SABER with DNA-Exchange (Exchange-SABER).

The detectable reactive molecules may comprise tyramide, such as used in tyramide signal amplification (TSA) or multiplexed catalyzed reporter deposition (CARD)-FISH. In some embodiments, the detectable reactive molecule may be releasable and/or cleavable from a detectable label such as a fluorophore. In some embodiments, a method disclosed herein comprises multiplexed analysis of a biological sample comprising consecutive cycles of probe hybridization, fluorescence imaging, and signal removal, where the signal removal comprises removing the fluorophore from a fluorophore-labeled reactive molecule (e.g., tyramide). Exemplary detectable reactive reagents and methods are described in US 6,828,109, US 2019/0376956, US 2019/0376956, US 2022/0026433, US 2022/0128565, and US 2021/0222234.

In some embodiments, a signal associated with a probe disclosed herein can be detected using hybridization chain reaction (HCR). HCR is an enzyme-free nucleic acid amplification based on a triggered chain of hybridization of nucleic acid molecules starting from HCR monomers, which hybridize to one another to form a nicked nucleic acid polymer. This polymer is the product of the HCR reaction which is ultimately detected in order to indicate the presence of the target analyte. HCR is described in detail in Dirks and Pierce, 2004, PNAS, 101(43), 15275-15278 and in US 7,632,641 and US 7,721,721 (see also US 2006/00234261; Chemeris et al, 2008 Doklady Biochemistry and Biophysics, 419, 53-55; Niu et al, 2010, 46, 3089-3091; Choi et al, 2010, Nat. Biotechnol. 28(11), 1208-1212; and Song et al, 2012, Analyst, 137, 1396-1401). HCR monomers typically comprise a hairpin, or other metastable nucleic acid structure. In the simplest form of HCR, two different types of stable hairpin monomer, referred to here as first and second HCR monomers, undergo a chain reaction of hybridization events to form a long nicked double-stranded DNA molecule when an "initiator" nucleic acid molecule is introduced. The HCR monomers have a hairpin structure comprising a double stranded stem region, a loop region connecting the two strands of the stem region, and a single stranded region at one end of the double stranded stem region. The single stranded region which is exposed (and which is thus available for hybridization to another molecule, e.g. initiator or other HCR monomer) when the monomers are in the hairpin structure may be known as the "toehold region" (or "input domain"). The first HCR monomers each further comprise a sequence which is complementary to a sequence in the exposed toehold region of the second HCR monomers. This sequence of complementarity in the first HCR monomers may be known as the "interacting region" (or "output domain"). Similarly, the second HCR monomers each comprise an interacting region (output domain), e.g. a sequence which is complementary to the exposed toehold region (input domain) of the first HCR monomers. In the absence of the HCR initiator, these interacting regions are protected by the secondary structure (e.g. they are not exposed), and thus the hairpin monomers are stable or kinetically trapped (also referred to as "metastable"), and remain as monomers (e.g. preventing the system from rapidly equilibrating), because the first and second sets of HCR monomers cannot hybridize to each other. However, once the initiator is introduced, it is able to hybridize to the exposed toehold region of a first HCR monomer, and invade it, causing it to open up. This exposes the interacting region of the first HCR monomer (e.g. the sequence of complementarity to the toehold region of the second HCR monomers), allowing it to hybridize to and invade a second HCR monomer at the toehold region. This hybridization and invasion in turn opens up the second HCR monomer, exposing its interacting region (which is complementary to the toehold region of the first HCR monomers), and allowing it to hybridize to and invade another first HCR monomer. The reaction continues in this manner until all of the HCR monomers are exhausted (e.g. all of the HCR monomers are incorporated into a polymeric chain). Ultimately, this chain reaction leads to the formation of a nicked chain of alternating units of the first and second monomer species. The presence of the HCR initiator is thus required in order to trigger the HCR reaction by hybridization to and invasion of a first HCR monomer. The first and second HCR monomers are designed to hybridize to one another are thus may be defined as cognate to one another. They are also cognate to a given HCR initiator sequence. HCR monomers which interact with one another (hybridize) may be described as a set of HCR monomers or an HCR monomer, or hairpin, system.

An HCR reaction could be carried out with more than two species or types of HCR monomers. For example, a system involving three HCR monomers could be used. In such a system, each first HCR monomer may comprise an interacting region which binds to the toehold region of a second HCR monomer; each second HCR may comprise an interacting region which binds to the toehold region of a third HCR monomer; and each third HCR monomer may comprise an interacting region which binds to the toehold region of a first HCR monomer. The HCR polymerization reaction would then proceed as described above, except that the resulting product would be a polymer having a repeating unit of first, second and third monomers consecutively. Corresponding systems with larger numbers of sets of HCR monomers could readily be conceived. For exemplary complexes, see e.g., US 2020/0399689 and US 2022/0064697.

In some embodiments, a signal associated with a probe disclosed herein can be detected using linear oligo hybridization chain reaction (LO-HCR). In some embodiments, provided herein is a method of detecting an analyte in a sample comprising: (i) performing a linear oligo hybridization chain reaction (LO-HCR), wherein an initiator is contacted with a plurality of LO-HCR monomers of at least a first and a second species to generate a polymeric LO-HCR product hybridized to a target nucleic acid molecule, wherein the first species comprises a first hybridization region complementary to the initiator and a second hybridization region complementary to the second species, wherein the first species and the second species are linear, single-stranded nucleic acid molecules; wherein the initiator is provided in one or more parts, and hybridizes directly or indirectly to or is comprised in the target nucleic acid molecule; and (ii) detecting the polymeric product, thereby detecting the analyte. In some embodiments, the first species and/or the second species may not comprise a hairpin structure. In some embodiments, the plurality of LO-HCR monomers may not comprise a metastable secondary structure. In some embodiments, the LO-HCR polymer may not comprise a branched structure. In some embodiments, performing the linear oligo hybridization chain reaction comprises contacting the target nucleic acid molecule with the initiator to provide the initiator hybridized to the target nucleic acid molecule. In any of the embodiments herein, the target nucleic acid molecule and/or the analyte can be a sequence of an endogenous analyte, a ligated asymmetric probe, or an RCA product disclosed herein. Exemplary methods and compositions for LO-HCR are described in US 2021/0198723.

In some embodiments, the barcode sequences can be detected in with a method that comprises signal amplification by performing a primer exchange reaction (PER). In various embodiments, a primer with domain on its 3' end binds to a catalytic hairpin, and is extended with a new domain by a strand displacing polymerase. For example, a primer with domain 1 on its 3' ends binds to a catalytic hairpin, and is extended with a new domain 1 by a strand displacing polymerase, with repeated cycles generating a concatemer of repeated domain 1 sequences. In various embodiments, the strand displacing polymerase is Bst. In various embodiments, the catalytic hairpin includes a stopper which releases the strand displacing polymerase. In various embodiments, branch migration displaces the extended primer, which can then dissociate. In various embodiments, the primer undergoes repeated cycles to form a concatemer primer. In various embodiments, a plurality of concatemer primers is contacted with a sample comprising RCA products generated using methods described herein. In various embodiments, the RCA products may be contacted with a plurality of concatemer primers and a plurality of labeled probes. See e.g., U.S. Pat. Pub. No. US2019/0106733 for exemplary molecules and PER reaction components.

In some embodiments, the RCA products can be detected by providing detection probes, such as probes for performing a chain reaction that forms an amplification product, e.g., HCR. In some embodiments, the analysis comprises determining the sequence of all or a portion of the amplification product. In some embodiments, the analysis comprises detecting a sequence present in the amplification product. In some embodiments, the sequence of all or a portion of the amplification product is indicative of the identity of a region of interest in a target nucleic acid. In other embodiments, the provided methods involve analyzing, e.g., detecting or determining, one or more sequences present in the polynucleotide probes (e.g., a barcode sequence present in an overhang region of the first and/or second probe).

In some embodiments, a sequential decoding scheme involves detecting a plurality of signals from a given target in multiple cycles, and the target may be in the same position in the sample in the different cycles. In some embodiments, a method disclosed herein comprises the localized detection of the target nucleic acid sequences. In some embodiments, the target nucleic acid sequence is present at a fixed or defined location in the sample, and is detected at that location. The target nucleic acid sequence may be localized by virtue of being present *in situ* at its native location in the sample (e.g. a cell or tissue sample), or of being attached or otherwise localized to a target nucleic acid sequence and/or sequence of interest (e.g., SNP) which is present *in situ* at its native location in the sample. The target nucleic acid sequence may be immobilized in the sample, e.g., via crosslinking to other molecules in the sample or a matrix embedding the sample.

### V. COMPOSITIONS, KITS, AND SYSTEMS

In some embodiments, disclosed herein is a composition that comprises a complex containing a target nucleic acid, an asymmetric circularizable probe or probe set, e.g., any of the asymmetric circularizable probe or probe sets described herein. In some embodiments, the complex comprises a primer, e.g., for rolling circle amplification of a circularized circularizable probe or probe set.

In some embodiments, disclosed herein is a composition that comprises an amplification product containing monomeric units of a sequence complementary to a sequence of an asymmetric circularizable probe or probe set. In some embodiments, the amplification product is formed using any of the target nucleic acids, asymmetric circularizable probes and any of the amplification techniques described herein.

Also provided herein are kits, for example comprising one or more polynucleotides, e.g., any described in Section III, and reagents for performing the methods provided herein, for example reagents required for one or more steps comprising hybridization, ligation, amplification, detection, sequencing, and/or sample preparation as described herein. In some embodiments, the kit further comprises a target nucleic acid. In some embodiments, any or all of the polynucleotides are DNA molecules. In some embodiments, the target nucleic acid is a messenger RNA molecule. In some embodiments, the kit further comprises a ligase, for instance for forming a ligated circular probe from the circularizable probe or probe set. In some embodiments, the ligase has DNA-splinted DNA ligase activity. In some embodiments, the kit further comprises a polymerase, for instance for performing amplification of the circularizable probe or probe set. In some embodiments, the polymerase is capable of using the ligated circular probe as a template for amplification. In some embodiments, the kit further comprises a primer for amplification.

Disclosed herein in some aspects is a kit for analyzing a biological sample, comprising a circularizable probe or probe set comprising a 5' hybridization region and a 3' hybridization region that hybridize to adjacent first and second target regions, respectively, in a target nucleic acid, wherein the 5' hybridization region and the 3' hybridization region are different in length; the 5' hybridization region or the 3' hybridization region comprises an interrogatory sequence complementary to a sequence of interest in the first or second target region, respectively; and the interrogatory sequence is an internal sequence in the 5' hybridization region and does not comprise the 5' terminal nucleotide.

In some aspects, disclosed herein is a kit for analyzing a biological sample, comprising a circularizable probe comprising a 5' hybridization region and a 3' hybridization region that hybridize to adjacent first and second target regions, respectively, in a target nucleic acid, wherein the 5' hybridization region and the 3' hybridization region are different in length; the shorter one of the 5' hybridization region and the 3' hybridization region comprises an interrogatory nucleotide complementary to a single nucleotide of interest in the first or second target region, respectively; and the interrogatory nucleotide is not a 5' or 3' terminal nucleotide of the circularizable probe. In some embodiments, the 5' hybridization region is shorter than the 3' hybridization region, and the interrogatory nucleotide is any one of the second to the tenth nucleotides from the 5' end of the circularizable probe.

In some aspects, disclosed herein is a kit for analyzing a biological sample comprising a circularizable probe or probe set. In some embodiments, the circularizable probe or probe set comprises i) a first circularizable probe comprising a 5' hybridization region and a 3' hybridization region that hybridize to adjacent first and second target regions, respectively, in a target nucleic acid, wherein: the 5' hybridization region and the 3' hybridization region are different in length; the shorter one of the 5' hybridization region and the 3' hybridization region comprises a first interrogatory nucleotide complementary to a first variant of the single nucleotide of interest in the first or second target region, respectively; and the first interrogatory nucleotide is not a 5' or 3' terminal nucleotide of the circularizable probe; and ii) a second circularizable probe comprising the 5' hybridization region and the 3' hybridization region, except that the second circularizable probe comprises a second interrogatory nucleotide complementary to a second variant of the single nucleotide of interest. In some embodiments, the first circularizable probe or probe set comprise a first barcode sequence corresponding to the first variant and the second circularizable probe or probe set comprise a second barcode sequence corresponding to the second variant. In any of the embodiments herein, the kit can further comprise one or more intermediate probes that directly or indirectly hybridize to the first and/or second barcode sequences or complements thereof; and/or one or more detectably-labeled probes that directly or indirectly hybridize to the one or more intermediate probes. In any of the embodiments herein, the first and second interrogatory nucleotides can be any one of the second to the tenth nucleotides from the 5' end of the first or second circularizable probe or probe set, respectively.

The various components of the kit may be present in separate containers or certain compatible components may be pre-combined into a single container. In some embodiments, the kits further contain instructions for using the components of the kit to practice the provided methods.

In some embodiments, the kits can contain reagents and/or consumables required for performing one or more steps of the provided methods. In some embodiments, the kits contain reagents for fixing, embedding, and/or permeabilizing the biological sample. In some embodiments, the kits contain reagents, such as enzymes and buffers for ligation and/or amplification, such as ligases and/or polymerases. In some aspects, the kit can also comprise any of the reagents described herein, e.g., wash buffer and ligation buffer. In some embodiments, the kits contain reagents for detection and/or sequencing, such as barcode detection probes or detectable labels. In some embodiments, the kits optionally contain other components, for example nucleic acid primers, enzymes and reagents, buffers, nucleotides, modified nucleotides, reagents for additional assays.

### VI. APPLICATIONS

In some aspects, the provided embodiments can be applied in an *in situ* method of analyzing nucleic acid sequences, such as an *in situ* transcriptomic analysis or *in situ* sequencing, for example from intact tissues or samples in which the spatial information has been preserved. In some aspects, the embodiments can be applied in an imaging or detection method for multiplexed nucleic acid analysis. In some aspects, the provided embodiments can be used to identify or detect regions of interest in target nucleic acids.

In some embodiments, the region of interest is a single nucleotide of interest. In some embodiments, the single nucleotide of interest is a single-nucleotide polymorphism (SNP). In some embodiments, the single nucleotide of interest is a single-nucleotide variant (SNV). In some embodiments, the single nucleotide of interest is a single-nucleotide substitution. In some embodiments, the single nucleotide of interest is a point mutation. In some embodiments, the single nucleotide of interest is a single-nucleotide insertion. In some embodiments, the region of interest comprises a polymorphism including, but are not limited to multi-nucleotide changes, insertions, deletions, and translocations.

In some aspects, the embodiments can be applied in investigative and/or diagnostic applications, for example, for characterization or assessment of particular cell or a tissue from a subject. Applications of the provided method can comprise biomedical research and clinical diagnostics. For example, in biomedical research, applications comprise, but are not limited to, spatially resolved gene expression analysis for biological investigation or drug screening. In clinical diagnostics, applications comprise, but are not limited to, detecting gene markers such as disease, immune responses, bacterial or viral DNA/RNA for patient samples.

In some aspects, the embodiments can be applied to visualize the distribution of genetically encoded markers in whole tissue at subcellular resolution, for example, chromosomal abnormalities (inversions, duplications, translocations, etc.), loss of genetic heterozygosity, the presence of gene alleles indicative of a predisposition towards disease or good health, likelihood of responsiveness to therapy, or in personalized medicine or ancestry.

### VII. TERMINOLOGY

Unless defined otherwise, all terms of art, notations and other technical and scientific terms or terminology used herein are intended to have the same meaning as is commonly understood by one of ordinary skill in the art to which the claimed subject matter pertains. In some cases, terms with commonly understood meanings are defined herein for clarity and/or for ready reference, and the inclusion of such definitions herein should not necessarily be construed to represent a substantial difference over what is generally understood in the art.

The terms "polynucleotide" and "nucleic acid molecule" used interchangeably herein, refer to polymeric forms of nucleotides of any length, either ribonucleotides or deoxyribonucleotides. Thus, this term comprises, but is not limited to, single-, double-, or multi- stranded DNA or RNA, genomic DNA, cDNA, DNA-RNA hybrids, or a polymer comprising purine and pyrimidine bases or other natural, chemically or biochemically modified, non-natural, or derivatized nucleotide bases. The backbone of the polynucleotide can comprise sugars and phosphate groups (as may typically be found in RNA or DNA), or modified or substituted sugar or phosphate groups.

"Hybridization" as used herein may refer to the process in which two single-stranded polynucleotides bind non-covalently to form a stable double-stranded polynucleotide. In one aspect, the resulting double-stranded polynucleotide can be a "hybrid" or "duplex." "Hybridization conditions" typically include salt concentrations of approximately less than 1 M, often less than about 500 mM and may be less than about 200 mM. A "hybridization buffer" includes a buffered salt solution such as 5% SSPE, or other such buffers known in the art. Hybridization temperatures can be as low as 5°C, but are typically greater than 22°C, and more typically greater than about 30°C, and typically in excess of 37°C. Hybridizations are often performed under stringent conditions, e.g., conditions under which a sequence will hybridize to its target sequence but will not hybridize to other, non-complementary sequences. Stringent conditions are sequence-dependent and are different in different circumstances. For example, longer fragments may require higher hybridization temperatures for specific hybridization than short fragments. As other factors may affect the stringency of hybridization, including base composition and length of the complementary strands, presence of organic solvents, and the extent of base mismatching, the combination of parameters is more important than the absolute measure of any one parameter alone. Generally stringent conditions are selected to be about 5°C lower than the T*ₘ* for the specific sequence at a defined ionic strength and pH. The melting temperature T*ₘ* can be the temperature at which a population of double-stranded nucleic acid molecules becomes half dissociated into single strands. Several equations for calculating the T*ₘ* of nucleic acids can be used, for example, a simple estimate of the T*ₘ* value may be calculated by the equation, T*ₘ* =81.5 + 0.41 (% G + C), when a nucleic acid is in aqueous solution at 1 M NaCl (see *e.g.,* Anderson and Young, Quantitative Filter Hybridization, in Nucleic Acid Hybridization (1985)). Other references (e.g., Allawi and SantaLucia, Jr., Biochemistry, 36:10581-94 (1997)) include alternative methods of computation which take structural and environmental, as well as sequence characteristics into account for the calculation of T*ₘ*.

In general, the stability of a hybrid is a function of the ion concentration and temperature. Typically, a hybridization reaction is performed under conditions of lower stringency, followed by washes of varying, but higher, stringency. Exemplary stringent conditions include a salt concentration of at least 0.01 M to no more than 1 M sodium ion concentration (or other salt) at a pH of about 7.0 to about 8.3 and a temperature of at least 25°C. For example, conditions of 5 × SSPE (750 mM NaCl, 50 mM sodium phosphate, 5 mM EDTA at pH 7.4) and a temperature of approximately 30°C are suitable for allele-specific hybridizations, though a suitable temperature depends on the length and/or GC content of the region hybridized. In one aspect, "stringency of hybridization" in determining percentage mismatch can be as follows: 1) high stringency: 0.1 × SSPE, 0.1% SDS, 65°C; 2) medium stringency: 0.2 × SSPE, 0.1% SDS, 50°C (also referred to as moderate stringency); and 3) low stringency: 1.0 × SSPE, 0.1% SDS, 50°C. It is understood that equivalent stringencies may be achieved using alternative buffers, salts and temperatures. For example, moderately stringent hybridization can refer to conditions that permit a nucleic acid molecule such as a probe to bind a complementary nucleic acid molecule. The hybridized nucleic acid molecules generally have at least 60% identity, including for example at least any of 70%, 75%, 80%, 85%, 90%, or 95% identity. Moderately stringent conditions can be conditions equivalent to hybridization in 50% formamide, 5 × Denhardt's solution, 5x SSPE, 0.2% SDS at 42°C, followed by washing in 0.2 × SSPE, 0.2% SDS, at 42°C. High stringency conditions can be provided, for example, by hybridization in 50% formamide, 5 × Denhardt's solution, 5 × SSPE, 0.2% SDS at 42°C, followed by washing in 0.1 × SSPE, and 0.1% SDS at 65°C. Low stringency hybridization can refer to conditions equivalent to hybridization in 10% formamide, 5 × Denhardt's solution, 6 × SSPE, 0.2% SDS at 22°C, followed by washing in 1x SSPE, 0.2% SDS, at 37°C. Denhardt's solution contains 1% Ficoll, 1% polyvinylpyrolidone, and 1% bovine serum albumin (BSA). 20 × SSPE (sodium chloride, sodium phosphate, ethylene diamide tetraacetic acid (EDTA)) contains 3M sodium chloride, 0.2M sodium phosphate, and 0.025 M EDTA. Other suitable moderate stringency and high stringency hybridization buffers and conditions are described, for example, in Sambrook et al., Molecular Cloning: A Laboratory Manual, 2nd ed., Cold Spring Harbor Press, Plainview, N.Y. (1989); and Ausubel et al., Short Protocols in Molecular Biology, 4th ed., John Wiley & Sons (1999).

Alternatively, substantial complementarity exists when an RNA or DNA strand will hybridize under selective hybridization conditions to its complement. Typically, selective hybridization will occur when there is at least about 65% complementary over a stretch of at least 14 to 25 nucleotides, preferably at least about 75%, more preferably at least about 90% complementary. *See* M. Kanehisa, Nucleic Acids Res. 12:203 (1984).

A "primer" used herein can be an oligonucleotide, either natural or synthetic, that is capable, upon forming a duplex with a polynucleotide template, of acting as a point of initiation of nucleic acid synthesis and being extended from its 3' end along the template so that an extended duplex is formed. The sequence of nucleotides added during the extension process is determined by the sequence of the template polynucleotide. Primers usually are extended by a DNA polymerase.

"Ligation" may refer to the formation of a covalent bond or linkage between the termini of two or more nucleic acids, *e.g.,* oligonucleotides and/or polynucleotides, in a template-driven reaction. The nature of the bond or linkage may vary widely and the ligation may be carried out enzymatically or chemically. As used herein, ligations are usually carried out enzymatically to form a phosphodiester linkage between a 5' carbon terminal nucleotide of one oligonucleotide with a 3' carbon of another nucleotide.

"Sequencing," "sequence determination" and the like means determination of information relating to the nucleotide base sequence of a nucleic acid. Such information may include the identification or determination of partial as well as full sequence information of the nucleic acid. Sequence information may be determined with varying degrees of statistical reliability or confidence. In one aspect, the term includes the determination of the identity and ordering of a plurality of contiguous nucleotides in a nucleic acid. "High throughput digital sequencing" or "next generation sequencing" means sequence determination using methods that determine many (typically thousands to billions) of nucleic acid sequences in an intrinsically parallel manner, e.g., where DNA templates are prepared for sequencing not one at a time, but in a bulk process, and where many sequences are read out preferably in parallel, or alternatively using an ultra-high throughput serial process that itself may be parallelized. Such methods include but are not limited to pyrosequencing (for example, as commercialized by 454 Life Sciences, Inc., Branford, Conn.); sequencing by ligation (for example, as commercialized in the SOLiD^{™} technology, Life Technologies, Inc., Carlsbad, Calif.); sequencing by synthesis using modified nucleotides (such as commercialized in TruSeq^{™} and HiSeq^{™} technology by Illumina, Inc., San Diego, Calif.; HeliScope^{™} by Helicos Biosciences Corporation, Cambridge, Ma.; and PacBio RS by Pacific Biosciences of California, Inc., Menlo Park, Calif.), sequencing by ion detection technologies (such as Ion Torrent^{™} technology, Life Technologies, Carlsbad, Calif.); sequencing of DNA nanoballs (Complete Genomics, Inc., Mountain View, Calif.); nanopore-based sequencing technologies (for example, as developed by Oxford Nanopore Technologies, LTD, Oxford, UK), and like highly parallelized sequencing methods.

"SNP" or "single nucleotide polymorphism" may include a genetic variation between individuals; *e.g.,* a single nitrogenous base position in the DNA of organisms that is variable. SNPs are found across the genome; much of the genetic variation between individuals is due to variation at SNP loci, and often this genetic variation results in phenotypic variation between individuals. SNPs for use in the present disclosure and their respective alleles may be derived from any number of sources, such as public databases (U.C. Santa Cruz Human Genome Browser Gateway (genome.ucsc.edu/cgi-bin/hgGateway) or the NCBI dbSNP website (www.ncbi.nlm.nih gov/SNP/), or may be experimentally determined as described in U.S. Pat. No. 6,969,589; and US Pub. No. 2006/0188875 entitled "Human Genomic Polymorphisms.". Although the use of SNPs is described in some of the embodiments presented herein, it will be understood that other biallelic or multi-allelic genetic markers may also be used. A biallelic genetic marker is one that has two polymorphic forms, or alleles. As mentioned above, for a biallelic genetic marker that is associated with a trait, the allele that is more abundant in the genetic composition of a case group as compared to a control group is termed the "associated allele," and the other allele may be referred to as the "unassociated allele." Thus, for each biallelic polymorphism that is associated with a given trait (*e.g.,* a disease or drug response), there is a corresponding associated allele. Other biallelic polymorphisms that may be used with the methods presented herein include, but are not limited to multinucleotide changes, insertions, deletions, and translocations. It will be further appreciated that references to DNA herein may include genomic DNA, mitochondrial DNA, episomal DNA, and/or derivatives of DNA such as amplicons, RNA transcripts, cDNA, DNA analogs, *etc.* The polymorphic loci that are screened in an association study may be in a diploid or a haploid state and, ideally, would be from sites across the genome.

"Multiplexing" or "multiplex assay" herein may refer to an assay or other analytical method in which the presence and/or amount of multiple targets, *e.g.,* multiple nucleic acid target sequences, can be assayed simultaneously by using more than one capture probe conjugate, each of which has at least one different detection characteristic, *e.g.,* fluorescence characteristic (for example excitation wavelength, emission wavelength, emission intensity, FWHM (full width at half maximum peak height), or fluorescence lifetime) or a unique nucleic acid or protein sequence characteristic.

The term "about" as used herein refers to the usual error range for the respective value readily known to the skilled person in this technical field. Reference to "about" a value or parameter herein comprises (and describes) embodiments that are directed to that value or parameter per se.

As used herein, the singular forms "a," "an," and "the" comprise plural referents unless the context clearly dictates otherwise. For example, "a" or "an" means "at least one" or "one or more."

Throughout this disclosure, various aspects of the claimed subject matter are presented in a range format. It should be understood that the description in range format is merely for convenience and brevity and should not be construed as an inflexible limitation on the scope of the claimed subject matter. Accordingly, the description of a range should be considered to have specifically disclosed all the possible sub-ranges as well as individual numerical values within that range. For example, where a range of values is provided, it is understood that each intervening value, between the upper and lower limit of that range and any other stated or intervening value in that stated range is encompassed within the claimed subject matter. The upper and lower limits of these smaller ranges may independently be comprised in the smaller ranges, and are also encompassed within the claimed subject matter, subject to any specifically excluded limit in the stated range. Where the stated range comprises one or both of the limits, ranges excluding either or both of those comprised limits are also comprised in the claimed subject matter. This applies regardless of the breadth of the range.

Use of ordinal terms such as "first", "second", "third", etc., in the claims to modify a claim element does not by itself connote any priority, precedence, or order of one claim element over another or the temporal order in which acts of a method are performed, but are used merely as labels to distinguish one claim element having a certain name from another element having a same name (but for use of the ordinal term) to distinguish the claim elements. Similarly, use of a), b), etc., or i), ii), etc. does not by itself connote any priority, precedence, or order of steps in the claims. Similarly, the use of these terms in the specification does not by itself connote any required priority, precedence, or order.

### EXAMPLES

The following examples are included for illustrative purposes only and are not intended to limit the scope of the present disclosure.

### Example 1: KRAS mutation detection with asymmetric padlock probes.

This example describes the detection of point mutations and/or SNPs by directly targeting *KRAS* mRNA molecules using asymmetric padlock probes. Various padlock probes targeting common *KRAS* mutations for RNA-templated padlock ligation and subsequent RCA of the ligated padlock probe were designed and tested for their specificity in detecting the mutations. The padlock probes can be used for direct RNA (dRNA) *in situ* profiling such as mutation profiling in cancer and miRNA profiling in tissues, for example, in human tumor sections and human tissue microarrays (TMAs).

### Methods

ME180 and A549 cells were seeded on slides and allowed to attach. The cells were then fixed in formaldehyde in PBS and permeabilized. The slides were washed in PBS and dehydrated through an ethanol series of 70% and 100%. The slides were then hydrated, e.g., by a brief wash with PBS-T.

For probe hybridization, the cells were washed twice in PBS-T and then a probe pool of mutant and wildtype probes were added in hybridization buffer including SSC and formamide and incubated. Then, the probe hybridization mixture was removed and the cells were washed in pre-warmed wash buffers including SSC and formamide, then washed with PBS-T.

For ligation, the cells were washed once in PBS-T, and then ligation reaction mix was added. Ligation reaction mixture for the asymmetric padlock probes on RNA contained T4 RNA ligase buffer, RNAse inhibitor and T4 RNA ligase. Ligation reaction mixture for the symmetric padlock probes on cDNA contained Tth Ligase (BLIRT) and RNaseH (BLIRT). The cells were washed twice in PBS-T and RCA reaction mixture (containing Phi29 reaction buffer, dNTPs, Phi29 polymerase) was added and incubated for rolling circle amplification (RCA).

Subsequently, the cells were washed in PBS-T twice and detection probes were hybridized to the RCA products *in situ* (Cy5 labelled probes to *KRAS* wild type probes, Cy3 labelled probes to *KRAS* mutant probes) hybridization buffer containing SSC and formamide. The cells were washed in PBS-T three times, stained with DAPI, washed three times again, and then mounted in Slowfade mounting medium. Cells were imaged in fluorescent microscope with 20x objective and RCA products quantified using Cell profiler software.

### Results

To evaluate the specificity of chimeric asymmetric padlock probes, multiple padlock probes (PLPs) targeting common *KRAS* mutations for RNA-templated RCA were designed and tested using *KRAS*-mutated cell lines (Table 1). The probes were chimeric DNA/RNA probes with a ribonucleotide at the 3' end and 5' and 3' arms were of asymmetric lengths. These probes allow for direct detection of the RNA targets, as they are substrates for RNA-templated ligation. The asymmetric padlock probes were compared to a symmetric DNA probe, which requires the target RNA to be converted into cDNA by reverse transcription prior to DNA-templated ligation of the probe.

**Table 1: Selected KRAS mutations and cell lines**

| **Cell Line** | **Tissue** | **KRAS Mutant** | **Genomic Coordinates** | **Number of Mutant Alleles** |
|---|---|---|---|---|
| **A549** | Lung | G12S | GRCh38, 12:25245351 | 2 |
| **A427** | Lung | G12D | GRCh38, 12:25245350 | 1 |
| **ME-180** | Cervix | Wild Type | GRCh38, 12:25245351 | 0 |

Of the asymmetric probe designs evaluated, the 10-20 m5 asymmetric probe was chosen for further evaluation. The 10-20 m5 probe has a 10-nucleotide 5' arm with an internal interrogatory nucleotide at the fifth base (m5) and a 20-nucleotide 3' arm **(****FIG. 4A). FIGS. 4B-4C** show the results of the validation of the 10-20 m5 probes in a *KRAS*-mutant cell line (A549) and a *KRAS*-wildtype (ME180) cell line. **FIG. 4B** shows the expected bias in count using the 10-20 m5 probes in direct RNA (dRNA) detection is comparable to results using symmetric padlock probes and the cDNA-based hybridization-based *in situ* sequencing approach (cDNA control) described in Gyllborg et al., "Hybridization-based in situ sequencing (HybISS) for spatially resolved transcriptomics in human and mouse brain tissue," Nucleic Acids Res. 2020;48(19):e112. However, the 10-20 m5 probes showed a higher fold change in expected counts in both the *KRAS*-mutant cells (A549) and the *KRAS-*wildtype (ME180) cells as compared to the cDNA control **(****FIG. 4C****).** Thus, detection efficiency of single nucleotide of interest using asymmetric padlock probes on RNA is increased when compared to cDNA-based detection, while maintaining detection specificity.

To evaluate the effect of the position of the interrogatory base in a 10-20 asymmetric probe design, 10-20 probes having a mutation at either the first position (10-20 m1 as shown in **FIG. 5A**), third position (10-20 m3 as shown in **FIG. 5B**), or fifth position (10-20 m5), were tested. Efficiency of detection with each probe design was evaluated in ME180 (KRAS-wildtype) and A549 (KRAS-mutant) cell lines.

**Table 2A: Efficiency of detection with various 10-20 probes**

| **Cell Line/Probe Configuration** | Total RCPs, normalized |
|---|---|
| ME-180 / 10-20 m1 | 1.64 |
| ME-180 / 10-20 m3 | 1.20 |
| ME-180 / 10-20 m5 | 1.00 |
| A549 / 10-20 m1 | 0.43 |
| A549 / 10-20 m3 | 0.99 |
| A549 / 10-20 m5 | 1.00 |

**Table 2B: RCP counts per probe with various 10-20 probes designs**

| **Cell Line/Probe Configuration** | RCP counts per probe | |
|---|---|---|
| ME-180 / 10-20 m1 | WT count/nuclei | 0.11 |
| | Mutant count/nuclei | 0.30 |
| ME-180 / 10-20 m3 | WT count/nuclei | 0.14 |
| | Mutant count/nuclei | 0.16 |
| ME-180 / 10-20 m5 | WT count/nuclei | 0.19 |
| | Mutant count/nuclei | 0.06 |
| A549/ 10-20 m1 | WT count/nuclei | 0.03 |
| | Mutant count/nuclei | 0.26 |
| A549 / 10-20 m3 | WT count/nuclei | 0.23 |
| | Mutant count/nuclei | 0.44 |
| A549 / 10-20 m5 | WT count/nuclei | 0.53 |
| | Mutant count/nuclei | 0.15 |

The efficiency of detection was found to be comparable between the 10-20 m1, 10-20 m3 and 10-20 m5 probes (Table 2A). The values in Table 2B represent the ratio of wildtype or mutant *KRAS* transcripts detected over the number of nuclei. Probes with symmetric 15-nucleotide arms and an interrogatory base at the 3' end showed comparable efficiency (number of transcripts detected) as asymmetric 10-20 m1, 10-20 m3 and 10-20 m5 probes. However, probes with symmetric 15-nucleotide arms and an interrogatory base at the 3' end showed poor specificity of detection compared to asymmetric 10-20 m1, 10-20 m3 and 10-20 m5 probes **(****FIGS. 6A-6B****).**

In view of the results, asymmetric padlock probes can efficiently and specifically detect single nucleotide changes in a target nucleic acid. Asymmetric padlock probes can be applied to *in situ* sequencing of target RNAs in human tumor sections and human tissue microarrays (TMAs), and can also be used for further applications such as mutation profiling in cancer and profiling of microRNAs.

### Example 2: Mutation detection in mouse brain sections using asymmetric padlock probes.

In this example, the efficiency and specificity of targeting mRNAs *in situ* using asymmetric padlock probes were assessed.

### Methods

Detection of *pcp4* and *neurod6* were performed on mouse brain tissue samples. Mouse brains were, right after surgical removal and without any fixation, imbedded into OCT medium and directly frozen on dry ice, and thereafter stored at -80°C until usage. 10 µm sections were then cut with a cryostat, and sections collected on Superfrost glass slides. Sections were then shortly fixated in formaldehyde in PBS and washed once in PBS Tween, after which they were permeabilized. After the permeabilization, slides were washed twice in PBS and dehydrated through an ethanol series. Secure seal chamber was mounted on the slide covering the tissue section, and the tissue was hydrated by a brief wash with PBS-T.

To target mRNAs with chimeric asymmetric padlock probes, the section was, after the brief rehydration wash, immersed into probe hybridization mixture including SSC and formamide and a pool of asymmetric padlock probes and incubated. Each pool contained a matching padlock probe in addition to three non-matching padlock probes. After that the section was washed in pre-warmed buffer including SSC and formamide. Finally, the section was washed in PBS-T. Following the washes performed after hybridization, ligation reaction mix including T4 ligase buffer and T4 ligase was added to the section.

For RCA, the section was immersed in rolling circle amplification mixture, containing Phi29 polymerase buffer, dNTPs, RCA primer and Phi29 polymerase and incubated. Subsequently, the section was washed in PBS-T twice.

For detection of the RCA products, intermediate probes (L-shaped probes that hybridize to barcode sequences in RCA products with an overhang that hybridizes to a fluorescently-labeled probe) and detection probes (fluorescently-labeled probes) were then hybridized. L-probes were hybridized to the RCA products *in situ* in hybridization buffer including SSC and formamide. Detection probes (serving as anchor probes in the *in situ* sequencing reaction) were then hybridized to the RCA products *in situ* in hybridization buffer. TrueBlack was then applied to all sections. The sections were then imaged in fluorescent microscope with 20x objective, with evaluation of RCA products on 3 fields of view per section.

### Results

Symmetric probes with an interrogatory ribonucleotide base at the 3' end **(****FIG. 7A****)** were generally more efficient for detection of G and C mutations than for detection of A and U mutations in a target RNA as measured by total signal ratio (the ratio of RCA products per detection channel and total RCA products) **(****FIG. 7B****).** To test the efficiency and specificity of mutation detection *in situ* using asymmetric padlock probes, probes for *pcp4* in the 10-20 probe design were evaluated on mouse brain sections. The efficiency and specificity of four probe sets (Probe Set A, Probe Set T, Probe Set G, and Probe Set C) for detection of a single nucleotide of interest in *pcp4* transcripts in mouse brain sections were determined **(****FIG. 8A****).** Each probe set comprises a probe with the matching base and three probes each with a mismatching base. For instance, Probe Set G comprises a probe with the matching base G and three probes with mismatching bases A, C, and T. The detection efficiency of each probe set is shown in **FIG. 8B****.** Each probe set was able to specifically detect the expected base **(****FIG. 8C****).**

The present disclosure is not intended to be limited in scope to the particular disclosed embodiments, which are provided, for example, to illustrate various aspects of the present disclosure. Various modifications to the compositions and methods described will become apparent from the description and teachings herein.

## Claims

1. A method for analyzing a biological sample, comprising:
a) contacting the biological sample with a circularizable probe, wherein:
the circularizable probe comprises a 5' hybridization region and a 3' hybridization region that hybridize to adjacent first and second target regions, respectively, in a target nucleic acid in the biological sample,
the 5' hybridization region and the 3' hybridization region are different in length,
the 5' hybridization region or the 3' hybridization region comprises an interrogatory sequence complementary to a sequence of interest in the first or second target region, respectively,
the interrogatory sequence does not comprise a ligatable end, and
wherein the interrogatory sequence is in the shorter one of the 5' hybridization region and the 3' hybridization region;
b) circularizing the circularizable probe hybridized to the target nucleic acid to form a circularized probe; and
c) detecting the circularized probe or a product thereof in the biological sample.

2. The method of claim 1, wherein the target nucleic acid comprises RNA.

3. The method of any of claims 1-2, wherein the 5' hybridization region is shorter than the 3' hybridization region.

4. The method of any of claims 1-2, wherein the 5' hybridization region is longer than the 3' hybridization region.

5. The method of any of claims 1-4, wherein the lengths of the 5' hybridization region and the 3' hybridization region differ by 1, 2, 3, 4, 5, 6, 7, 8, 9, 10, 11, 12, 13, 14, 15, 16, 17, 18, 19, 20, 21, 22, 23, 24, or 25 nucleotides, optionally wherein the 5' hybridization region comprises the interrogatory sequence and is shorter than the 3' hybridization region by about 5, 6, 7, 8, 9, 10, 11, 12, 13, 14, or 15 nucleotides.

6. The method of any of claims 1-5, wherein the interrogatory sequence in the circularizable probe is one, two, three, four, or five nucleotides in length.

7. The method of claim 6, wherein the interrogatory sequence is an interrogatory nucleotide, and the sequence of interest is a single nucleotide of interest selected from the group consisting of a single-nucleotide polymorphism (SNP), a single-nucleotide variant (SNV), a single-nucleotide substitution, a point mutation, a single-nucleotide deletion, and a single-nucleotide insertion.

8. The method of claim 7, wherein the interrogatory nucleotide is the second, third, fourth, fifth, sixth, seventh, eighth, ninth, or tenth nucleotide of the 5' hybridization region, with the 5' terminal nucleotide of the circularizable probe being the first nucleotide of the 5' hybridization region, optionally wherein the interrogatory nucleotide is the fourth, fifth, or sixth nucleotide of the 5' hybridization region.

9. The method of any of claims 1-8, further comprising prior to the circularizing step, a step of removing molecules of the circularizable probe that are not bound to the target nucleic acid from the biological sample.

10. The method of any of claims 1-9, further comprising generating the product of the circularized probe *in situ* in the biological sample, optionally wherein the product is generated using rolling circle amplification (RCA).

11. The method of any of claims 1-10, wherein the method comprises imaging the biological sample to detect the circularized probe or product thereof, wherein the imaging comprises detecting a signal associated with a fluorescently labeled probe that directly or indirectly binds to a rolling circle amplification product of the circularized probe.

12. The method of claim 11, wherein a sequence of the rolling circle amplification product is analyzed *in situ* in the biological sample, wherein the sequence of the rolling circle amplification product is analyzed by sequential hybridization, sequencing by hybridization, sequencing by ligation, sequencing by synthesis, sequencing by binding, or a combination thereof.

13. The method of claim 12, wherein the sequence of the rolling circle amplification product comprises one or more barcode sequences or complements thereof, wherein the one or more barcode sequences or complements thereof correspond to the target nucleic acid and/or the sequence of interest.

14. The method of any of claims 1-13 wherein the biological sample is a tissue sample.

15. A kit for analyzing a biological sample, comprising a circularizable probe comprising a 5' hybridization region and a 3' hybridization region that hybridize to adjacent first and second target regions, respectively, in a target nucleic acid, wherein:
the 5' hybridization region and the 3' hybridization region are different in length,
the 5' hybridization region or the 3' hybridization region comprises an interrogatory sequence complementary to a sequence of interest in the first or second target region, respectively,
the interrogatory sequence does not comprise a ligatable end, and
the interrogatory sequence is in the shorter one of the 5' hybridization region and the 3' hybridization region.

## Patentansprüche

1. Verfahren zum Analysieren einer biologischen Probe, umfassend:
a) Inkontaktbringen der biologischen Probe mit einer zirkularisierbaren Sonde, wobei:
die zirkularisierbare Sonde eine 5'-Hybridisierungsregion und eine 3'-Hybridisierungsregion umfasst, die jeweils mit einer benachbarten ersten und zweiten Zielregion in einer Zielnukleinsäure in der biologischen Probe hybridisieren,
die 5'-Hybridisierungsregion und die 3'-Hybridisierungsregion unterschiedlich lang sind,
die 5'-Hybridisierungsregion oder die 3'-Hybridisierungsregion eine Abfragesequenz umfasst, die jeweils komplementär zu einer Sequenz von Interesse in der ersten oder zweiten Zielregion ist,
die Abfragesequenz kein ligierbares Ende umfasst, und
wobei sich die Abfragesequenz in der kürzeren aus der 5'-Hybridisierungsregion und der 3'-Hybridisierungsregion befindet;
b) Zirkularisieren der zirkularisierbaren Sonde, die mit der Zielnukleinsäure hybridisiert ist, um eine zirkularisierte Sonde zu bilden; und
c) Nachweisen der zirkularisierten Sonde oder eines Produkts davon in der biologischen Probe.

2. Verfahren nach Anspruch 1, wobei die Zielnukleinsäure RNA umfasst.

3. Verfahren nach einem der Ansprüche 1-2, wobei die 5'-Hybridisierungsregion kürzer als die 3'-Hybridisierungsregion ist.

4. Verfahren nach einem der Ansprüche 1-2, wobei die 5'-Hybridisierungsregion länger als die 3'-Hybridisierungsregion ist.

5. Verfahren nach einem der Ansprüche 1-4, wobei sich die Längen der 5'-Hybridisierungsregion und der 3'-Hybridisierungsregion um 1, 2, 3, 4, 5, 6, 7, 8, 9, 10, 11, 12, 13, 14, 15, 16, 17, 18, 19, 20, 21, 22, 23, 24 oder 25 Nukleotide unterscheiden, wobei die 5'-Hybridisierungsregion die Abfragesequenz umfasst und um etwa 5, 6, 7, 8, 9, 10, 11, 12, 13, 14 oder 15 Nukleotide kürzer als die 3'-Hybridisierungsregion ist.

6. Verfahren nach einem der Ansprüche 1-5, wobei die Abfragesequenz in der zirkularisierbaren Sonde eine, zwei, drei, vier oder fünf Nukleotide lang ist.

7. Verfahren nach Anspruch 6, wobei die Abfragesequenz ein Abfragenukleotid ist und die Sequenz von Interesse ein einzelnes Nukleotid von Interesse ist, ausgewählt aus der Gruppe bestehend aus einem Einzelnukleotidpolymorphismus (SNP), einer Einzelnukleotidvariante (SNV), einer Einzelnukleotidsubstitution, einer Punktmutation, einer Einzelnukleotiddeletion und einer Einzelnukleotidinsertion.

8. Verfahren nach Anspruch 7, wobei das Abfragenukleotid das zweite, dritte, vierte, fünfte, sechste, siebte, achte, neunte oder zehnte Nukleotid der 5'-Hybridisierungsregion ist, wobei das 5'-terminale Nukleotid der zirkularisierbaren Sonde das erste Nukleotid der 5'-Hybridisierungsregion ist, optional, wobei das Abfragenukleotid das vierte, fünfte oder sechste Nukleotid der 5'-Hybridisierungsregion ist.

9. Verfahren nach einem der Ansprüche 1-8, das ferner vor dem Zirkularisierungsschritt einen Schritt des Entfernens von Molekülen der zirkularisierbaren Sonde, die nicht an die Zielnukleinsäure gebunden sind, aus der biologischen Probe umfasst.

10. Verfahren nach einem der Ansprüche 1-9, das ferner ein Erzeugen des Produkts der zirkularisierten Sonde *in situ* in der biologischen Probe umfasst, wobei das Produkt optional unter Verwendung von Rolling-Circle-Amplifikation (RCA) erzeugt wird.

11. Verfahren nach einem der Ansprüche 1-10, wobei das Verfahren eine Bildgebung der biologischen Probe umfasst, um die zirkularisierte Sonde oder das Produkt davon nachzuweisen, wobei die Bildgebung ein Nachweisen eines Signals umfasst, das mit einer fluoreszenzmarkierten Sonde assoziiert ist, die direkt oder indirekt an ein Rolling-Circle-Amplifikationsprodukt der zirkularisierten Sonde bindet.

12. Verfahren nach Anspruch 11, wobei eine Sequenz des Rolling-Circle-Amplifikationsprodukts *in situ* in der biologischen Probe analysiert wird, wobei die Sequenz des Rolling-Circle-Amplifikationsprodukts durch sequentielle Hybridisierung, Sequenzierung durch Hybridisierung, Sequenzierung durch Ligation, Sequenzierung durch Synthese, Sequenzierung durch Bindung oder eine Kombination davon analysiert wird.

13. Verfahren nach Anspruch 12, wobei die Sequenz des Rolling-Circle-Amplifikationsprodukts eine oder mehrere Barcodesequenzen oder Komplemente davon umfasst, wobei die eine oder die mehreren Barcodesequenzen oder Komplemente davon einer Zielnukleinsäure und/oder der Sequenz von Interesse entsprechen.

14. Verfahren nach einem der Ansprüche 1-13, wobei die biologische Probe eine Gewebeprobe ist.

15. Kit zum Analysieren einer biologischen Probe, umfassend eine zirkularisierbare Sonde, die eine 5'-Hybridisierungsregion und eine 3'-Hybridisierungsregion umfasst, die jeweils mit einer benachbarten ersten und zweiten Zielregion in einer Zielnukleinsäure hybridisieren, wobei:
die 5'-Hybridisierungsregion und die 3'-Hybridisierungsregion unterschiedlich lang sind,
die 5'-Hybridisierungsregion oder die 3'-Hybridisierungsregion eine Abfragesequenz umfasst, die jeweils komplementär zu einer Sequenz von Interesse in der ersten oder zweiten Zielregion ist,
die Abfragesequenz kein ligierbares Ende umfasst, und
sich die Abfragesequenz in der kürzeren aus der 5'-Hybridisierungsregion und der 3'-Hybridisierungsregion befindet.

## Revendications

1. Procédé d'analyse d'un échantillon biologique, comprenant :
a) la mise en contact de l'échantillon biologique avec une sonde circularisable, dans lequel :
la sonde circularisable comprend une région d'hybridation 5' et une région d'hybridation 3' qui s'hybrident à des première et deuxième régions cibles adjacentes, respectivement, dans un acide nucléique cible dans l'échantillon biologique,
la région d'hybridation 5' et la région d'hybridation 3' sont de longueurs différentes,
la région d'hybridation 5' ou la région d'hybridation 3' comprend une séquence d'interrogation complémentaire d'une séquence d'intérêt dans la première ou la deuxième région cible, respectivement,
la séquence d'interrogation ne comprend pas d'extrémité ligaturable, et
dans lequel la séquence d'interrogation est dans la plus courte parmi la région d'hybridation 5' et la région d'hybridation 3' ;
b) la circularisation de la sonde circularisable hybridée à l'acide nucléique cible pour former une sonde circularisée ; et
c) la détection de la sonde circularisée ou d'un produit de celle-ci dans l'échantillon biologique.

2. Procédé selon la revendication 1, dans lequel l'acide nucléique cible comprend de l'ARN.

3. Procédé selon l'une quelconque des revendications 1 et 2, dans lequel la région d'hybridation 5' est plus courte que la région d'hybridation 3'.

4. Procédé selon l'une quelconque des revendications 1 et 2, dans lequel la région d'hybridation 5' est plus longue que la région d'hybridation 3'.

5. Procédé selon l'une quelconque des revendications 1 à 4, dans lequel les longueurs de la région d'hybridation 5' et de la région d'hybridation 3' diffèrent par 1, 2, 3, 4, 5, 6, 7, 8, 9, 10, 11, 12, 13, 14, 15, 16, 17, 18, 19, 20, 21, 22, 23, 24, ou 25 nucléotides, facultativement dans lequel la région d'hybridation 5' comprend la séquence d'interrogation et est plus courte que la région d'hybridation 3' d'environ 5, 6, 7, 8, 9, 10, 11, 12, 13, 14, ou 15 nucléotides.

6. Procédé selon l'une quelconque des revendications 1 à 5, dans lequel la séquence d'interrogation dans la sonde circularisable a une longueur d'un, deux, trois, quatre ou cinq nucléotides.

7. Procédé selon la revendication 6, dans lequel la séquence d'interrogation est un nucléotide d'interrogation, et la séquence d'intérêt est un nucléotide unique d'intérêt choisi dans le groupe constitué par un polymorphisme mononucléotide (SNP), un variant mononucléotidique (SNV), une substitution mononucléotidique, une mutation ponctuelle, une délétion mononucléotidique et une insertion mononucléotidique.

8. Procédé selon la revendication 7, dans lequel le nucléotide d'interrogation est le deuxième, troisième, quatrième, cinquième, sixième, septième, huitième, neuvième ou dixième nucléotide de la région d'hybridation 5', le nucléotide terminal 5' de la sonde circularisable étant le premier nucléotide de la région d'hybridation 5', facultativement dans lequel le nucléotide d'interrogation est le quatrième, cinquième, ou sixième nucléotide de la région d'hybridation 5'.

9. Procédé selon l'une quelconque des revendications 1 à 8, comprenant en outre, avant l'étape de circularisation, une étape d'élimination des molécules de la sonde circularisable qui ne sont pas liées à l'acide nucléique cible de l'échantillon biologique.

10. Procédé selon l'une quelconque des revendications 1 à 9, comprenant en outre la génération du produit de la sonde circularisée *in situ* dans l'échantillon biologique, facultativement dans lequel le produit est généré en utilisant une amplification par cercle roulant (RCA).

11. Procédé selon l'une quelconque des revendications 1 à 10, dans lequel le procédé comprend l'imagerie de l'échantillon biologique pour détecter la sonde circularisée ou le produit de celle-ci dans lequel l'imagerie comprend la détection d'un signal associé à une sonde marquée par fluorescence qui se lie directement ou indirectement au produit d'amplification par cercle roulant de la sonde circularisée.

12. Procédé selon la revendication 11, dans lequel une séquence du produit d'amplification par cercle roulant est analysée *in situ* dans l'échantillon biologique, dans lequel la séquence du produit d'amplification par cercle roulant est analysée par hybridation séquentielle, séquençage par hybridation, séquençage par ligature, séquençage par synthèse, séquençage par liaison, ou une combinaison de ceux-ci.

13. Procédé selon la revendication 12, dans lequel a séquence du produit d'amplification par cercle roulant comprend une ou plusieurs séquences de code à barres ou des compléments de celles-ci, dans lequel une ou plusieurs séquences de code à barres ou des compléments de celles-ci correspondent à l'acide nucléique cible et/ou à la séquence d'intérêt.

14. Procédé selon l'une quelconque des revendications 1 à 13, dans lequel l'échantillon biologique est un échantillon tissulaire.

15. Kit pour analyser un échantillon biologique, comprenant une sonde circularisable comprenant une région d'hybridation 5' et une région d'hybridation 3' qui s'hybrident à des première et deuxième régions cibles adjacentes, respectivement, dans un acide nucléique cible, dans lequel :
la région d'hybridation 5' et la région d'hybridation 3' sont de longueurs différentes,
la région d'hybridation 5' ou la région d'hybridation 3' comprend une séquence d'interrogation complémentaire d'une séquence d'intérêt dans la première ou la deuxième région cible, respectivement,
la séquence d'interrogation ne comprend pas d'extrémité ligaturable, et
la séquence d'interrogation est dans la plus courte parmi la région d'hybridation 5' et la région d'hybridation 3'.
